(19) Europäisches Patentamt
European Patent Office
Office européen des brevets

(11) EP 1 861 387 B1

(12) EUROPEAN PATENT SPECIFICATION

(45) Date of publication and mention of the grant of the patent:
**16.04.2014 Bulletin 2014/16**

(21) Application number: **06715777.6**

(22) Date of filing: **27.01.2006**

(51) Int Cl.:
***C07D 401/04*** (2006.01) ***C07D 235/04*** (2006.01)
***A61K 31/4184*** (2006.01) ***A61K 31/4164*** (2006.01)
***A61P 29/00*** (2006.01) ***A61P 17/04*** (2006.01)

(86) International application number:
**PCT/KR2006/000324**

(87) International publication number:
**WO 2006/080821 (03.08.2006 Gazette 2006/31)**

(54) **BENZIMIDAZOLE DERIVATIVES AND PHARMACEUTICAL COMPOSITIONS THEREOF**

BENZIMIDAZOL-DERIVATE UND DEREN PHARMAZEUTISCHE ZUSAMMENSETZUNGEN

DERIVES DE BENZIMIDAZOLE ET LEURS COMPOSITIONS PHARMACEUTIQUES

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.01.2005 KR 20050008183**
**18.10.2005 KR 20050098349**

(43) Date of publication of application:
**05.12.2007 Bulletin 2007/49**

(73) Proprietor: **DAEWOONG PHARMACEUTICAL CO., LTD.**
**Sungnam,**
**Kyunggi-do 461-120 (KR)**

(72) Inventors:
- **KIM, Ji Duck,**
**401-1701, Seohong Maeul Hyosung Apt.**
**Gyeonggi-do, 449-150 (KR)**
- **YOON, Hong Chul**
**Gyeonggi-do, 463-070 (KR)**
- **KIM, In Woo**
**Seoul, 152-055 (KR)**
- **HYUN, Hyae Jung**
**Yongin-si, Gyeonggi-do, 449-814 (KR)**

(74) Representative: **Ede, Eric et al**
**Murgitroyd & Company**
**Scotland House**
**165-169 Scotland Street**
**Glasgow G5 8PL (GB)**

(56) References cited:
**WO-A1-00/26192** **WO-A1-99/07703**
**WO-A1-03/032984** **WO-A1-03/066673**
**WO-A1-2004/035549** **WO-A2-2004/011439**
**FR-A- 1 488 286** **US-A1- 2002 022 624**
**US-A1- 2003 100 582** **US-B1- 6 696 437**

- **PERRY R J: "A novel palladium-catalyzed synthesis of 2-arylbenzimidazols", JOURNAL OF ORGANIC CHEMISTRY, AMERICAN CHEMICAL SOCIETY, EASTON.; US, vol. 58, 1 January 1993 (1993-01-01), pages 7016-7021, XP002134804, ISSN: 0022-3263, DOI: DOI:10.1021/JO00077A019**
- **PLA-DALMAU A: "2-(2'-hydroxyphenyl) benzothiazoles, -benzoxazoles, and -benzimidazoles for plastic scintillation applications", JOURNAL OF ORGANIC CHEMISTRY, AMERICAN CHEMICAL SOCIETY, EASTON.; US, vol. 60, no. 17, 25 September 1995 (1995-09-25), pages 5468-5473, XP002382177, ISSN: 0022-3263, DOI: DOI:10.1021/JO00122A027**
- **KREBS ET AL.: "Superradiant properties of 4,4'-bis(1H-phenanthro[9,10-d]imidazol-2-y l) biphenyl and how a laser dye with exceptional stability can be obtained in only one synthetic step", TETRAHEDRON LETTERS, vol. 42, 2001, pages 6753-6757, XP002623555,**
- **BAUER, CYMERMAN: "The Chemotherapy of Tuberculosis. Part II. Some N-substituted p-Phenylbenzamidines", JOURNAL OF THE CHEMICAL SOCIETY, 1950, pages 2078-2080, XP008133362,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 861 387 B1

- ITO ET AL.: 'Pharmacologival studies of a new non-steroidal antiimflammatory drug: 2-(5-ethylpyridin-2-yl)benzimidazole (KB-1043)' YRZNEIMITTEL.FORSCHUNG vol. 32, no. 1, 1982, pages 49 - 55, XP008121167
- KANE J J ET AL: "Polymorphism in 2,2'-diphenyl-5,5'-bibenzimidazole", JOURNAL OF HETEROCYCLIC CHEMISTRY, WILEY-BLACKWELL PUBLISHING, INC, US, vol. 7, no. 4, 1 August 1970 (1970-08-01), pages 943-946, XP002657411, ISSN: 0022-152X, DOI: 10.1002/JHET.5570070434 [retrieved on 2009-03-11]
- BLETTNER ET AL.: "Parallel Synthesis of Polyethylene Glycol Supported Biaryl Benzimidazoles and Imidazopyrazines", SYNLETT, no. 3, 1999, pages 307-310,
- YU ET AL.: "Microwave-assisted synthesis of aryl and heteroaryl derivatives of benzimidazole", HETEROCYCLES, vol. 60, no. 6, 2003, pages 1457-1460, XP008150141,
- ROBERTSON D W ET AL: "STRUCTURE-ACTIVITY RELATIONSHIPS OF ARYLIMIDAZOPYRIDINE CARDIOTONICS: DISCOVERY AND INOTROPIC ACTIVITY OF 2-Ú2-METHOXY-4- (METHYLSULFINYL)PHENYL 3/4 -1 H-IMIDAZOÚ4,5-C 3/4 PYRIDINE", JOURNAL OF MEDICINAL CHEMISTRY, AMERICAN CHEMICAL SOCIETY, US, vol. 28, no. 6, 1 June 1985 (1985-06-01), pages 717-727, XP000577047, ISSN: 0022-2623, DOI: 10.1021/JM00383A006

## Description

### Technical Field

The present invention relates, in general, to novel benzoimidazole derivatives and, more particularly, to novel benzoimidazole derivatives functioning as antagonists to a vanilloid receptor (capsaicin receptor; Transient Receptor Potential Channel, Vanilloid subfamily member 1; TRPV-1; Vanilloid receptor-1; VR-1). Also, the present invention is concerned with a method for preparing the benzoimidazole derivatives, uses of the derivatives and pharmaceutical compositions comprising the derivatives as active ingredients.

### Background Art

The vanilloid receptor, the receptor for capsaicin (trans-8-methyl-N-vanillyl-6-nonenamide), has long been assumed to exist. Finally, it was cloned in 1997 and called vanilloid receptor subtype 1 (hereinafter referred to as "VR-1") by Caterina et al. (Caterina et al., Nature, 1997, 389, 816). Located on small unmyelinated nerve fibers (C-fibers) and myelinated nerve fibers (A-fibers), VR-1 is known as an ion channel which plays an important role in sensitizing pain stimuli by introducing the strong influx of cations such as calcium and sodium ions into the nerve endings upon activation in response to external or internal stimuli. External stimuli capable of activating VR-1 are reported to include heat and acids as well as vanilloid compounds (Tominaga et al., Neuron, 1998, 21, 531). As internal stimuli to VR-1, there are leukotriene metabolites such as 12-hydroperoxyeicosa tetraenoic acid (12-HPETE) (Hwang at al., PNAS, 2000, 97, 3655), and arachidonic acid derivatives such as anandamide (Premkumar et al., Nature, 2000, 408, 985).

On the basis of these physiological activities, VR1 has attracted intensive attention as an integral controller playing a pivotal role in transferring various external injurable stimuli into nerve cells. According to a report, VR1 knock-out mice responded like normal mice to general stimuli, but showed greatly reduced pain response to heat or thermal hyperalgesia, which reflects the importance of VR1 against noxious stimuli (Caterina et al., Science, 2000, 288, 306).

VR1 is concentratively expressed in primary sensory neurons (Caterina et al., Nature, 1997, 389, 816), which are responsible for controlling functions of internal organs such as the skin, the bones, the bladder, the gastrointestinal tract, the lungs, and so on. In addition, being distributed in other neurons on the central nervous system, the kidneys, the stomach, and T-cells (Nozawa et al., Neuroscience Letter, 2001, 309, 33; Yiangou et al., Lancet (North America Edition), 2001, 357, 1338 ; Birder et al., PNAS, 2001, 98, 13396) and throughout the entire body, VR1 is inferred to play an important role in cell division and cellular signal control.

Indications found, thus far, to be associated with the control mechanism of the activity of VR1 include pain, acute pain, chronic pain, neuropathic pain, postoperative pain, migraines, arthralgia, neuropathy, nerve injury, diabetic neuropathy, neurological illness, neurodermatitis, strokes, bladder hypersensitivity, irritable bowel syndrome, respiratory disorders such as asthma, chronic obstructive pulmonary disease, etc., irritation to the skin, eyes, and mucous membranes, itching, fever, gastric-duodenal ulcer, inflammatory intestinal diseases, and urge incontinence (Korean Pat. Laid-Open Publication No. 10-2004-0034804), and an anti-obestic effect (Pharmacol. Rev., 1986, 38, 179).

Based on pharmaceutical mechanisms, both agonists and antagonists of VR1 may be used for the treatment of the above-mentioned diseases. Pain alleviating effects of VR1 agonists show the pharmaceutical mechanism based on the desensitization of capsaicin-sensitive sensory nerves. That is, VR1 agonists cause pain and irritation of sensory nerves so as to desensitize them to other noxious stimuli.
Due to the induction of pain in the early stage, VR1 agonists are developed only as local analgesics. In contrast, acting through the mechanism of blocking sensory nerves from recognizing pain signals, VR1 antagonists do not cause early pain or irritation, and have been studied for use in the treatment of systemic diseases.
As compounds capable of modulating VR1 activity, agonists such as capsaicin, DA-5018, resiniferatoxin, etc. are used as pain drugs or are under clinical study (Szallasi, J. Med chem., 2004, 47, 2717), while 20 VR1 antagonists including capsazepine and iodoresiniferatoxin are under pre-clinical study (WO0208221, WO03062209, WO2004055003, WO2004055004, WO2004002983, WO0216317, WO2004035549, WO2004014871, WO03099284, WO03022809, WO02090326, WO02072536, WO03068749, WO2004033435, WO02076946, WO0095420, WO03055484, WO03014064, WO03080578, WO03097586, WO03070247, W003029199). WO2004035549 describes benzimidazole derivatives and proposes their use as vanilloid receptor ligands for example in pharmaceutical compositions for the treatment of diseases, such as vanilloid-receptor-mediated diseases.
Featuring superior VR-I antagonistic effects, the novel benzoimidazole derivatives according to the present invention have chemical structures different from those of the VR1 antagonists reported thus far.
Although the compounds included within the scope of the present invention themselves are VR1 antagonists, the possibility is not excluded that modified forms thereof in an intracellular environment or metabolites thereof act as effective principles responsible for the medicinal activity.

Disclosure of the Invention

Leading to the present invention, intensive and thorough research on the modulation of the activity of VR-1, conducted by the present inventors, resulted in the finding that benzoimidazole derivatives different in chemical structure from antagonists known in the art can act as excellent antagonists having potent medicinal effects (pain and inflammation relief and anti-ulcer effect) and are accepted as highly safe from measurements in animal models.

Therefore, it is an object of the present invention to provide novel benzoimidazole derivatives highly inhibitory of the activity of VR-1, or innoxious salts or solvates thereof.

It is another object of the present invention to disclose a method for preparing the novel benzoimidazole derivatives, or innoxious salts or solvates thereof.

It is a further object of the present invention to provide pharmaceutical compositions inhibitory of the activity of VR-1, comprising the novel benzoimidazole derivatives, or innoxious salts or solvates thereof.

Best Mode for Carrying Out the Invention

In accordance with an aspect of the present invention as defined in claim 1, this disclosure relates to a novel benzoimidazole derivative represented by the following chemical formula 1:

Chemical Formula 1

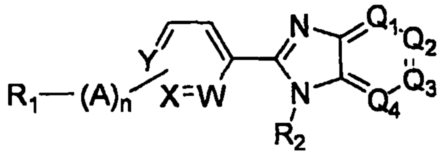

wherein

$R_1$ is hydrogen; a lower alkyl having 1 to 8 carbon atoms; a lower alkenyl having 2 to 6 carbon atoms; a lower alkoxy having 1 to 8 carbon atoms; a haloalkyl having 1 to 6 carbon atoms; a haloalkoxy having 1 to 6 carbon atoms; a halogen atom; a nitro; a hydroxy; a hydroxymethyl; a cyano; an amino or amide group which is mono- or di-substituted with an alkyl having 1 to 6 carbon atoms, or is non-substituted; cycloalkyl, pyridine, pyrimidine, pyrazole, pyrazine, phenyl, benzyl, imidazole, morpholine, benzodioxole, benzothiazole, benzoimidazole, indole, pyrazolone, thiophene, furan, thiazole, isothiazole, oxazole, isooxazole, triazole, oxodiazole, thiadiazole, indazole, pyrrolidine, chromonyl, piperidine, morpholinethyl or dihydropyrazole group which is mono-, di-or tri-substituted with a substituent selected from among a lower alkyl having 1 to 8 carbon atoms, a lower alkenyl having 2 to 6 carbon atoms, a lower alkoxy having 1 to 8 carbon atoms, a haloalkyl having 1 to 6 carbon atoms, a haloalkoxy having 1 to 6 carbon atoms, a halogen atom, a nitro, a hydroxy, a cyano, an amino or amide group which is mono- or di-substituted with an alkyl having 1 to 6 carbon atoms or is non-substituted, or sulfonamide, or is non-substituted; or $(CH_2)pAr$ group,
A is O, NH, NHCO, CO, $CO_2$, SO, $SO_2$, or $NHSO_2$,
n is an integer equal to 0 or 1,
W is N or $CR_3$,
X is N or $CR_4$,
Y is N or $CR_5$,
$R_2$ is hydrogen; a lower alkyl having 1 to 8 carbon atoms; a lower alkenyl having 2 to 6 carbon atoms; a lower alkoxy having 1 to 8 carbon atoms; a haloalkyl having 1 to 6 carbon atoms; a haloalkoxy having 1 to 6 carbon atoms; a halogen atom; nitro; hydroxy; hydroxymethyl; cyano; phosphoric acid; phosphateester; an amino or amide which is mono- or di-substituted with a lower alkyl having 1 to 6 carbon atoms; sulfanyl; sulfone; sulfoxide; sulfonamide; urea, carbamate, carbonate; ketone; ester; carboxylic acid; or methoxyethanol group; cycloalkyl, pyridine, pyrimidine, pyrazole, pyrazine, phenyl, benzyl, imidazole, morpholine, benzodioxole, benzothiazole, benzoimidazole, indole, pyrazolone, thiophene, furan, thiazole, isothiazole, oxazole, isooxazole, triazole, oxodiazole, thiadiazole, indazole, pyrrolidine, chromonyl, piperidine, morpholinethyl, or dihydropyrazole group which is mono-, di- or tri-substituted with a substituent selected from among hydrogen, a lower alkyl having 1 to 8 carbon atoms, a lower alkenyl having 2 to 6 carbon atoms, a lower alkoxy having 1 to 8 carbon atoms, a haloalkyl having 1 to 6 carbon atoms, a haloalkoxy having 1 to 6 carbon atoms, a halogen atom, nitro, hydroxy, cyano, an amino or amide which is mono- or di-substituted with a lower alkyl having 1 to 6 carbon atoms or non-substituted, sulfanyl, sulfone, sulfoxide, sulfonamide, urea, carbamate, carbonate, ketone, ester or carboxylic acid,
$Q_1$ is N or $CR_6$,

$Q_2$ is N or $CR_7$,
$Q_3$ is N or $CR_8$,
$Q_4$ is N or $CR_9$,
Ar is selected from among

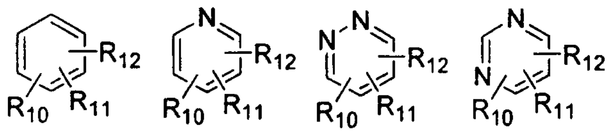

;

p is an integer of 0, 1, 2, 3 or 4;

$R_3$, $R_4$, and $R_5$ may be the same or different, and are independently hydrogen; a lower alkyl having 1 to 8 carbon atoms; a lower alkenyl having 2 to 6 carbon atoms; a lower alkoxy having 1 to 6 carbon atoms; a haloalkyl having 1 to 6 carbon atoms; a haloalkoxy having 1 to 6 carbon atoms; a halogen atom; nitro; hydroxy; cyano; sulfanyl; sulfone; sulfoxide; sulfonamide; urea; carbamate; carbonate; ketone; ester; carboxylic acid; an amino or amide group which is mono- or di-substituted with an alkyl having 1 to 3 carbon atoms or is non-substituted; a phenyl or benzyl group which is substituted with a substituent selected from among hydrogen, a lower alkyl having 1 to 6 carbon atoms, a lower alkenyl having 2 to 4 carbon atoms, a lower alkoxy having 1 to 6 carbon atoms, a haloalkyl having 1 to 6 carbon atoms, a haloalkoxy having 1 to 6 carbon atoms or a halogen atom; or any two of $R_3$, $R_4$, and $R_5$ together form a saturated, partially saturated, or unsaturated 5-, 6- or 7-membered heteromonocyclic ring or a saturated, partially saturated, or unsaturated 6-, 7-, 8-, 9-, 10- or 11-membered heterobicyclic ring compound which is mono-, di-, tri- or tetra-substituted with a nitrogen atom, an oxygen atom or a sulfur atom, or is non-substituted,

$R_6$, $R_7$, $R_8$, and $R_9$ may be the same or different, and are independently hydrogen; a lower alkyl having 1 to 8 carbon atoms; a lower alkenyl having 2 to 6 carbon atoms; a lower alkoxy having 1 to 8 carbon atoms; a haloalkyl having 1 to 6 carbon atoms; a haloalkoxy having 1 to 6 carbon atoms; a halogen atom; a nitro; hydroxy; hydroxymethyl; cyano; an amino or amide which is mono- or di-substituted with a lower alkyl having 1 to 6 carbon atoms or is non-substituted; sulfanyl; sulfone; sulfoxide; sulfonamide; urea; carbamate; carbonate; ketone; ester; carboxylic acid group; cycloalkyl, pyridine, pyrimidine, pyrazole, phenyl, benzyl, imidazole, morpholine, benzodioxole, benzothiazole, benzoimidazole, indole, pyrazolone, thiophene, furan, thiazole, isothiazole, oxazole, isooxazole, triazole, oxodiazole, thiadiazole, indazole, thiomorpholine, thiazolidine, oxazolidine, pyrrolidine, chromonyl, piperidine, morpholinethyl, or dihydropyrazole group which is mono-, di- or tri-substituted with a substituent selected from among hydrogen, a lower alkyl having 1 to 8 carbon atoms, a lower alkenyl having 2 to 6 carbon atoms, a lower alkoxy having 1 to 8 carbon atoms, a haloalkyl having 1 to 6 carbon atoms, a haloalkoxy having 1 to 6 carbon atoms, a halogen atom, a nitro, hydroxy, cyano, an amino or amide which is mono- or di-substituted with a lower alkyl having 1 to 6 carbon atoms or is non-substituted, sulfanyl, sulfone, sulfoxide, sulfonamide, urea, carbamate, carbonate, ketone, ester, carboxylic acid, and

any two of $R_6$, $R_7$, $R_8$, and $R_9$ form together a saturated, partially saturated or unsaturated 5-, 6- or 7-membered heteromonocyclic or a saturated, partially saturated or unsaturated 6-, 7-, 8-, 9-, 10- or 11-membered heterobicyclic ring compound which is mono-, di-, tri- or tetra-substituted with a nitrogen atom, an oxygen atom or a sulfur atom or is non-substituted,

$R_{10}$, $R_{11}$, $R_{12}$ may be the same or different, and are independently hydrogen; a lower alkyl having 1 to 8 carbon atoms; a cycloalkyl; a lower alkenyl having 2 to 6 carbon atoms; a lower alkoxy having 1 to 6 carbon atoms; a haloalkyl having 1 to 6 carbon atoms; a halogen atom; nitro; hydroxy; hydroxymethyl; cyano; sulfanyl; sulfone; sulfoxide; sulfonamide; urea; carbamate; carbonate; ketone; an amino or amide which is mono- or di-substituted with a lower alkyl having 1 to 3 carbon atoms or is non-substituted, carboxylic acid, or carboxylester group, and

any two of $R_{10}$, $R_{11}$, and $R_{12}$ form together a saturated or unsaturated heteromonocyclic or heterobicyclic ring compound .

In claimed embodiments, the novel benzoimidazole derivative of the present invention comprise the compounds:

2-[4-(3-chloropyridin-2-yl)phenyl]-6-trifluoromethyl-1H-benzoimidazole,
6-chloro-2-[4-(3-chloropyridin-2-yl)phenyl]-1H-benzoimidazole,
6-tert-butyl-2-[4-(3-chloropyridin-2-yl)phenyl]-1H-benzoimidazole,
2-[4-(3-chloropyridin-2-yl)phenyl]-6-fluoro-1H-benzoimidazole,
2-[4-(3-chloropyridin-2-yl)phenyl]-6-methoxy-1H-benzoimidazole,
4-chloro-2-[4-(3-chloropyridin-2-yl)phenyl]-6-trifluoromethyl-1H-benzoimidazole,
2-[4-(3-chloropyridin-2-yl)phenyl]-4,6-bistrifluoromethyl-1H-benzoimidazole,

2-[4-(3-chloropyridin-2-yl)phenyl]-6-trifluoromethyl-1H-imidazo[4,5-b]pyridine,
2-[4-(3-chloropyridin-2-yl)phenyl]-3H-imidazo[4,5-b]pyridine,
2-[4-(3-chloropyridin-2-yl)phenyl]-6,7-dimethyl-1H-benzoimidazole,
2-[4-(3-chloropyridin-2-yl)phenyl]-1H-benzoimidazole, 2-[4-(3-chloropyridin-2-yl)phenyl]-5,6-dichloro-1H-benzoimidazole,
6-bromo-2-(4-(3-chloropyridin-2-yl)phenyl)-1H-benzoimidazole,
4-bromo-2-(4-(3-chloropyridin-2-yl)phenyl)-6-(trifluoromethyl)-1H-benzoimidazole,
6-bromo-2-(4-(3-chloropyridin-2-yl)phenyl-1H-imidazo[4,5-b]pyridine,
4,6-dibromo-2-[4-(3-chloropyridin-2-yl)phenyl]-1H-benzoimidazole,
2-[4-(3-chloropyridin-2-yl)phenyl]-6-morpholin-4-yl-1H-benzoimidazole,
2-[4-(3-chloropyridin-2-yl)phenyl]-6-thiomorpholin-4-yl-1H-benzoimidazole,
2-[4-(3-chloropyridin-2-yl)phenyl]-4-morpholin-4-yl-6-trifluoromethyl-1H-benzoimidazole,
2-[4-(3-chloropyridin-2-yl)phenyl]-4-thiomorpholin-4-yl-6-trifluoromethyl-1H-benzoimidazole,
2-[4-(3-chloropyridin-2-yl)phenyl]-4-pyrrolidin-l-yl-6-trifluoromethyl-1H-benzoimidazole,
{2-[4-(3-chloropyridin-2-yl)phenyl]-6-trifluoromethyl-1H-benzoimidazole-4-yl}diethylamine,
2-[4-(3-chloropyridin-2-yl)phenyl]-6-morpholin-4-yl-1H-imidazo[4,5-b]pyridine,
2-[4-(3-chloropyridin-2-yl)phenyl]-6-pyrrolidin-l-yl-1H-imidazo[4,5-b]pyridine,
6-tert-butyl-2-[4-(3,5-dichloropyridin-2-yl)phenyl]-1H-benzoimidazole,
2-[4-(3,5-dichloropyridin-2-yl)phenyl]-4,6-bistrifluoromethyl-1H-benzoimidazole,
6-*tert*-butyl-2-[4-(3-chloro-5-trifluoromethylpyridin-2-yl)phenyl]-1H-benzoimidazole,
2-[9-(3-chloro-5-trifluoromethylpyridin-2-yl)phenyl]-4,6-bistrifluoromethyl-1H-benzoimidazole,
2-[4-(3-chloro-5-trifluoromethylpyridin-2-yl)phenyl]-5,7-bistrifluoromethyl-1H-benzoimidazole,
N-[4'-(4,6-bistrifluoromethyl-1H-benzoimidazole-2-yl)-3-fluorobiphenyl-4-yl]methanesulfonamide,
6-tert-butyl-2-[4-(3-trifluoromethylpyridin-2-yl)phenyl]-1H-benzoimidazole,
4-chloro-6-trifluoromethyl-2-[4-(3-trifluoromethylpyridin-2-yl)phenyl]-1H-benzoimidazole,
6-chloro-2-[4-(3-trifluoromethylpyridin-2-yl)phenyl]-1H-benzoimidazole,
4,6-bistrifluoromethyl-2-[4-(3-trifluoromethyl pyridin-2-yl)phenyl]-1H-benzoimidazole,
6-trifluoromethyl-2-[4-(3-trifluoromethylpyridin-2-yl)phenyl]-1H-imidazo[4,5-b]pyridine,
5,6-dichloro-2-[4-(3-trifluoromethylpyridin-2-yl)phenyl]-1H-benzoimidazole,
4-bromo-6-(trifluoromethyl)-2-(4-(3-(trifluoromethyl) pyridin-2-yl)phenyl)-1H-benzoimidazole,
6-bromo-2-(4-(3-(trifluoromethyl)pyridin-2-yl)phenyl)-1H-benzoimidazole,
6-fluoro-2-(4-(3-(trifluoromethyl)pyridin-2-yl)phenyl)-1H-benzoimidazole,
6-bromo-2-(4-(3-(trifluoromethyl)pyridin-2-yl)phenyl-1H-imidazo[4,5-b]pyridine,
4,6-dibromo-2-[4-(3-trifluoromethylpyridin-2-yl)phenyl]-1H-benzoimidazole,
6-morpholin-4-yl-2-[4-(3-trifluoromethylpyridin-2-yl)phenyl]-1H-benzoimidazole,
6-thiomorpholin-4-yl-2-[4-(3-trifluoromethylpyridin-2-yl)phenyl]-1H-benzoimidazole,
diethyl-{2-[4-(3-trifluoromethylpyridin-2-yl)phenyl]-3H-benzoimidazo-5-yl}amine,
4-morpholin-4-yl-6-trifluoromethyl-2-[4-(3-trifluoromethylpyridin-2-yl)phenyl]-1H-benzoimidazole,
4-thiomorpholin-4-yl-6-trifluoromethyl-2-[4-(3-trifluoromethylpyridin-2-yl)phenyl]-1H-benzoimidazole,
4-pyrrolidin-1-yl-6-trifluoromethyl-2-[4-(3-trifluoromethylpyridin-2-yl)phenyl]-1H-benzoimidazole,
6-*tert*-butyl-2-(4-pyridin-2-ylphenyl)-1H-benzoimidazole,
6-((trifluoromethyl)-2-(4-(pyridin-2-yl)phenyl)-1H-benzoimidazole,
4-chloro-6-(trifluoromethyl)-2-(4-(pyridin-2-yl)phenyl)-1H-benzoimidazole,
5,6-dichloro-2-(4-(pyridin-2-yl)phenyl)-1H-benzoimidazole,
4,6-bis(trifluoromethyl)-2-(4-(pyridin-2-yl)phenyl)-1H-benzoimidazole,
6-fluoro-2-(4-(pyridin-2-yl)phenyl)-1H-benzoimidazole,
6-bromo-2-(4-(pyridin-2-yl)phenyl)-1H-imidazo[4,5-b]pyridine,
4,6-dibromo-2-(4-pyridin-2-ylphenyl)-1H-benzoimidazole,
2-[4-(6-chloro-5-methylpyridazin-3-yl)phenyl]-4,6-bistrifluoromethyl-1H-benzoimidazole,
2-[4-(6-chloro-5-methylpyridazin-3-yl)phenyl]-5,7-bistrifluoromethyl-1H-benzoimidazole,
4-bromo-6-(trifluoromethyl)-2-(4-(4-(trifluoromethyl)pyrimidin-2-yl)phenyl)-1H-benzoimidazole,
4,6-dibromo-2-[4-(4-trifluoromethylpyrimidin-2-yl)phenyl]-1H-benzoimidazole,
6-t*ert*-butyl-2-(4-(pyrimidin-2-yl)phenyl)-1H-benzoimidazole,
2-((4-(6-(trifluoromethyl)-1H-benzoimidazole-2-yl)phenyl)pyridin-3-carbonitrile,
2-((4-(6-(*tert*-butyl-1H-benzoimidazole-2-yl)phenyl)pyridin-3-carbonitrile,
6'-(4,6-dibromo-1H-benzoimidazole-2-yl)-[2,3']bipyridyl-3-carbonitrile,
2-[4-(3-chloropyrazin-2-yl)phenyl]-6-trifluoromethyl-1H-benzoimidazole,
6-*tert*-butyl-2-[4-(3-chloropyrazin-2-yl)phenyl]-1H-benzoimidazole,

6-bromo-2-[4-(3-chloropyrazin-2-yl)phenyl]-1H-benzoimidazole,
5,6-dichloro-2-[4-(3-chloropyrazin-2-yl)phenyl]-1H-benzoimidazole,
6-bromo-2-[4-(3-chloropyrazin-2-yl)phenyl]-1H-imidazo[4,5-b]pyridine,
6-tert-butyl-2-[4-(3-chloropyridin-2-yl)-2-fluorophenyl]-1H-benzoimidazole,
4-chloro-2-[4-(3-chloropyridin-2-yl)-2-fluorophenyl]-6-trifluoromethyl-1H-benzoimidazole,
2-[4-(3-chloropyridin-2-yl)-2-fluorophenyl]-6-methoxy-1H-benzoimidazole,
6-tert-butyl-2-[4-(3,5-dichloropyridin-2-yl)-2-fluorophenyl]-1H-benzoimidazole,
4-chloro-2-[4-(3,5-dichloropyridin-2-yl)-2-fluorophenyl]-6-trifluoromethyl-1H-benzoimidazole,
2-[4-(3,5-dichloropyridin-2-yl)-2-fluorophenyl]-4,6-bistrifluoromethyl-1H-benzoimidazole,
4-chloro-2-[4-(3-chloro-5-trifluoromethylpyridin-2-yl)-2-fluorophenyl]-6-trifluoromethyl-1H-benzoimidazole,
2-[4-(3-chloro-5-trifluoromethylpyridin-2-yl)-2-fluorophenyl]-6-trifluoromethyl-1H-imidazo[4,5-b]pyridine,
2-[4-(3-chloro-5-trifluoromethylpyridin-2-yl)-2-fluorophenyl]-6-methoxy-1H-benzoimidazole,
N-[3,3'-difluoro-4'-(6-methoxy-1H-benzoimidazole-2-yl)biphenyl-9-yl]methanesulfonamide,
2-[2-fluoro-4-(3-trifluoromethylpyridin-2-yl)phenyl]-6-trifluoromethyl-1H-benzoimidazole,
6-*tert*-butyl-2-[2-fluoro-4-(3-trifluoromethylpyridin-2-yl)phenyl]-1H-benzoimidazole,
6-chloro-2-[2-fluoro-4-(3-trifluoromethylpyridin-2-yl)phenyl]-1H-benzoimidazole,
5-chloro-2-[2-fluoro-4-(3-trifluoromethylpyridin-2-yl)phenyl]-1H-benzoimidazole,
4-chloro-2-[2-fluoro-4-(3-trifluoromethylpyridin-2-yl)phenyl]-6-trifluoromethyl-1H-benzoimidazole,
7-chloro-2-[2-fluoro-4-(3-trifluoromethylpyridin-2-yl)phenyl]-5-trifluoromethyl-1H-benzoimidazole,
2-((2-fluoro-4-pyridin-2-yl)phenyl-6-trifluoromethyl-1H-benzoimidazole,
6-*tert*-butyl-2-(2-fluoro-4-pyridin-2-yl)phenyl-1H-benzoimidazole,
6-chloro-2-(2-fluoro-4-pyridin-2-yl)phenyl-1H-benzoimidazole,
2-((2-fluoro-4-pyridin-2-yl)phenyl-4,6-bistrifluoro methyl-1H-benzoimidazole,
2-[2-chloro-4-(3-chloropyridin-2-yl)phenyl]-6-trifluoromethyl-1H-benzoimidazole,
4-chloro-2-[2-chloro-4-(3-chloropyridin-2-yl)phenyl]-6-trifluoromethyl-1H-benzoimidazole,
2-[2-chloro-4-(3-chloropyridin-2-yl)phenyl]-6-trifluoromethyl-1H-imidazo[4,5-b]pyridine,
2-[2-chloro-4-(3,5-dichloropyridin-2-yl)phenyl]-6-trifluoromethyl-1H-benzoimidazole,
4-chloro-2-[2-chloro-4-(3,5-dichloropyridin-2-yl)phenyl]-6-trifluoromethyl-1H-benzoimidazole,
2-[2-chloro-4-(3,5-dichloropyridin-2-yl)phenyl]-4,6-bistrifluoromethyl-1H-benzoimidazole,
2-[2-chloro-4-(3,5-dichloropyridin-2-yl)phenyl]-6-trifluoromethyl-1H-imidazo[4,5-b]pyridine,
2-[2-chloro-4-(3-chloro-5-trifluoromethylpyridin-2-yl)phenyl]-6-trifluoromethyl-1H-benzoimidazole,
4-chloro-2-[2-chloro-4-(3-chloro-5-trifluoromethylpyridin-2-yl)phenyl]-6-trifluoromethyl-1H-benzoimidazole,
N-[4'-(6-*tert*-butyl-1H-benzoimidazole-2-yl)-3'-chloro-3-fluorobiphenyl-4-yl]methanesulfonamide,
N-[3'-chloro-4'-(4-chloro-6-trifluoromethyl-1H-benzoimidazole-2-yl)-3-fluorobiphenyl-4-yl]methanesulfonamide,
N-[4'-(4,6-bistrifluoromethyl-1H-benzoimidazole-2-yl)-3'-chloro-3-fluorobiphenyl-4-yl]methanesulfonamide,
2-[2-chloro-4-(3-trifluoromethylpyridin-2-yl)phenyl]-6-trifluoromethyl-1H-benzoimidazole,
4-chloro-2-[2-chloro-4-(3-trifluoromethylpyridin-2-yl)phenyl]-6-trifluoromethyl-1H-benzoimidazole,
2-[2-chloro-4-(3-trifluoromethylpyridin-2-yl)phenyl]-4,6-bistrifluoromethyl-1H-benzoimidazole,
6-tert-butyl-2-[2-chloro-4-(3-methylpyridin-2-yl)phenyl]-1H-benzoimidazole,
6-chloro-2-(2-chloro-4-pyridin-2-ylphenyl)-1H-benzoimidazole,
2-((2-chloro-4-pyridin-2-ylphenyl)-6-trifluoromethyl-1H-imidazo[4,5-b]pyridine,
2-((2-chloro-4-pyridin-2-ylphenyl)-6-methoxy-1H-benzoimidazole,
2-[4-(3-chloropyridin-2-yl)naphthalen-1-yl]-6-trifluoromethyl-1H-benzoimidazole,
6-*tert*-butyl-2-[4-(3-chloropyridin-2-yl)naphthalen-1-yl]-1H-benzoimidazole,
2-[4-(3-chloropyridin-2-yl)naphthalen-1-yl]-4,6-bistrifluoromethyl-1H-benzoimidazole,
2-[4-(3-chloropyridin-2-yl)naphthalen-1-yl]-6-trifluoromethyl-1H-imidazo[4,5-b]pyridine,
6-tert-butyl-2-[4-(3,5-dichloropyridin-2-yl)naphthalen-1-yl]-1H-benzoimidazole,
2-[4-(3,5-dichloropyridin-2-yl)naphthalen-1-yl]-4,6-bistrifluoromethyl-1H-benzoimidazole,
6-*tert*-butyl-2-[4-(3-chloro-5-trifluoromethylpyridin-2-yl)naphthalen-1-yl]-1H-benzoimidazole,
2-[4-(3-chloro-5-trifluoromethylpyridin-2-yl)naphthalen-1-yl]-4,6-bistrifluoromethyl-1H-benzoimidazole,
N-{4-[4-(4,6-bistrifluoromethyl-1H-benzoimidazole-2-yl)naphthalen-1-yl]-2-fluorophenyl}methanesulfonamide,
6-trifluoromethyl-2-[4-(3-trifluoromethylpyridin-2-yl)naphthalen-1-yl]-1H-benzoimidazole,
6-tert-butyl-2-(4-pyridin-2-ylnaphthalen-1-yl)-1H-benzoimidazole,
2-((4-pyridin-2-ylnaphthalen-1-yl)-4,6-bistrifluoromethyl-1H-benzoimidazole,
6-*tert*-butyl-2-[3-chloro-4-(3-chloropyridin-2-yl)phenyl]-1H-benzoimidazole,
2-[3-chloro-4-(3,5-dichloropyridin-2-yl)phenyl]-6-trifluoromethyl-1H-benzoimidazole,
6-tert-butyl-2-[3-chloro-4-(3,5-dichloropyridin-2-yl)phenyl]-1H-benzoimidazole,
2-[3-chloro-4-(3,5-dichloropyridin-2-yl)phenyl]-6-methoxy-1H-benzoimidazole,

2-[3-chloro-4-(3,5-dichloropyridin-2-yl)phenyl]-5-methoxy-1H-benzoimidazole,
2-[3-chloro-4-(3-chloro-5-trifluoromethylpyridin-2-yl)phenyl]-6-trifluoromethyl-1H-benzoimidazole,
6-*tert*-butyl-2-[3-chloro-4-(3-chloro-5-trifluoromethylpyridin-2-yl)phenyl]-1H-benzoimidazole,
2-[3-chloro-4-(3-chloro-5-trifluoromethylpyridin-2-yl)phenyl]-6-methoxy-1H-benzoimidazole,
2-[3-chloro-4-(3-chloro-5-trifluoromethylpyridin-2-yl)phenyl]-5-methoxy-1H-benzoimidazole,
N-[2'-chloro-4'-(4-chloro-6-trifluoromethyl-1H-benzoimidazole-2-yl)-3-fluorobiphenyl-4-yl]methanesulfonamide,
N-[4'-(4,6-bistrifluoromethyl-1H-benzoimidazole-2-yl)-2'-chloro-3-fluorobiphenyl-4-yl]methanesulfonamide,
2-((3-chloro-4-pyridin-2-ylphenyl)-6-trifluoromethyl-1H-benzoimidazole,
6-*tert*-butyl-2-(3-chloro-4-pyridin-2-ylphenyl)-1H-benzoimidazole,
6-chloro-2-(3-chloro-4-pyridin-2-ylphenyl)-1H-benzoimidazole,
4-chloro-2-(3-chloro-4-pyridin-2-ylphenyl)-6-trifluoromethyl-1H-benzoimidazole,
5,6-dichloro-2-(3-chloro-4-(pyridin-2-yl)phenyl)-1H-benzoimidazole,
6-bromo-2-(3-chloro-4-(pyridin-2-yl)phenyl)-1H-benzoimidazole,
2-[3-chloro-4-(3-trifluoromethylpyridin-2-yl)phenyl]-6-trifluoromethyl-1H-benzoimidazole,
6-*tert*-butyl-2-[3-chloro-4-(3-trifluoromethylpyridin-2-yl)phenyl]-1H-benzoimidazole,
6-chloro-2-[3-chloro-4-(3-trifluoromethylpyridin-2-yl)phenyl]-1H-benzoimidazole,
2-[3-chloro-4-(3-trifluoromethylpyridin-2-yl)phenyl]-6-methoxy-1H-benzoimidazole,
2-[3-chloro-4-(3-methylpyridin-2-yl)phenyl]-6-trifluoromethyl-1H-benzoimidazole,
3-chloro-6'-(6-trifluoromethyl-1H-benzoimidazole-2-yl)-[2,3']bipyridine,
3-chloro-6'-(6-chloro-1H-benzoimidazole-2-yl)-[2,3']bipyridine,
3-chloro-6'-(4-chloro-6-trifluoromethyl-1H-benzoimidazole-2-yl)-[2,3']bipyridine,
6'-(4,6-bistrifluoromethyl-1H-benzoimidazole-2-yl)-3-chloro-[2,3']bipyridine,
4-bromo-2-(5-(3-chloropyridin-2-yl)pyridin-2-yl)-6-(trifluoromethyl)-1H-benzoimidazole,
6-bromo-2-(5-(3-chloropyridin-2-yl)pyridin-2-yl)-1H-benzoimidazole,
6'-(6-*tert*-butyl-1H-benzoimidazole-2-yl)-3-chloro-[2,3']bipyridine,
3-chloro-6'-(5,6-dichloro-1H-benzoimidazole-2-yl)-[2,3']bipyridine,
3-chloro-6'-(4,6-dibromo-1H-benzoimidazole-2-yl)-[2,3']bipyridine,
6'-(6-bromo-1H-imidazo[4,5-b]pyridine-2-yl)-3-chloro-[2,3']bipyridine,
3-chloro-6'-(6-morpholin-4-yl-1H-benzoimidazole-2-yl)-[2,3']bipyridine,
3-chloro-6'-(6-thiomorpholin-4-yl-1H-benzoimidazole-2-yl)-[2,3']bipyridine,
3-chloro-6'-(4-morpholin-4-yl-6-trifluoromethyl-1H-benzoimidazole-2-yl)-[2,3']bipyridine,
3-chloro-6'-(4-thiomorpholin-4-yl-6-trifluoromethyl-1H-benzoimidazole-2-yl)-[2,3']bipyridine,
3-chloro-6'-(4-pyrrolidin-1-yl-6-trifluoromethyl-1H-benzoimidazole-2-yl)-[2,3']bipyridine,
3,5-dichloro-6'-(6-trifluoromethyl-1H-benzoimidazole-2-yl)-[2,3']bipyridine,
6'-(6-*tert*-butyl-1H-benzoimidazole-2-yl)-3,5-dichloro-[2,3']bipyridine,
3,5-dichloro-6'-(6-chloro-1H-benzoimidazole-2-yl)-[2,3']bipyridine,
3,5-dichloro-6'-(4-chloro-6-trifluoromethyl-1H-benzoimidazole-2-yl)-[2,3']bipyridine,
6'-(4,6-bistrifluoromethyl-1H-benzoimidazole-2-yl)-3,5-dichloro-[2,3']bipyridine,
6'-(4,6-bistrifluoromethyl-1H-benzoimidazole-2-yl)-3-chloro-5-trifluoromethyl-[2,3']bipyridine,
6'-(6-methoxy-1H-benzoimidazole-2-yl)-3-chloro-5-trifluoromethyl-[2,3']bipyridine,
3-trifluoromethyl-6'-(6-trifluoromethyl-1H-benzoimidazole-2-yl)-[2,3']bipyridine,
6'-(6-*tert*-butyl-1H-benzoimidazole-2-yl)-3-trifluoromethyl-[2,3']bipyridine,
6'-(6-chloro-1H-benzoimidazole-2-yl)-3-trifluoromethyl-[2,3']bipyridine,
6'-(4-chloro-6-trifluoromethyl-1H-benzoimidazole-2-yl)-3-trifluoromethyl-[2,3']bipyridine,
6'-(5-trifluoromethyl-7-chloro-1H-benzoimidazole-2-yl)-3-trifluoromethyl-[2,3']bipyridine,
6'-(4,6-bistrifluoromethyl-1H-benzoimidazole-2-yl)-3-trifluoromethyl-[2,3']bipyridine,
6'-(5,6-dichloro-1H-benzoimidazole-2-yl)-3-trifluoromethyl-[2,3']bipyridine,
4-bromo-6-(trifluoromethyl)-2-(5-(3-(trifluoromethyl)pyridin-2-yl)pyridin-2-yl)-1H-benzoimidazole,
6-bromo-2-(5-(3-(trifluoromethyl)pyridin-2-yl)pyridin-2-yl)-1H-benzoimidazole,
6-fluoro-2-(5-(3-(trifluoromethyl)pyridin-2-yl)pyridin-2-yl)-1H-benzoimidazole,
6'-(6-bromo-1H-imidazo[4,5-b]pyridine-2-yl)-3-trifluoromethyl[2,3']bipyridine,
6'-(4,6-dibromo-1H-benzoimidazole-2-yl)-3-trifluoromethyl-[2,3']bipyridine,
6'-(6-morpholin-4-yl-1H-benzoimidazo-2-yl)-3-trifluoromethyl-[2,3']bipyridine,
6'-(4-morpholin-4-yl-6-trifluoromethyl-1H-benzoimidazole-2-yl)-3-trifluoromethyl-[2,3']bipyridine,
6'-(6-chloro-1H-benzoimidazole-2-yl)-[2,3']bipyridine,
6'-(6-trifluoromethyl-1H-imidazo[4,5-b]pyridine-2-yl)-[2,3']bipyridine,
5,6-dichloro-2-(5-(pyridin-2-yl)pyridin-2-yl)-1H-benzoimidazole,
6-*tert*-butyl-2-(6-naphthalen-1-ylpyridin-3-yl)-1H-benzoimidazole,

2-((6-naphthalen-1-ylpyridin-3-yl)-4,6-bistrifluoromethyl-1H-benzoimidazole,
2-((6-naphthalen-1-ylpyridin-3-yl)-5,7-bistrifluoromethyl-1H-benzoimidazole,
6-((trifluoromethyl)-2-(6-(naphthalen-1-yl)pyridin-3-yl)-1H-benzoimidazole,
6-chloro-2-(6-(naphthalen-1-yl)pyridin-3-yl)-1H-benzoimidazole,
2-((4-pyrrol-1-ylphenyl]-6-trifluoromethyl-1H-benzoimidazole,
2-((4-pyrrol-1-ylphenyl)-3H-imidazo[4,5-b]pyridine, 6-chloro-2-(4-pyrrol-1-ylphenyl]-1H-benzoimidazole,
6-tert-butyl-2-(4-pyrrol-1-ylphenyl]-1H-benzoimidazole,
6-fluoro-2-(4-pyrrol-1-ylphenyl]-1H-benzoimidazole, 2-biphenyl-4-yl-6-trifluoromethyl-1H-benzoimidazole,
2-biphenyl-4-yl-3H-imidazo[4,5-b]pyridine,
2-biphenyl-4-yl-6-chloro-1H-benzoimidazole,
2-biphenyl-4-yl-6-tert-butyl-1H-benzoimidazole,
2-biphenyl-4-yl-6-fluoro-1H-benzoimidazole,
2-biphenyl-4-yl-6-methoxy-1H-benzoimidazole,
2-((4-phenoxyphenyl-6-trifluoromethyl)-1H-benzoimidazole,
2-((4-phenoxyphenyl)-3H-imidazo[4,5-b]pyridine,
6-chloro-2-(4-phenoxyphenyl)-1H-benzoimidazole,
6-*tert*-butyl-2-(4-phenoxyphenyl)-1H-benzoimidazole,
6-fluoro-2-(4-phenoxyphenyl)-1H-benzoimidazole,
6-methoxy-2-(4-phenoxyphenyl)-1H-benzoimidazole,
2-[3-(4-chloropyrazol-1-ylmethyl)phenyl]-6-trifluoromethyl-1H-benzoimidazole,
6-chloro-2-[3-(4-chloropyrazol-1-ylmethy)phenyl]-1H-benzoimidazole,
6-*tert*-butyl-2-[3-(4-chloropyrazol-1-ylmethyl)phenyl]-1H-benzoimidazole,
2-[3-(4-chloropyrazol-1-ylmethyl)phenyl]-6-fluoro-1H-benzoimidazole,
phenyl-[4-(6-trifluoromethyl-1H-benzoimidazole-2-yl)phenyl]methanone,
[4-((6-tert-butyl-1H-benzoimidazole-2-yl)phenyl]phenylmethanone,
2-[4-(3-chloropyridin-2-yl)phenyl]-6-trifluoromethyl-1H-benzoimidazole chloride,
6-*tert*-butyl-2-[4-(3-chloropyridin-2-yl)phenyl]-1H-benzoimidazole chloride,
6-*tert*-butyl-2-[4-(3-chloropyridin-2-yl)naphthalen-1-yl]-1H-benzoimidazole chloride,
6'-(4,6-bistrifluoromethyl-1H-benzoimidazole-2-yl)-3-chloro[2,3']bipyridine chloride,
3-trifluoromethyl-6'-(6-trifluoromethyl-1H-benzoimidazole-2-yl)-[2,3']bipyridine chloride,
6-tert-butyl-2-[4-(3-chloropyridin-2-yl)phenyl]-1H-benzoimidazole sodium salt,
3-trifluoromethyl-6'-(6-trifluoromethyl-1H-benzoimidazole-2-yl)-[2,3']bipyridine sulfate,
3-trifluoromethyl-6'-(6-trifluoromethyl-1H-benzoimidazole-2-yl)-[2,3']bipyridine phosphate,
3-trifluoromethyl-6'-(6-trifluoromethyl-1H-benzoimidazole-2-yl)-[2,3']bipyridine methanesulfonate, and
3-trifluoromethyl-6'-(6-trifluoromethyl-1H-benzoimidazole-2-yl)-[2,3']bipyridine benzenesulfonate.

In accordance with another aspect of the present disclosure, a method for preparing the compounds of Chemical Formula 1 is provided. The method may be conducted to chemically synthesize the compounds in accordance with the following reaction scheme, but is not limited thereto. The reaction scheme guides the preparation of representative compounds of the present invention, but modifications may be made to reagents and starting materials well known in the art so as to employ other compounds.

Having chiral centers, some of the compounds represented by Chemical Formula 1 may be in form of enantiomers. Therefore, it should be noted that all optical isomers, R or S type stereoisomers, racemates and enantiomers of the compounds of Chemical Formula 1 are included within the scope of the present invention.

In accordance with a further aspect of the present invention, there is provided a pharmaceutical composition comprising a compound as claimed as an active ingredient in an effective amount in combination with a pharmaceutically acceptable carrier.

The following reaction scheme 1 accounts for the synthesis of the compounds represented by Chemical Formula 1:

Reaction Scheme 1

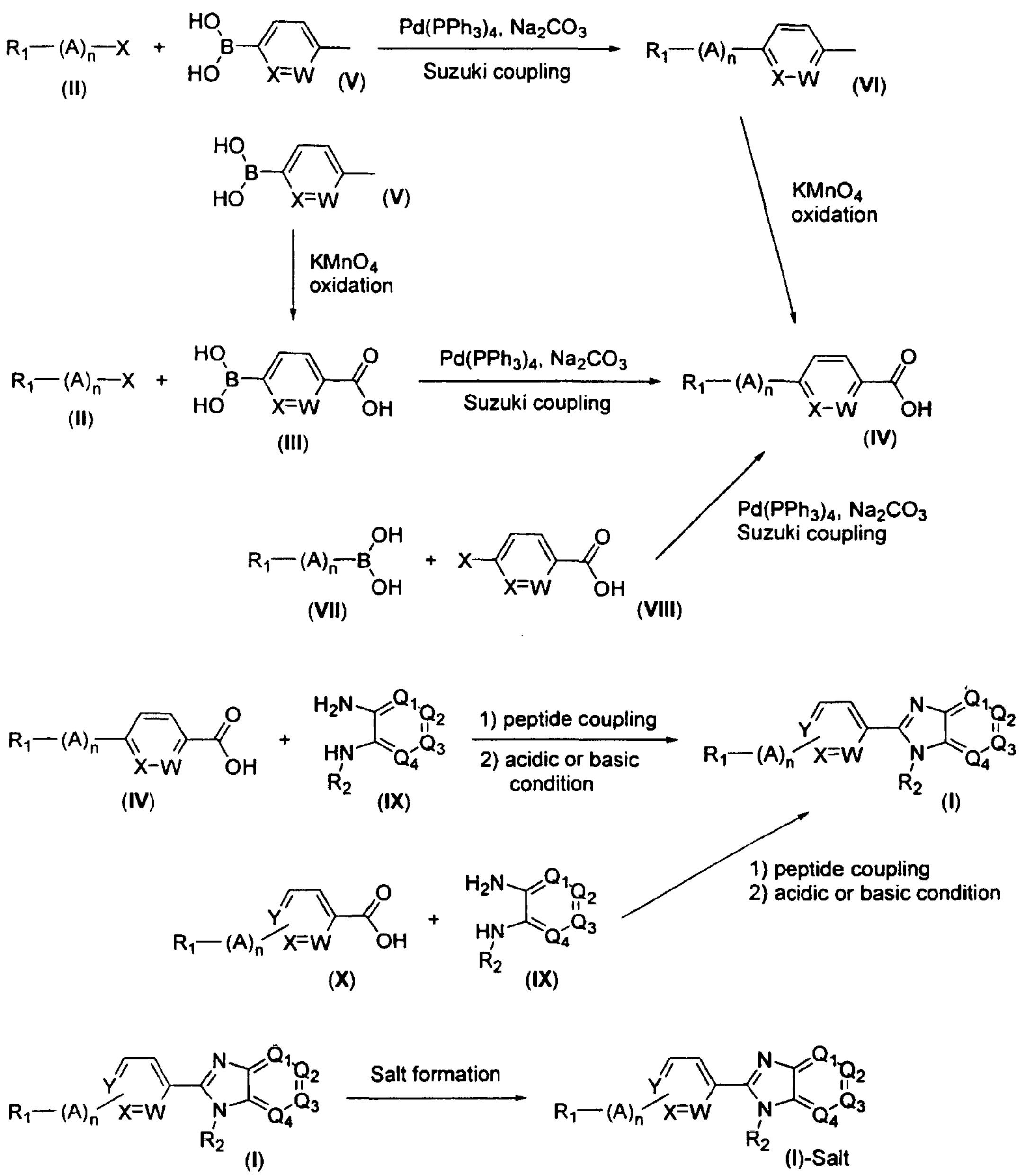


As shown in Reaction Scheme 1, benzoic acid derivatives (IX) useful in synthesizing the object compound benzoimidazole compound 1 can be prepared by reacting commercially available halogen compounds (II) with boronic acid (III) in the presence of a palladium catalyst and a base in a typical reaction condition with the aid of microwaves (Tapolcsanyi et. al., Tetrahedron, 2002, 58, 10137).

If not commercially available, boronic acid can be obtained through the oxidation of methylboronic acid (V) (Sharma et. al., Bioorg. Med. Chem. Lett. 1998, 8, 3459-3464). Alternatively, methylboronic acid (V) is reacted with a halogen compound (II), followed by oxidation to afford the benzoic acid derivatives (IX).

On the other hand, halobenzoic acid (VIII) may be reacted with commercially available boronic acid (VII), or a commercially available benzoic acid (X) may be used.

Such synthetic or commercially available benzoic acid derivatives are condensed with diamine derivatives (IX) in the presence of O-(7-azabenzotriazol-1-yl)-N,N,N',N'-tetramethyleuronium hexafluorophosphate to synthesize the intermediates, that is, amide compounds, which are then subjected to cyclization into the object compound benzoimidazole 1. In this case, the preparation of the benzoimidazole 1 may be achieved in the presence of acetic acid, a hydrochloric acid solution (Roglic et. al., Pharmazie, 2001, 56, 803), $POCl_3$ (Ries, et. al., J. Med. Chem, 1993, 36, 4040), polyphosphoric acid (Ries, et. al., J. Med. Chem, 1993, 36, 4040), or pyridine (Abha et. al., Indian Journal of Chemistry,

2002, 41, 1978) in a heated condition or with the aid of microwave (Lu, et. al., Syn. Comm. 2002, 32, 3703).

As shown above, the compounds of Chemical Formula 1 may be in form of salts, particularly pharmaceutically acceptable salts. The pharmaceutically available salts suitable for use in the present invention are those typically used in the art, such as acid addition salts, and include those disclosed in the literature (J. Pharm. Sci., 1977, 66, 1).

Examples of pharmaceutically acceptable acid addition salts suitable for use in the present invention include salts of inorganic acids, such as hydrochloric acid, hydrobromic acid, phosphoric acid, orthophosphoric aicd, sulfuric acid, and so on, and salts of organic acids, such as methanesulfonic acid, benzensulfonic acid, toluenesulfonic acid, acetic acid, propionic acid, lactic acid, citric acid, fumaric acid, malic acid, succinic acid, salicylic acid, maleic acid, glycerophosphoric acid, acetylsalicylic acid, and so on.

In addition, pharmaceutically acceptable metal salts may be prepared using bases. Alkali metal salts or alkaline earth metal salts, for example, may be obtained by dissolving compounds in an excess of an alkali metal hydroxide or alkaline earth metal hydroxide solution, filtering off non-dissolved compound salts, and vaporizing and drying the filtrate. In this regard, sodium, potassium or calcium salts are pharmaceutically suitable metal salts. In addition, silver salts corresponding to the metal salts can be obtained by reacting alkaline metal or alkali earth metal with suitable silver salts (e.g., nitrate).

Pharmaceutically unacceptable salts and/or solvates of compounds of Chemical Formula 1 may be useful as intermediates for the preparation of pharmaceutically acceptable salts and/or solvates of compounds of Chemical Formula 1, or for the preparation of the compounds themselves of Chemical Formula 1.

Compounds of Chemical Formula 1 may be prepared in crystalline or non-crystalline forms. If crystalline, the compounds may be arbitrarily hydrated or solvated. Compounds with arbitrary numbers of water molecules as well as stoichiomatric hydrates fall into the scope of the present invention.

Suitable are solvates which are pharmaceutically acceptable, like hydrates.

Solvates suitable for use in the present invention comprise both stoichiometric and non-stoichiometric solvates.

It is believed that, because they possess antagonistic activity against the vanilloid receptor, compounds of Chemical Formula 1 and pharmaceutically acceptable salts thereof have potential to be used for the prevention and treatment of indications and the treatment of the pain relevant thereto.

The indications may be exemplified by pain, acute pain, chronic pain, neuropathic pain, postoperative pain, migraine, arthralgia, neuropathy, nerve injury, diabetic neuropathy, neurological illness, neurodermatitis, stroke, bladder hypersensitivity, irritable bowel syndrome, respiratory disorders such as asthma, chronic obstructive pulmonary disease, etc., burning, psoriasis, itching, vomiting, irritation of the skin, eyes, and mucous membranes, gastric-duodenal ulcers, inflammatory intestinal diseases, and inflammatory diseases.

Thus, the present invention also provides the claimed compounds, or pharmaceutically acceptable salts or solvates thereof, which are useful as active ingredients for the prevention and treatment of indications.

Particularly, the present invention provides the claimed compounds or pharmaceutically acceptable salts or solvates thereof, which are useful for the prevention and treatment of pain.

Further, the present disclosure provides a method for preventing or treating symptoms for which antagonism to the vanilloid receptor is helpful in the therapy thereof, comprising the administration of compounds of Chemical Formula 1, or pharmaceutical acceptable salts or solvates thereof in a therapeutically effective amount to mammalians having the symptoms.

The present invention also provides uses of the claimed compounds or pharmaceutically acceptable salts or solvates in the prevention and treatment of indications for the therapy of which antagonism to the vanilloid receptor is helpful.

For use in therapy, compounds are generally formulated according to standard pharmaceutical practice. Thus, the present invention provides a pharmaceutical composition comprising a compound as claimed or a pharmaceutically acceptable salt or solvate thereof, in combination with an additive such as a pharmaceutically acceptable carrier, adjuvant, or diluent. For example, the compounds may be dissolved in oils, propyleneglycol, or other solvents, which are usually used for the preparation of injections. Illustrative, but non-limitative examples of the carrier include physiological saline, polyethylene glycol, ethanol, vegetable oil, and isopropylmyristate. For topical use, the compounds of the present invention may be formulated into ointments or creams.

Below, a description will be given of formulation methods and carriers, but such is not intended to limit the present invention.

Pharmaceutical dosage forms of the compounds of the present invention include pharmaceutically acceptable salts or solvates of the compounds of the present invention alone or in combination with other pharmaceutically active compounds suitably bound or assembled thereto.

The compounds of the present invention may be dissolved, suspended or emulsified in an aqueous solvent such as physiological saline, 5% dextrose, etc., or a nonaqueous solvent, such as synthetic fatty acid glyceride, higher fatty acid esters, propylene glycol, etc. The formulation of the present invention may comprise conventional additives such as dissolving agents, isotonic agents, suspensions, emulsifying agents, and preservatives.

Depending on a patient's state and weight, severity of disease, dosage form, and administration route and period, the administration dose of the compounds of the present invention may be suitably selected by those skilled in the art. For effective therapy, the compounds of the present invention are administered in a dose from 0.0001 to 100 mg/ weight kg a day and preferably in a dose from 0.001 to 100 mg/weight kg a day. Administration may be conducted once or many times in a partitioned manner in a day.

According to the administration method, the pharmaceutical composition may comprise the compound of the present invention in an amount from 0.001 to 99 weight%, and preferably in an amount from 0.01 to 60 weight%.

The pharmaceutical composition of the present invention may be administered via various routes to mammalians such as mice, rats, livestock, humans, etc. All administration types may be expected, including, for example, oral or rectal administration, intravenous, intramuscular, subcutaneous, intraendometrial or intracerbroventricular injection.

A better understanding of the present invention may be obtained through the following examples which are set forth to illustrate, but are not to be construed as the limit of the present invention.

EXAMPLE 1: Preparation of 2-[4-(3-Chloropyridin-2-yl)-phenyl]-6-trifluoromethyl-1H-benzoimidazole

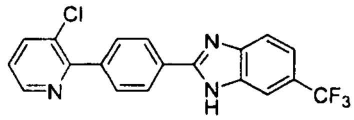

(1) Preparation of 4-(3-chloropyridin-2-yl)benzoic acid

To a solution of 5.3g (36.1mmol) 2,3-dichloropyridine in 200 mL 1,2-dimethoxyethane and 200mL distilled water were added 22.3g (0.21mol) $Na_2CO_3$, 5.0g (30.1mmol) 4-carboxylphenylboronic acid and 0.5g $Pd(PPh_3)_4$, followed by mixing for 18 hours under a heat flux condition by stirring. After being cooled to room temperature, the solution was 50% concentrated in a vacuum. The aqueous layer was washed with ethyl acetate and adjusted into pH 1 with conc. HCl. Thereafter, extraction with ethyl acetate was conducted three times and the organic layer was dried over magnesium sulfate and concentrated in a vacuum. The concentrate was separated using column chromatography (developing solvent: chloroform/methanol=10/1) to afford 5.3 g of 4-(3-chloropyridin-2-yl)benzoic acid as a white precipitate (yield: 75%).

$^1$H NMR ($CD_3OD$) $\delta$ : 8.57 (dd, 1H, *J*=4.8, 1.4Hz), 8.13 (dd, 2H, *J*=6.9, 1.7Hz), 8.03 (dd, 1H, *J*=8.1, 1.4Hz), 7.75 (dd, 2H, *J*=6.9, 1.7Hz), 7.44 (dd, 1H, *J*=8.1, 4.8Hz)

(2) Preparation of 4-(3-chloropyridin-2-yl)benzoic acid

To a solution of 0.5g (2.45mmol) 3-chloro-p-tolyl-pyridine in 5mL pyridine was added 2.5 mL distilled water, and then 1.36g (8.59mmol) potassium permanganate, followed by carefully stirring the solution to prevent potassium permanganate from contacting the container of the solution. Using a microwave reactor (Initiator Sixty, Biotage), the solution was allowed to react at 100°C for 5 min. The reaction solution was filtered to remove solids using celite. After the vacuum concentration of the filtrate, 10mL distilled water and 10mL ethyl acetate were added to the concentrate. The addition of a 1.0N hydrochloric acid solution increased the pH of the aqueous solution to 4.0 before stirring for 40 min. The separated ethyl acetate was dried over anhydrous magnesium sulfate and concentrated in a vacuum. A solution of the concentrate in methanol was separated using column chromatography (chloroform/methanol=10:1) to produce 0.35 g of 4-(3-chloropyridin-2-yl)benzoic acid (yield 61%).

(3) Preparation of 2-[4-(3-chloropyridin-2-yl)phenyl]-6-trifluoromethyl-1H-benzoimidazole

0.44g (2.5mmol) 4-trifluoromethylbenzene-1,2-diamine, 1.0 mL (5.0mmol) diisopropylethylamine and 1.1g (6mmol) O-(7-azabenzotriazol-yl)-N,N,N',N'-tetramethyleuronium hexafluorophosphate were added to a solution of 0.59g (2.5mmol) 4-(3-chloropyridin-2-yl)benzoic acid in 25 mL dimethylformamide, which was then stirred at room temperature for 16 hours and vacuum concentrated. The concentrate thus obtained was dissolved in ethyl acetate, washed with saturated $NaHCO_3$ and saturated NaCl, dried over magnesium sulfate, and concentrated in a vacuum. The residue was dissolved in acetic acid /toluene (15mL/1.5mL) and stirred at 75°C for 3 hours, followed by vacuum concentration. Again, the concentrate was dissolved in ethyl acetate, washed with saturated $NaHCO_3$ and saturated NaCl, dried over magnesium sulfate, and vacuum concentrated. The residue was separated using column chromatography (developing solvent: chloroform/methanol=30/1) to produce 0.77 g of 2-[4-(3-chloropyridin-2-yl)phenyl]-6-trifluoromethyl-1H-ben-

zoimidazole (yield 82%).
$^1$H NMR ($CDCl_3$) δ: 8.65 (m, 1H), 8.06 (d, 2H, *J*=8.4Hz), 7.93 (s, 1H), 7.85 (d, 1H, *J*=8.2Hz), 7.77 (d, 2H, *J*=8.4Hz), 7.69 (d, 1H, *J*=8.2Hz), 7.52 (d, 1H, *J*=8.2Hz), 7.34-7.30 (m, 1H)

(4) Preparation of 2-[4-(3-chloropyridin-2-yl)phenyl]-6-trifluoromethyl-1H-benzoimidazole

To a solution of 0.58g (2.5mmol) 4-(3-chloropyridin-2-yl)benzoic acid in 30mL anhydrous pyridine was added 0.44g (2.5mmol) 4-trifluoromethylbenzene-1,2-diamine, followed by stirring for 6 hours in a heat flux condition. After cooling to room temperature, cold water (100mL) containing HCl (10mL) was added to the solution, which was then allowed to stand for 30 min to form precipitates. These were separated using column chromatography (developing solvent: chloroform/methanol= 30/1) to produce 0.70 g of 2-[4-(3-chloropyridin-2-yl)phenyl]-6-trifluoromethyl-1H-benzoimidazole (yield 75%).

(5) Preparation of 2-[4-(3-chloropyridin-2-yl)phenyl]-6-trifluoromethyl-1H-benzoimidazole

To a solution of 0.64g (2.75 mmol) 4-(3-chloropyridin-2-yl)benzoic acid in 25 mL 4N HCl was added 0.44g (2.5mmol) 4-trifluoromethylbenzene-1,2-diamine, followed by stirring at 180°C for 6 hours. The solution was cooled to room temperature before the addition of 10% $NaHCO_3$ (10mL) and then extraction with ethyl acetate. The organic layer was dried over magnesium sulfate and vacuum concentrated. The residue thus obtained was separated using column chromatography (developing solvent: chloroform/methanol=30/1) to produce 0.61 g of 2-[4-(3-chloropyridin-2-yl)phenyl]-6-trifluoromethyl-1H-benzoimidazole (yield 65%).

(6) Preparation of 2-[4-(3-chloropyridin-2-yl)phenyl]-6-trifluoromethyl-1H-benzoimidazole

To a solution of 0.64g (2.75mmol) 4-(3-chloropyridin-2-yl)benzoic acid in 20mL $POCl_3$ was added 0.44g (2.5mmol) 4-trifluoromethylbenzene-1,2-diamine, followed by stirring for 3 hours in a heat flux condition. The solution was cooled to room temperature and dissolved in distilled water. After the adjustment of pH to 8-10 with ammonia, extraction was conducted using ethyl acetate. The organic layer was dried over magnesium sulfate and vacuum concentrated. The residue thus obtained was separated using column chromatography (developing solvent: chloroform/methanol=30/1) to produce 0.61 g of 2-[4-(3-chloropyridin-2-yl)phenyl]-6-trifluoromethyl-1H-benzoimidazole (yield 65%).

(7) Preparation of 2-[4-(3-chloropyridin-2-yl)phenyl]-6-trifluoromethyl-1H-benzoimidazole

To a solution of 0.70g (3.0mmol) 4-(3-chloropyridin-2-yl)benzoic acid in 25 g polyphosphoric acid was added 0.44g (2.5mmol) 4-trifluoromethylbenzen-1,2-diamine, followed by stirring at 150°C for 20 hours. Then, the solution was cooled to room temperature, dissolved in distilled water and adjusted to pH 9 to form precipitates. These precipitates were purified using column chromatography(developing solvent: chloroform/methanol=30/1) to afford 0.61 g of 2-[4-(3-chloropyridin-2-yl)phenyl]-6-trifluoromethyl-1H-benzoimidazole (yield 65%).

(8) Preparation of 2-[4-(3-chloropyridin-2-yl)phenyl]-6-trifluoromethyl-1H-benzoimidazole

A solution of 0.70g (3.0mmol) 4-(3-chloropyridin-2-yl)benzoic acid and 0.44g (2.5mmol) 4-trifluoromethylbenzene-1,2-diamine in 10 mL polyphosphoric acid was stirred for 5 min and irradiated for 15 min with microwaves in a microwave reactor (Initiator Sixty, Biotage). After cooling to room temperarture, 20mL distilled water was added to the solution which was then adjusted to a neutral pH value with NaOH. The precipitates thus obtained was filtered and separarted using column chromatography (developing solvent: chloroform/methanol=30/1) to produce 0.72 g of 2-[4-(3-chloropyridin-2-yl)phenyl]-6-trifluoromethyl-1H-benzoimidazole (yield 77%).

(9) Preparation of 2-[4-(3-chloropyridin-2-yl)phenyl]-6-trifluoromethyl-1H-benzoimidazole

0.44g (2.5mmol) 4-trifluoromethylbenzene-1,2-diamine, 1.0mL(5.0mmol) diisopropylethylamine and 1.1g (6mmol) 0-(7-azabenzotriazol-1-yl)-N,N,N',N'-tetramethyleuronium

hexafluorophosphate were added to a solution of 0.59g (2.5mmol) 4-(3-chloropyridin-2-yl)benzoic acid in 25mL dimethylformamide, which was then stirred for 5 min, irradiated for 15 min with microwaves in a microwave reactor (Initiator Sixty, Biotage) and concentrated in a vacuum. The concentrate was dissolved in ethyl acetate, washed with saturated $NaHCO_3$ and saturated NaCl, and dried over magnesium sulfate, followed by vacuum concentration. The residue thus obtained was dissolved in acetic acid (15mL) and stirred for 5 min before irradiation for 15 min with microwaves in a microwave reactor (Initiator Sixty, Biotage). Again, the reaction solution was cooled to room temperature, vacuum concentrated, dissolved in ethyl acetate, washed with saturated $NaHCO_3$ and saturated NaCl, and dried over magnesium sulfate, followed by vacuum concentration. The residue was separated using column chromatography (developing solvent: chloroform/methanol=30/1) to produce 0.81 g of 2-[4-(3-chloropyridin-2-yl)phenyl]-6-trifluoromethyl-1H-benzoimidazole (yield 90%).

EXAMPLES 2 TO 16

The same procedure as in (3) of Example 1 was performed to produce Compounds 2 to 16.

| Cpds | Structures | Yields | Spectrum Data ($^1$H NMR) |
|---|---|---|---|
| 2 | 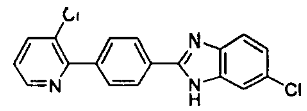 | 81 | ($CDCl_3$) δ: 8.66 (dd, 1H, *J*=4.6, 1.4Hz), 8.03 (d, 2H, *J*=8.4Hz), 7.86 (dd, 1H, *J*=8.1, 1.4Hz), 7.79 (d, 2H, *J*=8.4Hz), 7.58 (br, 1H), 7.53-7.51 (m, 1H), 7.34-7.29 (m, 1H), 7.25-7.22 (m, 1H) |
| 3 | 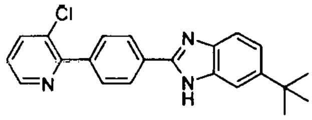 | 88 | ($CDCl_3$) δ: 8.57 (d, 1H, *J*=4.7Hz), 8.03 (d, 2H, *J*=8.2Hz), 7.77 (d, 1H, *J*=8.0Hz), 7.62 (d, 2H, *J*=8.2Hz), 7.51 (s, 1H), 7.46 (d, 1H, *J*=8.5Hz), 7.25-7.21 (m, 2H), 1.27 (s, 9H) |
| 4 | 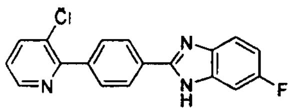 | 65 | ($CDCl_3$) δ: 8.64 (dd, 1H, *J*=4.7, 1.4Hz), 8.06(d, 2H, *J*=8.4Hz), 7.85 (d, 1H, *J*=1.4Hz), 7.82 (d, 2H, *J*=8.4Hz), 7.56 (m, 1H), 7.32-7.28 (m, 2H), 7.06-7.00 (m, 1H) |
| 5 | 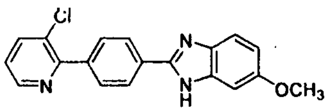 | 66 | ($CDCl_3$) δ: 8.57 (dd, 1H, *J*=4.6, 1.4Hz), 7.98(d, 2H, *J*=8.4Hz), 7.78 (dd, 1H, *J*=8.1, 1.4Hz), 7.67 (d, 2H, *J*=8.4Hz), 7.41 (d, 1H, *J*=8.8Hz), 7.25-7.21 (m, 1H), 6.94 (d, 1H, *J*=2.3Hz), 6.79 (dd, 1H, *J*=8.9, 2.4Hz), 3.72 (s, 3H) |
| 6 | 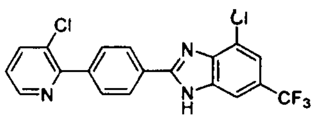 | 75 | ($CDCl_3$) δ: 8.68 (d, 1H), 8.09 (d, 2H), 7.89-7.83 (m, 4H), 7.55 (s, 1H), 7.35-7.26 (m, 1H) |
| 7 | 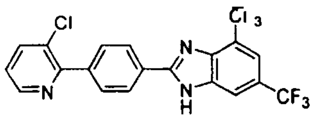 | 78 | ($CDCl_3$) δ: 8.65 (d, 1H), 8.17-8.14 (m, 3H), 7.88-7.79 (m, 4H), 7.32 (dd, 1H) |
| 8 | 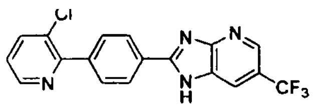 | 81 | ($CDCl_3$) δ: 8.72 (d, 1H), 8.41 (s, 1H), 8.26 (d, 2H), 7.95 (d, 2H), 7.85 (d, 1H), 7.48-7.21 (m, 2H) |
| 9 | 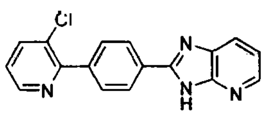 | 52 | ($CDCl_3$) δ: 8.59-8.62 (m, 2H), 8.13-8.11 (m, 1H), 7.90-7.85 (m, 1H), 7.84-7.81 (m, 1H), 7.78 (d, 2H, *J*=8.3Hz), 7.52 (d, 2H, *J*=8.3Hz), 7.27-7.23 (m, 1H) |
| 10 | 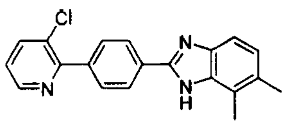 | 90 | ($CD_3OD$) δ: 8.61 (d, 1H), 8.25 (d, 2H), 8.04 (d, 1H), 7.91 (d, 2H), 7.48-7.40 (m, 2H), 7.21 (d, 1H), 2.59 (s, 3H), 2.44 (s, 3H) |
| 11 | 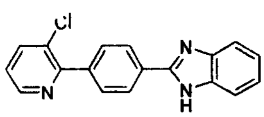 | 93 | ($CD_3OD$) δ: 8. 64 (d, 1H), 8.26 (d, 2H), 8.06 (d, 1H), 8.00 (d, 2H), 7.84-7.80 (m, 2H), 7.58-7.52 (m, 2H), 7.50-7.47 (m, 1H) |

(continued)

| Cpds | Structures | Yields | Spectrum Data ($^1$H NMR) |
|---|---|---|---|
| 12 | 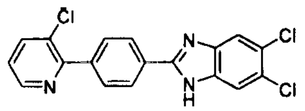 | 94 | ($CD_3OD$) δ: 8.62-8.58 (m, 1H), 8.22 (d, 2H), 8.03 (d, 1H), 7.88 (d, 2H), 7.77-7.73 (m, 2H), 7.47-7.44 (m, 1H) |
| 13 | 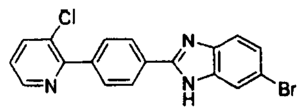 | 62 | ($CD_3OD$) δ: 8.57 (dd, 1H), 8.18 (d, 2H), 8.00 (dd, 1H), 7.84 (d, 2H), 7.76 (d, 1H), 7.54 (d, 1H), 7.39-7.45 (m, 2H) |
| 14 | 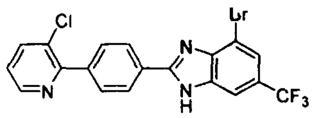 | 83 | ($CD_3OD$) δ: 8.61 (dd, 1H), 8.32 (d, 2H), 8.04 (dd, 1H), 7.91-7.88 (m, 3H), 7.73 (d, 1H), 7.47 (dd, 1H) |
| 15 | 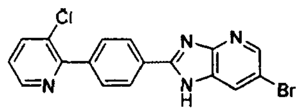 | 57 | ($CD_3OD$) δ: 8.60 (dd, 1H), 8.10 (d, 2H), 8.03 (dd, 1H), 7.96 (dd, 1H), 7.84-7.80 (m, 3H), 7.47 (dd, 1H) |
| 16 | 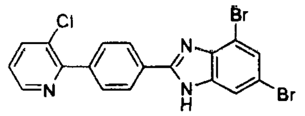 | 85 | ($CDCl_3$) δ: 8.64 (d, 1H), 8.12 (d, 2H), 7.86-7.74 (m, 4H), 7.57 (d, 1H), 7.32 (dd, 1H) |

EXAMPLE 17: Preparation of 2-[4-(3-chloropyridin-2-yl)phenyl]-6-morpholin-4-yl-1H-benzoimidazole

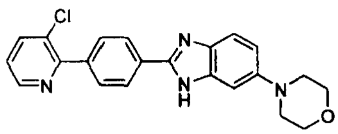

To a solution of 162mg (0.42mmol) 6-bromo-2-(4-(3-chloropyridin-2-yl)phenyl)-1H-benzoimidazole in 6 mL dichloromethane were added 0.2mL (1.15 mmol) N,N-dimethylisopropylethylamine and 0.1mL(0.57mmol) 2-(trimethylsilyl)ethoxymethyl chloride, followed by stirring at room temperature for 16 hours. After vacuum concentration, 26μL (0.30mmol) morpholine, 56.3mg (0.013mmol) tris(dibenzylidine acetone)dipalladium(0), 10mg (0.038 mmol) 2-(di-t-butylphosphino)biphenyl, and 38.8mg (0.41mmol) sodium-t-botoxide were added to the concentrate, which was then dissolved in 2.5mL toluene and allowed to react at 90°C for 10 min in a microwave reactor. After being cooled to room temperature, the product was dissolved in 5mL ethyl acetate and filtered with diatomite. The filtrate was dried over magnesium sulfate and vacuum concentrated. The concentrate was dissolved in 3 mL of a mixture of trifluoro fluoroacetic acid and methylene chloride (1:1) and stirred for 12 hours. Neutralization with 10% NaOH, extraction with ethyl acetate, drying with magnesium sulfate and vacuum concentration were sequentially conducted in that order. The residue thus obtained was separated using column chromatography (developing solvent: hexane/ethyl acetate=1/1) to produce 115mg of 2-[4-(3-chloropyridin-2-yl)phenyl]-6-morpholin-4-yl-1H-benzoimidazole (yield 70%).
$^1$H NMR ($CD_3OD$) δ: 8.64 (d, 1H), 8.18 (d, 2H), 8.10 (d, 1H), 7.87 (d, 2H), 7.66 (d, 1H), 7.61-7.45 (m, 2H), 7.21(dd, 1H), 3.91-3.87(m, 4H), 3.32-3.27(m, 4H)

EXAMPLES 18 TO 24

The same procedure as in Example 17 was performed to produce Compounds 18 to 24.

| Cpds | Structures | Yields | Spectrum Data ($^1$H NMR) |
|---|---|---|---|
| 18 | 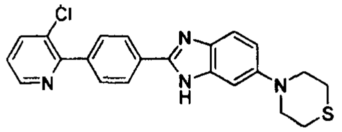 | 65 | ($CD_3OD$) δ: 8.65 (d, 1H), 8.23 (d, 2H), 8.08-8.03 (m, 3H), 7.88 (m, 1H), 7.75 (d, 1H), 7.38 (dd, 1H), 7.23(d, 1H), 3.70-3.67 (m, 4H), 2.83-2.79(m, 4H) |
| 19 | 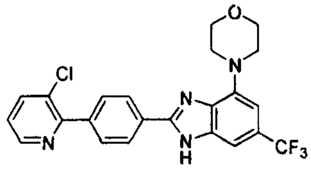 | 59 | ($CD_3OD$) δ : 8.63 (dd, 1H), 8.31 (d, 2H), 8.16 (d, 1H), 7.92 (m, 3H), 7.80 (s, 1H), 7.50-7.48 (m, 1H), 4.01-3.98 (m, 4H), 3.40-3.3.7 (m, 4H) |
| 20 | 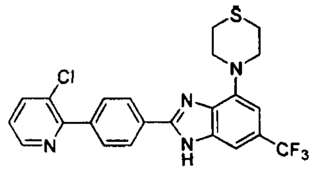 | 63 | ($CD_3OD$) δ : 8.63 (d, 1H), 8.31 (d, 2H), 8.16 (d, 1H), 7.88-7.80 (m, 3H), 7.77 (s, 1H), 7.52-7.47 (m, 1H), 3.69-3.67 (m, 4H), 2.97-2.94 (m, 4H) |
| 21 | 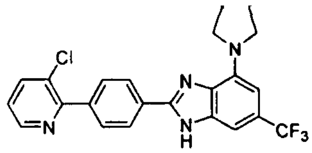 | 74 | ($CD_3OD$) δ : 8. 63 (d, 1H), 8.24 (d, 2H), 8.05 (d, 1H), 7.95-7.92 (m, 3H), 7.99 (m, 1H), 7.34 (s, 1H), 3.81 (m, 4H), 2.17 (m, 4H) |
| 22 | 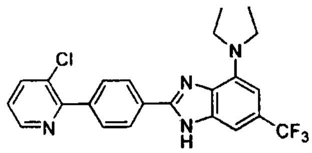 | 74 | ($CD_3OD$) δ : 8.63 (d, 1H), 8.23 (d, 2H), 8.06 (d, 2H), 7.96-7.93 (m, 2H), 7.49 (m, 1H), 7.34 (s, 1H), 3.05-2.95 (m, 4H), 1.03 (t, 6H) |
| 23 | 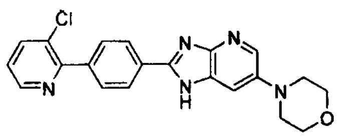 | 51 | ($CD_3OD$) δ : 8.60 (d, 1H), 8.26 (d, 2H), 8.04 (d, 1H), 7.86 (d, 2H), 7.64 (d, 1H), 7.45-7.39 (m, 2H), 3.82-3.78 (m, 4H), 3.34-3.30 (m, 4H) |
| 24 | 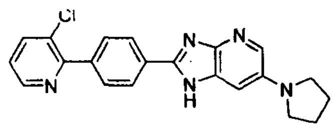 | 51 | ($CD_3OD$) δ : 8.61 (d, 1H), 8.25 (d, 2H), 8.03 (d, 1H), 7.85 (d, 2H), 7.64 (d, 1H), 7.44-7.38 (m, 2H), 4.39-4.35 (m, 4H), 2.16-2.05 (m, 4H) |

EXAMPLE 25: Preparation of 6-*tert*-butyl-2-[4-(3,5-dichloropyridin-2-yl)phenyl]-1H-benzoimidazole

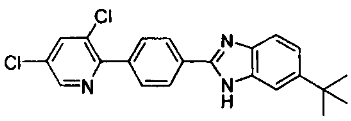

(1) Preparation of 4-(3,5-dichloropyridin-2-yl)benzoic acid

The same procedure as in (1) of Example 1 was performed to produce 4-(3,5-dichloropyridin-2-yl)benzoic acid (yield 79%).
$^1$H NMR ($CD_3OD$) δ : 8.74 (s, 1H), 8.27-8.24 (m, 3H), 7.92 (d, 2H)

(2) Preparation of 6-*tert*-butyl-2-[4-(3,5-dichloropyridin-2-yl)phenyl]-1H-benzoimidazole

The same procedure as in (3) of Example 1 was performed to produce 6-*tert*-butyl-2-[4-(3,5-dichloropyridin-2-yl)phenyl]-1H-benzoimidazole (yield 90%).
$^1H$ NMR ($CD_3OD$) δ : 8.62 (s, 1H), 8.20 (d, 2H), 8.15 (s, 1H), 7.88 (d, 2H), 7.64 (s, 1H), 7.56 (s, 1H), 7.41 (d, 1H), 1.42 (s, 9H)

EXAMPLE 26: Preparation of 2-[4-(3,5-dichloropyridin-2-yl)phenyl]-4,6-bistrifluoromethyl-1H-benzoimidazole

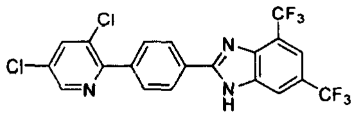

The same procedure as in (2) of Example 25 was performed to produce 2-[4-(3,5-dichloropyridin-2-yl)phenyl]-4,6-bistrifluoromethyl-1H-benzoimidazole (yield 72%).
$^1H$ NMR ($CD_3OD$) δ : 8.64 (d, 1H), 8.34 (d, 1H), 8.17-8.10 (m, 2H), 7.94 (d, 2H), 7.87-7.80 (m, 2H)

EXAMPLE 27: Preparation of 6-*tert*-butyl-2-[4-(3-chloro-5-trifluoromethylpyridin-2-yl)phenyl]-1H-benzoimidazole

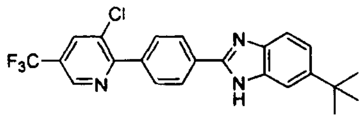

(1) Preparation of 4-(3-chloro-5-trifluoromethylpyridin-2-yl)benzoic acid

The same procedure as in (1) of Example 1 was performed to produce 4-(3-chloro-5-trifluoromethylpyridin-2-yl)benzoic acid (yield 75%).
$^1H$ NMR ($CD_3OD$) δ : 9.05 (d, 1H), 8.51 (d, 1H), 8.28 (dd, 2H), 7.97 (dd, 2H)

(2) Preparation of 6-*tert*-butyl-2-[4-(3-chloro-5-trifluoromethylpyridin-2-yl)phenyl]-1H-benzoimidazole

The same procedure as in (3) of Example 1 was performed to produce 6-*tert*-butyl-2-[4-(3-chloro-5-trifluoromethylpyridin-2-yl)phenyl]-1H-benzoimidazole (yield 87%).
$^1H$ NMR ($CD_3OD$) δ : 8.93 (s, 1H), 8.38 (s, 1H), 8.23 (d, 2H), 7.95 (d, 2H), 7.69-7.50 (m, 2H), 7.44-7.39 (m, 1H), 1.42 (s, 9H)

EXAMPLE 28: Preparation of 2-[4-(3-chloro-5-trifluoromethylpyridin-2-yl)phenyl]-4,6-bistrifluoromethyl-1H-benzoimidazole and 2-[4-(3-chloro-5-trifluoromethylpyridin-2-yl)phenyl]-5,7-bistrifluoromethyl-1H-benzoimidazole

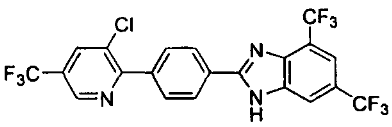

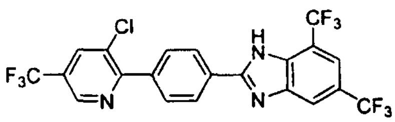

The same procedure as in (2) of Example 27 was performed to produce 2-[4-(3-chloro-5-trifluoromethylpyridin-2-yl)phenyl]-4,6-bistrifluoromethyl-1H-benzoimidazole and 2-[4-(3-chloro-5-trifluoromethylpyridin-2-yl)phenyl]-5,7-bistrifluoromethyl-1H-benzoimidazole (yield 68%).
$^1H$ NMR ($CD_3OD$) δ : 8.95 (d, 0.7H), 8.92 (d, 0.3H), 8.40-8.35 (m, 2.0H), 8.18 (s, 0.7H), 8.16 (s, 0.3H), 8.01-7.99 (m, 1.0H), 7.94-7.90 (m, 1H), 7.87 (d, 0.3H), 7.85 (d, 0.7H), 7.44 (s, 0.3H), 7.39 (d, 0.7H)

EXAMPLE 29: Preparation of N-[4'-(4,6-bistrifluoromethyl-1H-benzoimidazole-2-yl)-3-fluorobiphenyl-4-yl]methanesulfonamide

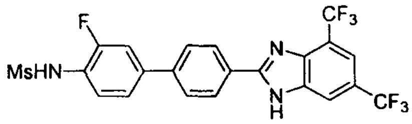

(1) Preparation of 4-(3-fluoro-4-methanesulfonylaminophenyl)benzoic acid

The same procedure as in (1) of Example 1 was performed to produce 4-(3-fluoro-4-methanesulfonylaminophenyl)benzoic acid (yield 58%).
$^1H$ NMR ($CD_3OD$) δ : 8.22 (dd, 2H), 7.87 (dd, 2H), 7.77-7.69 (m, 3H), 3.18 (s, 3H)

(2) Preparation of N-[4'-(4,6-bistrifluoromethyl-1H-benzoimidazole-2-yl)-3-fluorobiphenyl-4-yl]methanesulfonamide

The same procedure as in (3) of Example 1 was performed to produce N-[9'-(4,6-bistrifluoromethyl-1H-benzoimidazole-2-yl)-3-fluorobiphenyl-4-yl]methanesulfonamide (yield 62%).
$^1H$ NMR ($CD_3OD$) δ: 8.13 (d, 2H), 7.83 (d, 2H), 7.72 (s, 1H), 7.68-7.62 (m, 2H), 7.59-7.55 (m, 2H), 3.07 (s, 3H)

EXAMPLE 30: Preparation of 6-*tert*-butyl-2-[4-(3-trifluoromethylpyridin-2-yl)phenyl]-1H-benzoimidazole

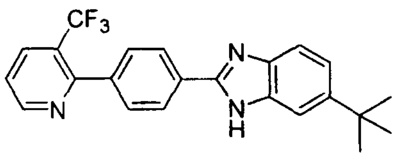

(1) Preparation of 4-(3-trifluoromethylpyridin-2-yl)benzoic acid

The same procedure as in (1) of Example 1 was performed to produce 4-(3-trifluoromethylpyridin-2-yl)benzoic acid (yield 84%).
$^1H$ NMR ($CD_3OD$) δ: 8.84 (d, 1H), 8.30 (d, 1H), 8.11 (d, 2H), 7.68-7.64 (m, 1H), 7.56 (d, 2H)

(2) Preparation of 6-tert-butyl-2-[4-(3-trifluoromethylpyridin-2-yl)phenyl]-1H-benzoimidazole

The same procedure as in (3) of Example 1 was performed to produce 6-tert-butyl-2-[4-(3-trifluoromethylpyridin-2-yl)phenyl]-1H-benzoimidazole (yield 93%).
$^1H$ NMR ($CDCl_3$) δ : 8.87 (d, 1H), 8.13 (s, 1H), 8.11 (d, 2H), 7.66 (s, 1H), 7.62 (d, 2H), 7.61 (s, 1H), 7.50-7.47 (m, 1H), 7.36 (d, 1H), 1.39 (s, 9H)

EXAMPLES 31 TO 40

The same procedure as in (3) of Example 1 was performed to produce compounds 31 to 40.

| Cpds. | Structures | Yield | Spectrum Data ($^1H$ NMR) |
|---|---|---|---|
| 31 | 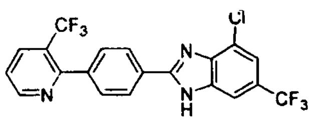 | 77 | ($CDCl_3$) δ : 8.92 (d, 1H), 8.23 (d, 2H), 8.16 (d, 1H), 8.00 (s, 1H), 7.67 (d, 2H), 7.62 (s, 1H), 7.58-7.52 (m, 1H) |

(continued)

| Cpds. | Structures | Yield | Spectrum Data (¹H NMR) |
|---|---|---|---|
| 32 | 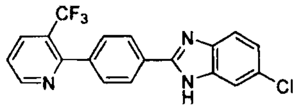 | 88 | ($CDCl_3$) δ : 8.86 (d, 1H), 8.20-8.11 (m, 3H), 7.70-7.58 (m, 4H), 7.52-7.48 (m, 1H), 7.29-7.27 (m, 1H) |
| 33 | 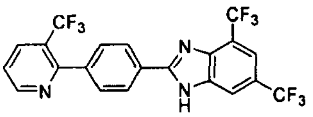 | 75 | ($CD_3OD$) δ : 8.90 (d, 1H), 8.38 (d, 2H), 8.19 (d, 1H), 7.84 (s, 1H), 7.74 (d, 2H), 7.70-7.65 (m, 2H) |
| 34 | 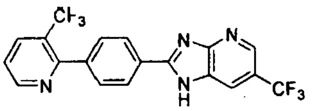 | 63 | ($CDCl_3$) δ : 8.95 (d, 1H), 8.72 (s, 1H), 8.41 (s, 1H), 8.26 (d, 2H), 8.18 (d, 1H), 7.74 (d, 2H), 7.58-7.53 (m, 1H) |
| 35 | 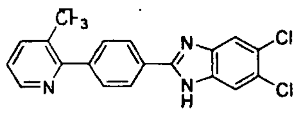 | 92 | ($CDCl_3$) δ : 8.91 (d, 1H), 8.14 (d, 1H), 8.06 (d, 2H), 7.79-7.73 (m, 2H), 7.65 (d, 2H), 7.51 (dd, 1H) |
| 36 | 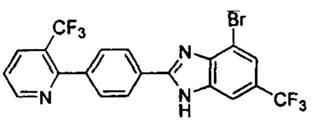 | 76 | ($CDCl_3$) δ : 8.95 (d, 1H), 8.19-8.12 (m, 3H), 7.96 (s, 1H), 7.73 (s, 1H), 7.65 (d, 2H), 7.57-7.53 (m, 1H) |
| 37 | 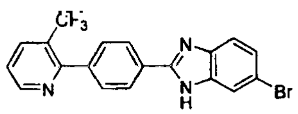 | 72 | ($CDCl_3$) δ : 8.90 (d, 1H), 8.30 (d, 2H), 8.12 (d, 1H), 7.91 (s, 1H), 7.64-7.58 (m, 3H), 7.49 (dd, 1H), 7.43 (dd, 1H) |
| 38 | 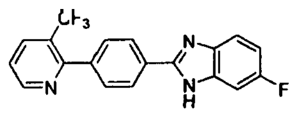 | 93 | ($CD_3OD$) δ : 8.86 (dd, 1H), 8.30 (dd, 1H), 8.19 (dd, 2H), 7.68-7.60 (m, 3H), 7.55 (dd, 1H), 7.32 (dd, 1H), 7.07 (dd, 1H) |
| 39 | 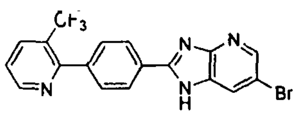 | 61 | ($CDCl_3$) δ : 8.93 (d, 1H), 8.45 (s, 1H), 8.29 (d, 2H), 8.26 (s, 1H), 8.15 (dd, 1H), 7.72 (d, 2H), 7.52 (dd, 1H) |
| 40 | 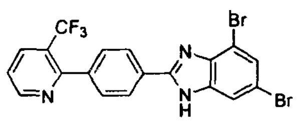 | 65 | ($CD_3OD$) δ : 8.85 (dd, 1H), 8.35-8.25 (m, 3H), 7.73 (br, 1H), 7.69-7.60 (m, 3H), 7.53 (d, 1H) |

EXAMPLE 41: Preparation of 6-morpholin-4-yl-2-[4-(3-trifluoromethylpyridin-2-yl)phenyl]-1H-benzoimidazole

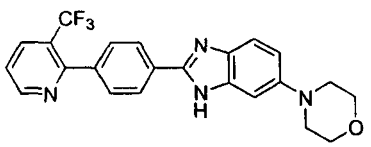

The same procedure as in Example 17 was performed to produce 6-morpholin-4-yl-2-[4-(3-trifluoromethylpyridin-2-yl)phenyl]-1H-benzoimidazole (yield 73%).

¹H NMR ($CD_3OD$) δ : 8.85 (d, 1H), 8.32 (dd, 1H), 8.17 (d, 2H), 7.69-7.62 (m, 3H), 7.58 (d, 1H), 7.14 (d, 1H), 7.09 (dd, 1H), 3.83 (dt, 4H), 3.18 (dt, 4H)

EXAMPLES 42 TO 46

The same procedure as in Example 41 was performed to produce Compounds 42 to 46.

| Cpds. | Structures | Yield | Spectrum Data ($^1$H NMR) |
|---|---|---|---|
| 42 | 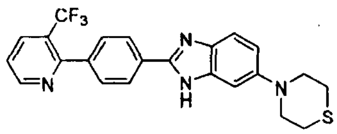 | 72 | ($CD_3OD$) δ : 8.84 (d, 1H), 8.29 (d, 1H), 8.17 (dd, 2H), 7.70-7.62 (m, 3H), 7.53 (d, 1H), 7.16 (d, 1H), 7.05 (dd, 1H), 3.48 (dt, 4H), 2.81 (dt, 4H) |
| 43 | 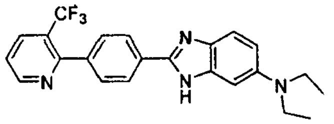 | 55 | ($CD_3OD$) δ : 8.84 (d, 1H), 8.32 (dd, 1H), 8.17 (d, 2H), 7.70-7.61 (m, 3H), 7.56 (d, 1H), 7.13 (d, 1H), 7.09 (dd, 1H), 3.11-3.02 (m, 4H), 1.05 (t, 6H) |
| 44 | 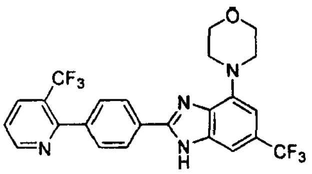 | 75 | ($CDCl_3$) δ : 8 95 (d, 1H), 8.16 (dd, 1H), 7.98 (d, 2H), 7.59-7.51 (m, 3H), 7.33 (s, 1H), 6.83 (s, 1H), 3.97 (dt, 4H), 3.65 (dt, 4H) |
| 45 | 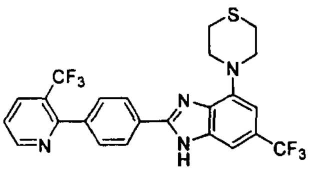 | 74 | ($CDCl_3$) δ : 8.92 (d, 1H), 8.15 (d, 1H), 8.09 (d, 2H), 7.61-7.51 (m, 3H), 7.45 (s, 1H), 6.97 (s, 1H), 3.90 (br, 4H), 2.90 (br, 4H) |
| 46 | 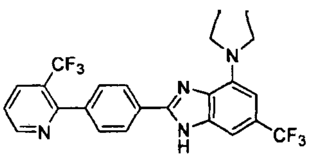 | 69 | ($CDCl_3$) δ : 8.89 (d, 1H), 8.12 (dd, 2H), 8.07 (br, 2H), 7.71 (d, 1H), 7.61 (dd, 2H), 7.49 (dd, 1H), 3.88 (br, 4H), 2.07 (br, 4H) |

EXAMPLE 47: Preparation of 6-*tert*-butyl-2-(4-pyridin-2-ylphenyl)-1H-benzoimidazole

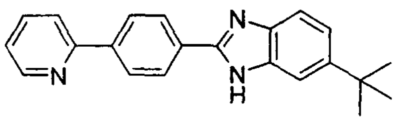

(1) Preparation of 4-(pyridin-2-yl)benzoic acid

The same procedure as in (1) of Example 1 was performed to produce 4-(pyridin-2-yl)benzoic acid (yield 80%).
$^1$H NMR ($CD_3OD$) δ : 8.66 (d, 1H), 8.14 (d, 2H), 8.07 (d, 2H), 7.94 (d, 2H), 7.43-7.40 (m, 1H)

(2) Preparation of 6-*tert*-butyl-2-(4-pyridin-2-ylphenyl)-1H-benzoimidazole

The same procedure as in (3) of Example 1 was performed to produce 6-tert-butyl-2-(4-pyridin-2-ylphenyl)-1H-benzoimidazole (yield 84%).
$^1$H NMR ($CD_3OD$) δ : 8.69 (d, 1H), 8.22 (s, 4H), 7.98 (dd, 2H), 7.69 (d, 1H), 7.64 (d, 1H), 7.55 (dd, 1H), 7.43 (dd, 1H), 1.41 (s, 9H)

EXAMPLES 48 TO 54

The same procedure as in (2) of Example 47 was performed to produce Compounds 48 to 54.

| Cpds. | Structures | Yield | Spectrum Data ($^1$H NMR) |
|---|---|---|---|
| 48 | 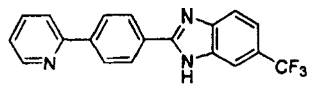 | 96 | ($CD_3OD$) δ : 8.66 (d, 1H), 8.24 (dd, 2H), 8.19 (dd, 2H), 7.98-7.93 (m, 3H), 7.78 (d, 1H), 7.57 (dd, 1H), 7.42 (dd, 1H) |
| 49 | 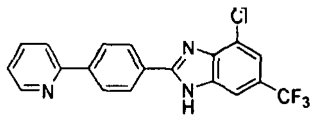 | 88 | ($CD_3OD$) δ : 8.67 (dd, 1H), 8.29 (dd, 2H), 8.19 (dd, 2H), 8.13 (s, 1H), 7.94 (dd, 2H), 7.56 (d, 1H), 7.41 (dd, 1H) |
| 50 | 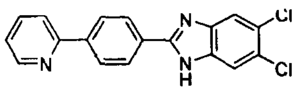 | 88 | ($CD_3OD$) δ : 8.67 (d, 1H), 8.24-8.16 (m, 4H), 7.97-7.92 (m, 2H), 7.78 (s, 2H), 7.42 (dd, 1H) |
| 51 | 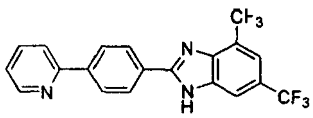 | 61 | ($CD_3OD$) δ : 8.65 (dd, 1H), 8.30 (dd, 2H), 8.15 (dd, 2H), 8.05 (d, 1H), 7.92 (dd, 2H), 7.78 (s, 1H), 7.39 (dd, 1H) |
| 52 | 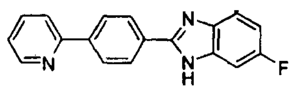 | 83 | ($CD_3OD$) δ : 8.67 (d, 1H), 8.23-8.15 (m, 4H), 7.97-7.94 (m, 2H), 7.61 (dd, 1H), 7.42 (dd, 1H), 7.33 (dd, 1H), 7.09 (dd, 1H) |
| 53 | 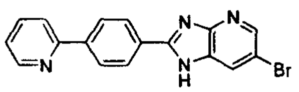 | 66 | ($CD_3OD$) δ : 8.66 (d, 1H), 8.27 (d, 1H), 8.18-8.12 (m, 3H), 8.07 (d, 2H), 7.95 (dd, 2H), 7.41 (dd, 1H) |
| 54 | 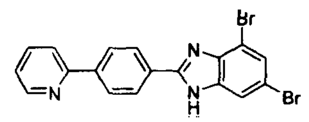 | 56 | ($CD_3OD$) δ : 8.55 (dd, 1H), 8.26 (d, 2H), 8.15 (dd, 2H), 7.96-7.90 (m, 2H), 7.68 (s, 1H), 7.58 (d, 1H), 7.41 (dd, 1H) |

EXAMPLE 55: Preparation of 2-[4-(6-chloro-5-methylpyridazin-3-yl)phenyl]-4,6-bistrifluoromethyl-1H-benzoimidazole-2-[4-(6-chloro-5-methylpyridazin-3-yl)phenyl]-5,7-bistrifluoromethyl-1H-benzoimidazole

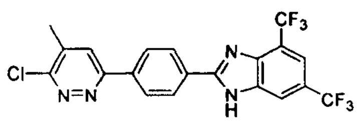

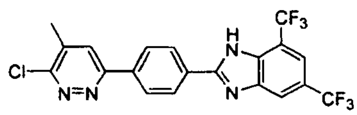

(1) Preparation of 4-(6-chloro-5-methylpyridazin-3-yl)benzoic acid

The same procedure as in (1) of Example 1 was performed to produce 4-(6-chloro-5-methylpyridazin-3-yl)benzoic acid (yield 52%).
$^1$H NMR ($CD_3OD$) δ : 8.20 (s, 1H), 8.20-8.16 (m, 4H), 2.53 (s, 3H)

(2) Preparation of 2-[4-(6-chloro-5-methylpyridazin-3-yl)phenyl]-4,6-bistrifluoromethyl-1H-benzoimidazole and 2-[9-(6-chloro-5-methylpyridazin-3-yl)phenyl]-5,7-bistrifluoromethyl-1H-benzoimidazole

The same procedure as in (3) of Example 1 was performed to produce 2-[9-(6-chloro-5-methylpyridazin-3-yl)phenyl]-4,6-bistrifluoromethyl-1H-benzoimidazole and 2-[4-(6-chloro-5-methylpyridazin-3-yl)phenyl]-5,7-bistrifluoromethyl-1H-benzoimidazole (yield 71%).
$^1$H NMR ($CD_3OD$) δ: 8.89 (d, 0.5H), 8.69 (d, 0.5H), 8.45-8.37 (m, 2.5H), 8.33-8.15 (m, 2.5H), 7.70-7.60 (m, 1.0H), 2.75 (s, 1.5H), 2.54 (s, 1.5H)

EXAMPLE 56: Preparation of 4-bromo-6-(trifluoromethyl)-2-(4-(4-(trifluoromethyl)pyrimidin-2-yl)phenyl)-1H-benzoimidazole

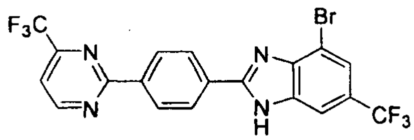

(1) Preparation of 4-(4-trifluoromethylpyrimidin-2-yl)benzoic acid

The same procedure as in (1) of Example 1 was performed to produce 4-(4-trifluoromethylpyrimidin-2-yl)benzoic acid (yield 77%).
$^1H$ NMR ($CD_3OD$) δ: 9.20 (d, 1H), 8.62 (dd, 2H), 8.19 (dd, 2H), 7.81 (d, 1H)

(2) Preparation of 4-bromo-6-(trifluoromethyl)-2-(4-(4-(trifluoromethyl)pyrimidin-2-yl)phenyl)-1H-benzoimidazole

The same procedure as in (3) of Example 1 was performed to produce 4-bromo-6-(trifluoromethyl)-2-(4-(4-(trifluoromethyl)pyrimidin-2-yl)phenyl)-1H-benzoimidazole (yield 72%).
$^1H$ NMR ($CDCl_3$) δ : 9.14 (d, 1H), 8.74 (d, 2H), 8.27 (d, 2H), 8.00 (s, 1H), 7.76 (s, 1H), 7.62 (d, 1H)

EXAMPLE 57: Preparation of 4,6-dibromo-2-[4-(4-trifluoromethylpyrimidin-2-yl)phenyl]-1H-benzoimidazole

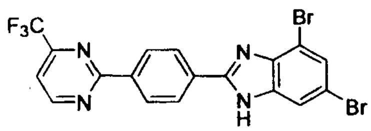

The same procedure as in (2) of Example 56 was performed to produce 4,6-dibromo-2-[4-(4-trifluoromethylpyrimidin-2-yl)phenyl]-1H-benzoimidazole (yield 64%).
$^1H$ NMR ($CD_3OD$) δ : 9.18 (d, 1H), 8.70 (dd, 1H), 8.56 (d, 2H), 8.36 (dd, 1H), 8.26 (s, 1H), 8.15 (d, 2H)

EXAMPLE 58: Preparation of 6-*tert*-butyl-2-(4-(pyrimidin-2-yl)phenyl)-1H-benzoimidazole

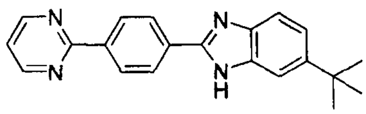

(1) Preparation of 4-(pyrimidin-2-yl)benzoic acid

The same procedure as in (1) of Example 1 was performed to produce 4-(pyrimidin-2-yl)benzoic acid (yield 74%).
$^1H$ NMR ($CD_3OD$) δ: 8.88 (d, 2H), 8.47 (d, 2H), 8.12 (d, 2H), 7.39 (t, 1H)

(2) Preparation of 6-*tert*-butyl-2-(4-(pyrimidin-2-yl)phenyl)-1H-benzoimidazole

The same procedure as in (3) of Example 1 was performed to produce 6-*tert*-butyl-2-(4-(pyrimidin-2-yl)phenyl)-1H-benzoimidazole (yield 92%).
$^1H$ NMR ($CD_3OD$) δ : 8.88 (d, 2H), 8.58 (dd, 2H), 8.21 (dd, 2H), 7.64 (d, 1H), 7.56 (d, 1H), 7.42-7.37 (m, 2H), 1.41 (s, 9H)

EXAMPLE 59: Preparation of 2-(4-(6-(trifluoromethyl)-1H-benzoimidazole-2-yl)phenyl)pyridin-3-carbonitrile

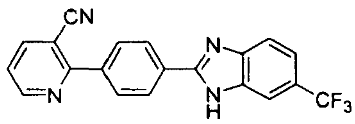

(1) Preparation of 4-(3-cyanopyridin-2-yl)benzoic acid

The same procedure as in (1) of Example 1 was performed to produce 4-(3-cyanopyridin-2-yl)benzoic acid (yield 59%).
$^1H$ NMR ($CD_3OD$) δ : 9.04 (dd, 1H), 8.46 (dd, 1H), 8.32 (d, 2H), 8.12 (d, 2H), 7.74 (dd, 1H)

(2) Preparation of 2-(4-(6-(trifluoromethyl)-1H-benzoimidazole-2-yl)phenyl)pyridin-3-carbonitrile

The same procedure as in (3) of Example 1 was performed to produce 2-(4-(6-(trifluoromethyl)-1H-benzoimidazole-2-yl)phenyl)pyridin-3-carbonitrile (yield 76%).
$^1H$ NMR ($CD_3OD$) δ : 8.89 (dd, 1H), 8.32-8.26 (m, 3H), 8.08 (dd, 2H), 7.90 (s, 1H), 7.75 (d, 1H), 7.60-7.53 (m, 2H)

EXAMPLES 60 AND 61

The same procedure as in (2) of Example 59 was performed to produce Compouds 60 and 61.

| Cpds. | Structures | Yield | Spectrum Data ($^1H$ NMR) |
|---|---|---|---|
| 60 | 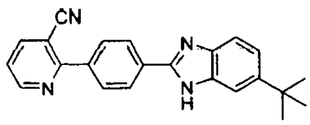 | 82 | ($CD_3OD$) δ : 8.89 (dd, 1H), 8.30 (dd, 1H), 8.25 (d, 2H), 8.07 (d, 2H), 7.64 (d, 1H), 7.59-7.55 (m, 2H), 7.42 (dd, 1H), 1.41 (s, 9H) |
| 61 | 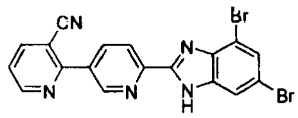 | 55 | ($CD_3OD$) δ : 8.88 (d, 1H), 8.31 (dd, 1H), 7.98 (d, 2H), 7.75 (d, 2H), 7.57 (dd, 1H), 7.53 (d, 1H), 7.50 (d, 1H) |

EXAMPLE 62: Preparation of 2-[4-(3-chloropyrazin-2-yl)phenyl]-6-trifluoromethyl-1H-benzoimidazole

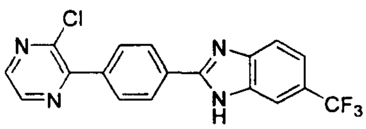

(1) Preparation of 4-(3-chloropyrazin-2-yl)benzoic acid

The same procedure as in (1) of Example 1 was performed to produce 4-(3-chloropyrazin-2-yl)benzoic acid (yield 79%).
$^1H$ NMR ($CD_3OD$) δ : 8.72 (d, 1H), 8.50 (d, 1H), 8.20 (d, 2H), 7.93 (d, 2H)

(2) Preparation of 2-[4-(3-chloropyrazin-2-yl)phenyl]-6-trifluoromethyl-1H-benzoimidazole

The same procedure as in (3) of Example 1 was performed to produce 2-[4-(3-chloropyrazin-2-yl)phenyl]-6-trifluoromethyl-1H-benzoimidazole (yield 96%).
$^1H$ NMR ($CDCl_3$) δ : 8.62 (d, 1H), 8.39 (d, 1H), 8.20 (d, 2H), 7.99 (s, 1H), 7.97 (d, 2H), 7.71 (s, 1H), 7.52 (dd, 1H)

EXAMPLES 63 AND 66

The same procedure as in (2) of Example 62 was performed to produce Compounds 63 to 66.

| Cpds. | Structures | Yield | Spectrum Data ($^1H$ NMR) |
|---|---|---|---|
| 63 | 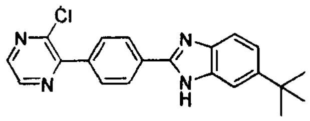 | 91 | ($CDCl_3$) δ : 8.58 (d, 1H), 8.35 (d, 1H), 8.21 (d, 2H), 7.91 (d, 2H), 7.66 (s, 1H), 7.58 (d, 1H), 7.36 (dd, 1H), 1.36 (s, 9H) |
| 64 | 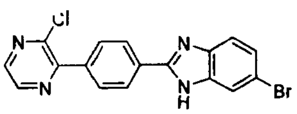 | 69 | ($CDCl_3$) δ : 8.62 (d, 1H), 8.40 (dd, 1H), 8.15 (dd, 2H), 7.99 (dd, 2H), 7.80 (s, 1H), 7.50 (s, 1H), 7.40 (dd, 1H) |
| 65 | 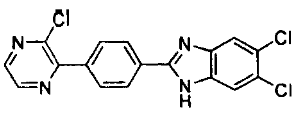 | 88 | ($CDCl_3$) 6 : 8.62 (d, 1H), 8.40 (dd, 1H), 8.12 (dd, 2H), 7.98 (dd, 2H), 7.92 (dd, 1H), 7.67 (s, 1H) |
| 66 | 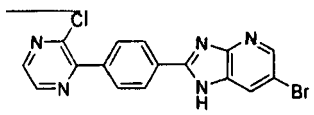 | 58 | ($CDCl_3$) δ : 8.60 (d, 1H), 8.37 (d, 1H), 8.09 (dd, 1H), 7.84 (dd, 2H), 7.51 (dd, 1H), 7.44 (dd, 2H) |

EXAMPLE 67: Preparation of 6-tert-butyl-2-[4-(3-chloropyridin-2-yl)-2-fluorophenyl]-1H-benzoimidazole

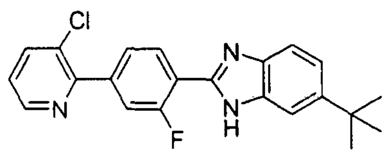

(1) Preparation of 4-(3-chloropyridin-2-yl)-2-fluorobenzoic acid

The same procedure as in (1) of Example 1 was performed to produce 4-(3-chloropyridin-2-yl)-2-fluorobenzoic acid (yield 77%).
$^1H$ NMR ($CD_3OD$) δ : 8.51 (d, 1H), 7.99-7.93 (m, 2H), 7.52-7.48 (m, 2H), 7.42-7.36 (m, 1H), 1.42 (s, 9H)

(2) Preparation of 6-tert-butyl-2-[4-(3-chloropyridin-2-yl)-2-fluorophenyl]-1H-benzoimidazole

The same procedure as in (3) of Example 1 was performed to produce 6-*tert*-butyl-2-[4-(3-chloropyridin-2-yl)-2-fluorophenyl]-1H-benzoimidazole (yield 91%).
$^1H$ NMR ($CD_3OD$) δ : 8.62 (d, 1H), 8.05 (dd, 1H), 8.03 (d, 1H), 7.74-7.70 (m, 2H), 7.66 (s, 1H), 7.47-7.43 (m, 2H)

EXAMPLES 68 AND 69

The same procedure as in (2) of Example 27 was performed to produce Compounds 68 and 69.

| Cpds. | Structures | Yield | Spectrum Data ($^1H$ NMR) |
|---|---|---|---|
| 68 | 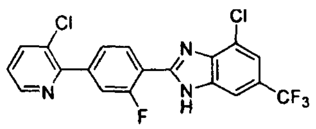 | 69 | ($CD_3OD$) δ : 8.62 (d, 1H), 8.50-8.45 (m, 1H), 8.05 (d, 1H), 7.92 (s, 1H), 7.79-7.70 (m, 2H), 7.61 (s, 1H), 7.50-7.45 (m, 1H) |

(continued)

| Cpds. | Structures | Yield | Spectrum Data ($^1$H NMR) |
| --- | --- | --- | --- |
| 69 | 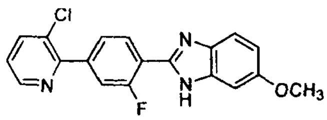 | 93 | ($CD_3OD$) δ : 8.62 (d, 1H), 8.28 (dd, 1H), 8.05 (d, 1H), 7.73 (d, 1H), 7.66 (s, 1H), 7.56-7.52 (m, 1H), 7.49-7.45 (m, 1H), 7.10 (s, 1H), 6.96 (dd, 1H), 3.88 (s, 3H) |

EXAMPLE 70: Preparation of 6-tert-butyl-2-[4-(3,5-dichloropyridin-2-yl)-2-fluorophenyl]-1H-benzoimidazole

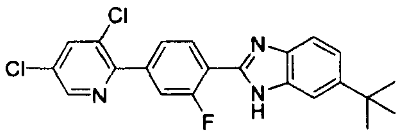

(1) Preparation of 4-(3,5-dichloropyridin-2-yl)-2-fluorobenzoic acid

The same procedure as in (1) of Example 1 was performed to produce 4-(3,5-dichloropyridin-2-yl)-2-fluorobenzoic acid (yield 69%).
$^1$H NMR ($CD_3OD$) δ : 8.55 (s, 1H), 8.07 (s, 1H), 7.93 (dd, 1H), 7.59-7.52 (m, 2H)

(2) Preparation of 6-*tert*-butyl-2-[4-(3,5-dichloropyridin-2-yl)-2-fluorophenyl]-1H-benzoimidazole

The same procedure as in (3) of Example 1 was performed to produce 6-tert-butyl-2-[4-(3,5-dichloropyridin-2-yl)-2-fluorophenyl]-1H-benzoimidazole (yield 88%).
$^1$H NMR ($CD_3OD$) δ : 8.64 (s, 1H), 8.27 (d, 1H), 8.16 (s, 1H), 77.72-7.68 (m, 3H), 7.60 (d, 1H), 7.45 (d, 1H), 1.42 (s, 9H)

EXAMPLES 71 AND 72

The same procedure as in (2) of Example 70 was performed to produce Compounds 71 and 72.

| Cpds. | Structures | Yield | Spectrum Data ($^1$H NMR) |
| --- | --- | --- | --- |
| 71 | 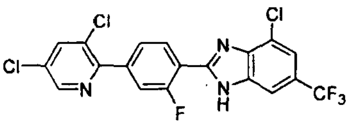 | 57 | ($CD_3OD$) δ : 8.65 (s, 1H), 8.45-8.39 (m, 1H), 8.18 (s, 1H), 7.91 (s, 1H), 7.85-7.78 (m, 2H), 7.61 (s, 1H) |
| 72 | 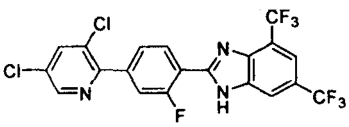 | 75 | ($CD_3OD$) δ : 8.66 (s, 1H), 8.65-8.49 (m, 1H), 8.20-8.15 (m, 2H), 7.82-7.74 (m, 3H) |

EXAMPLE 73: Preparation of 4-chloro-2-[4-(3-chloro-5-trifluoromethylpyridin-2-yl)-2-fluorophenyl]-6-trifluoromethyl-1H-benzoimidazole

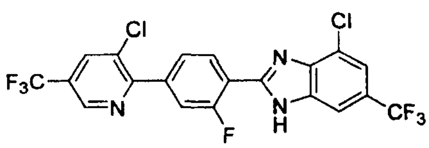

(1) Preparation of 4-(3-chloro-5-trifluoromethylpyridin-2-yl)-2-fluorobenzoic acid

The same procedure as in (1) of Example 1 was performed to produce 4-(3-chloro-5-trifluoromethylpyridin-2-yl)-2-fluorobenzoic acid (yield 72%).
$^1H$ NMR ($CD_3OD$) δ : 8.85 (d, 1H), 8.31 (d, 1H), 7.98 (dd, 1H), 7.60-7.51 (m, 2H)

(2) Preparation of 4-chloro-2-[4-(3-chloro-5-trifluoromethylpyridin-2-yl)-2-fluorophenyl]-6-trifluoromethyl-1H-benzoimidazole

The same procedure as in (3) of Example 1 was performed to produce 4-chloro-2-[4-(3-chloro-5-trifluoromethylpyridin-2-yl)-2-fluorophenyl]-6-trifluoromethyl-1H-benzoimidazole (yield 81%).
$^1H$ NMR ($CD_3OD$) δ : 8.96 (s, 1H), 8.42 (s, 2H), 7.92 (s, 1H), 7.88-7.82 (m, 2H), 7.62 (s, 1H)

EXAMPLES 74 AND 75

The same procedure as in (2) of Example 73 was performed to produce Compounds 74 and 75.

| pds. | Structures | yield | Spectrum Data ($^1H$ NMR) |
|---|---|---|---|
| 74 | 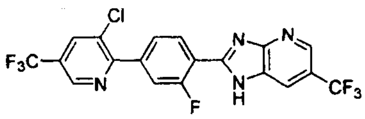 | 59 | ($CD_3OD$) δ : 8.97 (s, 1H), 8.76 (s, 1H), 8.43 (d, 2H), 8.36 (s, 1H), 7.89-7.83 (m, 2H) |
| 75 | 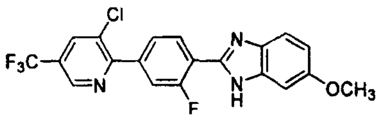 | 90 | ($CD_3OD$) δ : 8.95 (s, 1H), 8.41 (s, 1H), 8.31-8.27 (m, 1H), 7.83-7.78 (m, 2H), 7.57 (d, 1H), 7.16 (d, 1H), 6.95 (d, 1H), 3.88(s, 3H) |

EXAMPLE 76: Preparation of N-[3,3'-difluoro-4'-(6-methoxy-1H-benzoimidazole-2-yl)biphenyl-4-yl]methanesulfonamide

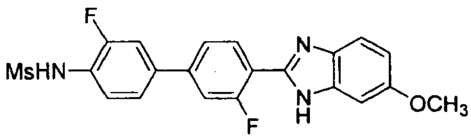

(1) Preparation of 4-(3-fluoro-4-methanesulfonylaminophenyl)-2-fluorobenzoic acid

The same procedure as in (1) of Example 1 was performed to produce 4-(3-fluoro-4-methanesulfonylaminophenyl)-2-fluorobenzoic acid (yield 52%).
$^1H$ NMR ($CD_3OD$) δ : 7.94-7.91 (m, 1H), 7.56-7.52 (m, 2H), 7.49-7.41 (m, 3H), 2.98 (s, 3H)

(2) Preparation of N-[3,3'-difluoro-4'-(6-methoxy-1H-benzoimidazole-2-yl)biphenyl-4-yl]methanesulfonamide

The same procedure as in (3) of Example 1 was performed to produce N-[3,3'-difluoro-4'-(6-methoxy-1H-benzoimidazole-2-yl)biphenyl-4-yl]methanesulfonamide (yield 55%).
$^1H$ NMR ($CD_3OD$) δ : 7.73 (d, 1H), 7.71 (d, 1H), 7.58 (s, 1H), 7.55-7.52 (m, 3H), 7.50-7.48 (m, 1H), 7.28-7.25 (m, 1H), 6.85 (d, 1H), 3.86 (s, 3H), 2.99 (s, 3H)

EXAMPLE 77 Preparation of 2-[2-fluoro-4-(3-trifluoromethylpyridin-2-yl)phenyl]-6-trifluoromethyl-1H-benzoimidazole

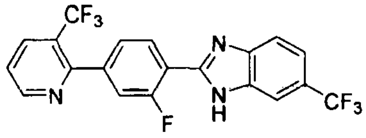

(1) Preparation of 2-fluoro-4-(3-trifluoromethylpyridin-2-yl)benzoic acid

The same procedure as in (1) of Example 1 was performed to produce 2-fluoro-4-(3-trifluoromethylpyridin-2-yl)benzoic acid (yield 71%).
$^1$H NMR ($CD_3OD$) δ : 8.85 (d, 1H), 8.30 (d, 1H), 8.00 (dd, 1H), 7.68 (dd, 1H), 7.38-7.31 (m, 2H)

(2) Preparation of 2-[2-fluoro-4-(3-trifluoromethylpyridin-2-yl)phenyl]-6-trifluoromethyl-1H-benzoimidazole

The same procedure as in (3) of Example 1 was performed to produce 2-[2-fluoro-4-(3-trifluoromethylpyridin-2-yl)phenyl]-6-trifluoromethyl-1H-benzoimidazole (yield 83%).
$^1$H NMR ($CD_3OD$) δ : 8.89 (d, 1H), 8.36-8.32 (m, 2H), 8.00 (s, 1H), 7.83 (d, 1H), 7.72-7.68 (m, 1H), 7.60 (d, 1H), 7.55 (s, 1H), 7.52 (d, 1H)

EXAMPLES 78 AND 80

The same procedure as in (2) of Example 77 was performed to produce Compounds 78 to 80.

| Cpds. | Structures | Yield | Spectrum Data ($^1$H NMR) |
|---|---|---|---|
| 78 | 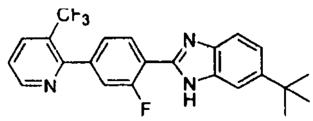 | 94 | ($CD_3OD$) δ : 8.88 (d, 1H), 8.34-8.26 (m, 2H), 7.70-7.65 (m, 2H), 7.61 (d, 1H), 7.51-7.42 (m, 3H), 1.43 (s, 9H) |
| 79 | 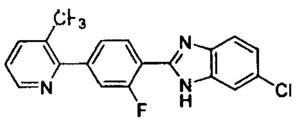 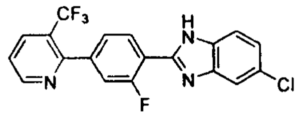 | 89 | ($CD_3OD$) δ : 8.89 (d, 0.6H), 8.84 (d, 0.4H), 8.47-8.43 (m, 0.6H), 8.35-8.28 (m, 1.0H), 8.25-8.20 (m, 0.4H), 7.95-7.88 (m, 1.6H), 7.73-7.42 (m, 3.0H), 7.42 (d, 0.4H), 7.34-7.26 (m, 1.0H) |
| 80 | 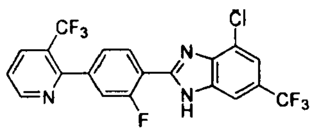 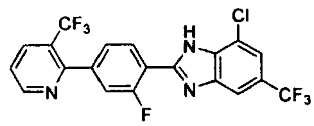 | 67 | ($CD_3OD$) δ : 8.89 (d, 0.6H), 8.84 (d, 0.4H), 8.49-8.45 (m, 0.6H), 8.35-8.30 (m, 1.0H), 7.95-7.90 (m, 1.0H), 7.68-7.41 (m, 3.4H), 7.33-7.26 (m, 1.0H) |

EXAMPLE 81: Preparation of 2-(2-fluoro-4-pyridin-2-yl)phenyl]-6-trifluoromethyl-1H-benzoimidazole

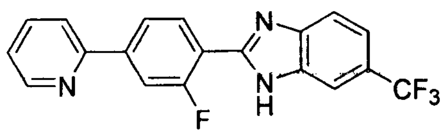

(1) Preparation of 2-fluoro-4-pyridin-2-ylbenzoic acid

The same procedure as in (1) of Example 1 was performed to produce 2-fluoro-4-pyridin-2-ylbenzoic acid (yield 69%).
$^1$H NMR ($CD_3OD$) δ : 7.79-7.74 (m, 2H), 7.46-7.34 (m, 3H), 7.11-7.02 (m, 2H)

(2) Preparation of 2-(2-fluoro-4-pyridin-2-yl)phenyl]-6-trifluoromethyl-1H-benzoimidazole

The same procedure as in (3) of Example 1 was performed to produce 2-(2-fluoro-4-pyridin-2-yl)phenyl]-6-trifluoromethyl-1H-benzoimidazole (yield 82%).
$^1$H NMR ($CDCl_3$) δ : 8.77-8.71 (m, 1H), 8.06-7.90 (m, 3H), 7.89-7.83 (m, 2H), 7.62-7.51 (m, 4H)

EXAMPLES 82 AND 84

The same procedure as in (2) of Example 81 was performed to produce Compounds 82 to 84.

| Cpds. | Structures | Yield | Spectrum Data ($^1$H NMR) |
|---|---|---|---|
| 82 | 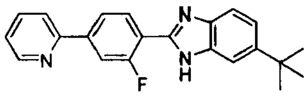 | 88 | ($CDCl_3$) δ : 8.90 (d, 1H), 8.65 (d, 1H), 7.92-7.80 (m, 6H), 7.52 (d, 2H), 1.25 (s, 9H) |
| 83 | 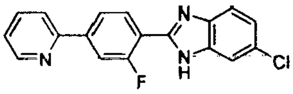 | 79 | ($CDCl_3$) δ : 8.74 (d, 1H), 8.02-7.97 (m, 2H), 7.88-7.82 (m, 2H), 7.70-7.65 (m, 1H), 7.34-7.27 (m, 4H) |
| 84 | 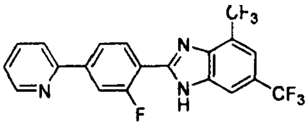 | 72 | ($CDCl_3$) δ : 8.22-8.18 (m, 1H), 8.08-8.04 (m, 1H), 7.82-7.79 (m, 1H), 7.63 (s, 1H), 7.55 (d, 1H), 7.39-7.26 (m, 4H) |

EXAMPLE 85: Preparation of 2-[2-chloro-4-(3-chloropyridin-2-yl)phenyl]-6-trifluoromethyl-1H-benzoimidazole

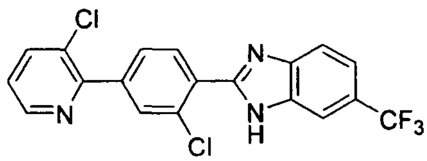

(1) Preparation of 2-chloro-4-(3-chloropyridin-2-yl)benzoic acid

The same procedure as in (1) of Example 1 was performed to produce 2-chloro-4-(3-chloropyridin-2-yl)benzoic acid (yield 80%).
$^1$H NMR ($CD_3OD$) δ : 8.60-8.58 (m, 1H), 8.02 (dd, 1H), 7.95 (d, 1H), 7.79 (d, 1H), 7.69 (dd, 1H), 7.48-7.44 (m, 1H)

(2) Preparation of 2-[2-chloro-4-(3-chloropyridin-2-yl)phenyl]-6-trifluoromethyl-1H-benzoimidazole

The same procedure as in (3) of Example 1 was performed to produce 2-[2-chloro-4-(3-chloropyridin-2-yl)phenyl]-6-trifluoromethyl-1H-benzoimidazole (yield 91%).
$^1$H NMR ($CD_3OD$) δ : 8.63 (d, 1H), 8.05-7.98 (m, 3H), 7.95 (s, 1H), 7.85 (d, 1H), 7.82 (s, 1H), 7.60 (d, 1H), 7.48-7.44 (m, 1H)

EXAMPLES 86 AND 87

The same procedure as in (2) of Example 85 was performed to produce Compounds 86 and 87.

| Cpds. | Structures | Yield | Spectrum Data ($^1H$ NMR) |
|---|---|---|---|
| 86 | 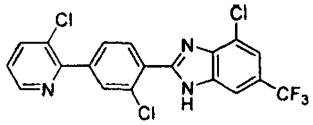 | 79 | ($CDCl_3$) δ : 8.78 (d, 1H), 8.25 (s, 1H), 8.22-8.17 (m, 1H), 7.98 (s, 1H), 7.97-7.92 (m, 2H), 7.77 (s, 1H), 7.60-7.55 (m, 1H) |
| 87 | 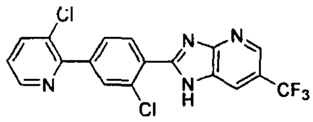 | 58 | ($CDCl_3$) δ : 8.77 (s, 1H), 8.68-8.65 (m, 1H), 8.49 (s, 1H), 7.99-7.89 (m, 4H), 7.45-7.38 (m, 1H) |

EXAMPLE 88: Preparation of 2-[2-chloro-4-(3,5-dichloropyridin-2-yl)phenyl]-6-trifluoromethyl-1H-benzoimidazole

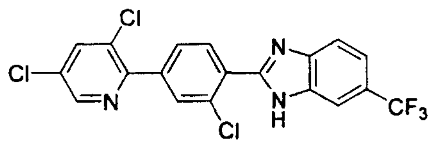

(1) Preparation of 2-chloro-9-(3,5-dichloropyridin-2-yl)benzoic acid

The same procedure as in (1) of Example 1 was performed to produce 2-chloro-4-(3,5-dichloropyridin-2-yl)benzoic acid (yield 72%).
$^1H$ NMR ($CD_3OD$) δ : 8.62 (d, 1H), 8.15 (d, 1H), 7.93 (d, 1H), 7.82 (d, 1H), 7.72 (dd, 1H)

(2) Preparation of 2-[2-chloro-4-(3,5-dichloropyridin-2-yl)phenyl]-6-trifluoromethyl-1H-benzoimidazole

The same procedure as in (3) of Example 1 was performed to produce 2-[2-chloro-4-(3,5-dichloropyridin-2-yl)phenyl]-6-trifluoromethyl-1H-benzoimidazole (yield 88%).
$^1H$ NMR ($CD_3OD$) δ : 8.63 (d, 1H), 8.16 (d, 1H), 8.03-7.95 (m, 3H), 7.87-7.82 (m, 2H), 7.59 (d, 1H)

EXAMPLES 89 AND 91

The same procedure as in (2) of Example 88 was performed to produce Compounds 89 to 91.

| Cpds. | Structures | Yield | Spectrum Data ($^1H$ NMR) |
|---|---|---|---|
| 89 | 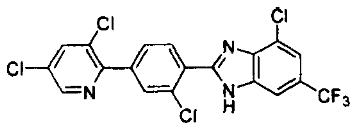 | 76 | ($CDCl_3$) δ : 8.61-8.58 (m, 2H), 8.03 (s, 1H), 7.93-7.87 (m, 3H), 7.64 (s, 1H) |
| 90 | 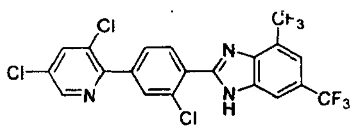 | 71 | ($CDCl_3$) δ : 8.66 (d, 1H), 8.61 (s, 1H), 8.32 (s, 1H), 7.98-7.86 (m, 4H) |
| 91 | 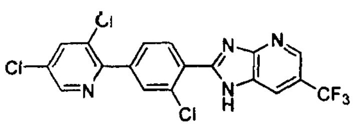 | 62 | ($CDCl_3$) δ : 8.78 (s, 1H), 8.63-8.55 (m, 2H), 8.50 (s, 1H), 7.97 (s, 1H), 7.93-7.83 (m, 2H) |

EXAMPLE 92: Preparation of 2-[2-chloro-4-(3-chloro-5-trifluoromethylpyridin-2-yl)phenyl]-6-trifluoromethyl-1H-benzoimidazole

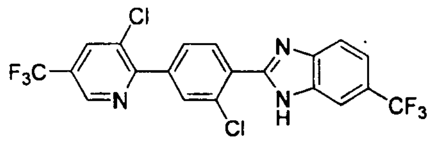

(1) Preparation of 2-chloro-4-(3-chloro-5-trifluoromethylpyridin-2-yl)benzoic acid

The same procedure as in (1) of Example 1 was performed to produce 2-Chloro-4-(3-chloro-5-trifluoromethylpyridin-2-yl)benzoic acid (yield 68%).
$^1H$ NMR ($CD_3OD$) δ: 8.92 (d, 1H), 8.38 (d, 1H), 7.96 (d, 1H), 7.87 (d, 1H), 7.77 (dd, 1H)

(2) Preparation of 2-[2-chloro-4-(3-chloro-5-trifluoromethylpyridin-2-yl)phenyl]-6-trifluoromethyl-1H-benzoimidazole

The same procedure as in (3) of Example 1 was performed to produce 2-[2-chloro-4-(3-chloro-5-trifluoromethylpyridin-2-yl)phenyl]-6-trifluoromethyl-1H-benzoimidazole (yield 88%).
$^1H$ NMR ($CDCl_3$) 8.85 (s, 1H), 8.37 (d, 1H), 8.08 (s, 1H), 8.01 (s, 1H), 7.94 (s, 1H), 7.88 (d, 1H), 7.78 (d, 1H), 7.56 (d, 1H)

EXAMPLE 93: Preparation of 4-chloro-2-[2-chloro-4-(3-chloro-5-trifluoromethylpyridin-2-yl)phenyl]-6-trifluoromethyl-1H-benzoimidazole

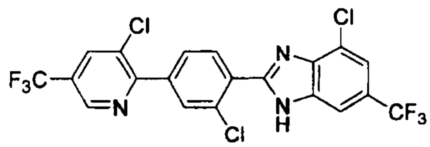

The same procedure as in (2) of Example 92 was performed to produce 4-chloro-2-[2-chloro-4-(3-chloro-5-trifluoromethylpyridin-2-yl)phenyl]-6-trifluoromethyl-1H-benzoimidazole (yield 71%).
$^1H$ NMR ($CDCl_3$) δ : 8.89 (s, 1H), 8.72 (d, 1H), 8.10 (s, 1H), 7.94-7.87 (m, 3H), 7.64 (s, 1H)

EXAMPLE 94: Preparation of N-[4'-(6-tert-butyl-1H-benzoimidazole-2-yl)-3'-chloro-3-fluorobiphenyl-4-yl]methanesulfonamide

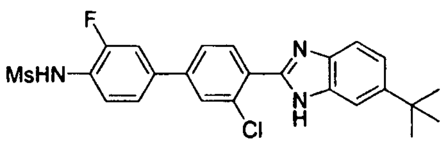

(1) Preparation of 4-(3-fluoro-4-methanesulfonylaminophenyl)-2-chlorobenzoic acid

The same procedure as in (1) of Example 1 was performed to produce 4-(3-fluoro-4-methanesulfonylaminophenyl)-2-chlorobenzoic acid (yield 54%).
$^1H$ NMR ($CD_3OD$) δ : 7.93 (d, 1H), 7.78 (d, 1H), 7.67-7.63 (m, 2H), 7.60-7.53 (m, 2H), 3.05 (s, 3H)

(2) Preparation of N-[4'-(6-*tert*-butyl-1H-benzoimidazole-2-yl)-3'-chloro-3-fluorobiphenyl-4-yl]methanesulfonamide

The same procedure as in (3) of Example 1 was performed to produce N-[4'-(6-*tert*-butyl-1H-benzoimidazole-2-yl)-3'-chloro-3-fluorobiphenyl-4-yl]methanesulfonamide (yield 60%).
$^1$H NMR ($CD_3OD$) δ : 7.94 (d, 1H), 7.89 (s, 1H), 7.75 (d, 1H), 7.67-7.56 (m, 5H), 7.44 (d, 1H), 3.06 (s, 3H), 1.41 (s, 9H)

EXAMPLES 95 AND 96

The same procedure as in (2) of Example 94 was performed to produce Compounds 95 and 96.

| Cpds. | Structures | Yields | Spectrum Data ($^1$H NMR) |
|---|---|---|---|
| 95 | 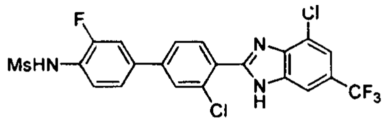 | 53 | ($CDCl_3$) δ : 7.75-7.64 (m, 3H), 7.47-7.43 (m, 2H), 7.34-7.27 (m, 2H), 6.63 (s, 1H), 3.10 (s, 3H) |
| 96 | 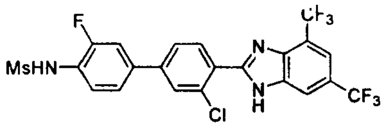 | 50 | ($CDCl_3$) δ : 7.98 (s, 1H), 7.74-7.68 (m, 2H), 7.48-7.43 (m, 2H), 7.32-7.27 (m, 2H), 6.65-6.63 (m, 1H), 3.11 (s, 3H) |

EXAMPLE 97: Preparation of 2-[2-chloro-4-(3-trifluoromethylpyridin-2-yl)phenyl]-6-trifluoromethyl-1H-benzoimidazole

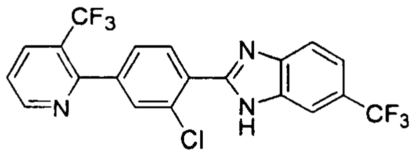

(1) Preparation of 2-chloro-4-(3-trifluoromethylpyridin-2-yl)benzoic acid

The same procedure as in (1) of Example 1 was performed to produce 2-chloro-4-(3-trifluoromethylpyridin-2-yl)benzoic acid (yield 73%).
$^1$H NMR ($CD_3OD$) δ: 8.78-8.76 (m, 1H), 8.20 (d, 1H), 7.91 (d, 1H), 7.80-7.76 (m, 2H), 7.41-7.39 (m, 1H)

(2) Preparation of 2-[2-chloro-4-(3-trifluoromethylpyridin-2-yl)phenyl]-6-trifluoromethyl-1H-benzoimidazole

The same procedure as in (3) of Example 1 was performed to produce 2-[2-chloro-4-(3-trifluoromethylpyridin-2-yl)phenyl]-6-trifluoromethyl-1H-benzoimidazole (yield 92%).
$^1$H NMR ($CDCl_3$) δ: 8.89 (d, 1H), 8.59 (d, 1H), 8.15 (d, 1H), 8.10-7.75 (m, 1H), 7.73 (s, 1H), 7.65-7.50 (m, 4H)

EXAMPLES 98 AND 99

The same procedure as in (2) of Example 97 was performed to produce Compounds 98 and 99.

| Cpds. | Structures | Yield | Spectrum Data ($^1$H NMR) |
|---|---|---|---|
| 98 | 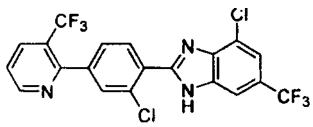 | 77 | ($CDCl_3$) δ : 8.92 (d, 1H), 8.69 (d, 1H), 8.17 (d, 1H), 8.08 (s, 1H), 7.76 (s, 1H), 7.66 (d, 2H), 7.60-7.56 (m, 1H) |
| 99 | 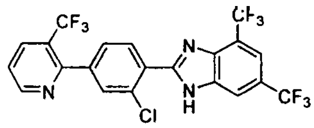 | 63 | ($CDCl_3$) δ : 8.89 (d, 1H), 8.64 (d, 1H), 8.32 (s, 1H), 8.17 (d, 1H), 7.86 (s, 1H), 7.77 (s, 1H), 7.68 (d, 1H), 7.60-7.55 (m, 1H) |

EXAMPLE 100: Preparation of 6-*tert*-butyl-2-[2-chloro-4-(3-methylpyridin-2-yl)phenyl]-1H-benzoimidazole

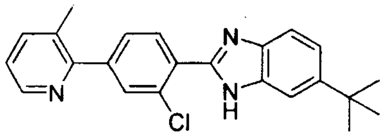

To a solution of 0.46g (3.6mmol) 2-chloro-3-methylpyridine in 20mL 1,2-dimethoxyethane and 20mL dilstilled water were added 2.2g (21.1mmol) $Na_2CO_3$, 0.6g (3.0mmol) 3-chloro-4-carboxylphenylboronic acid and 50mg $Pd(PPh_3)_4$, followed by stirring the solution for 15 hours in a heat flux condition and concentrating in a vacuum. The aqueous layer was washed with ethyl acetate and adjusted to a pH of 1 with conc. HCl. Thereafter, extraction with ethyl acetate was conducted many times and the organic layer was dried over magnesium sulfate and vacuum concentrated. The concentrate was dissolved in 25mL dimethylformamide and mixed with 0.5g (3.0mmol) 4-tert-butylbenzen-1,2-diamine, 1.2mL (6.0mmol) diisopropylethylamine and 1.3g (7.2mmol) O-(7-azabenzotriazol-1-yl)-N,N,N',N'-tetramethyleuronium hexafluorophosphate, followed by stirring at room temperature for 16 hours. The concentrate obtained by vaccum concentration was dissolved in ethyl acetate, washed with saturated $NaHCO_3$ and saturated NaCl, and dried over magnesium sulfate, followed by vacuum concentration. The residue thus obtained was dissolved in acetic acid/toluene (15mL/1.5mL), and the solution was stirred at 75°C for 3 hours and vacuum concentrated. Again, this concentrate was dissolved in ethyl acetate, washed with saturated $NaHCO_3$ and saturated NaCl, and dried over magnesium sulfate, followed by vacuum concentration. The residue was separated using column chromatography(developing solvent: chloroform/methanol=30/1) to produce 0.77g of 6-tert-butyl-2-[2-chloro-4-(3-methylpyridin-2-yl)phenyl]-1H-benzoimidazole (yield 68%).
$^1$H NMR ($CDCl_3$) $\delta$ : 8.69 (d, 1H), 8.56-8.52 (m, 1H), 8.06 (s, 1H), 7.73 (s, 1H), 7.53-7.48 (m, 1H), 7.42-7.39 (m, 1H), 7.31-7.27 (m, 1H), 7.18-7.14 (m, 1H), 6.89 (d, 1H), 2.67 (s, 3H), 1.38 (s, 9H)

EXAMPLE 101: Preparation of 6-chloro-2-(2-chloro-4-pyridin-2-ylphenyl)-1H-benzoimidazole

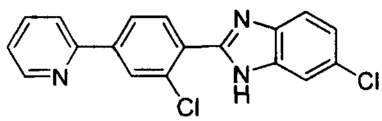

(1) Preparation of 2-chloro-4-pyridin-2-ylbenzoic acid

The same procedure as in (1) of Example 1 was performed to produce 2-chloro-4-pyridin-2-ylbenzoic acid (yield 65%).
$^1$H NMR ($CD_3OD$) $\delta$ : 8.63 (s, 1H), 8.05 (s, 1H), 7.99-7.89 (m, 2H), 7.41-7.39 (m, 2H), 6.65-6.63 (m, 1H)

(2) Preparation of 6-chloro-2-(2-chloro-4-pyridin-2-ylphenyl)-1H-benzoimidazole

The same procedure as in (3) of Example 1 was performed to produce 6-chloro-2-(2-chloro-4-pyridin-2-ylphenyl)-1H-benzoimidazole (yield 85%).
$^1$H NMR ($CDCl_3$) $\delta$ : 8.72 (d, 1H), 8.48 (d, 1H), 8.19 (s, 1H), 8.00 (d, 1H), 7.82-7.68 (m, 2H), 7.62 (s, 1H), 7.59 (d, 1H), 7.31-7.29 (m, 2H)

EXAMPLES 102 AND 103

The same procedure as in (2) of Example 101 was performed to produce Compounds 102 and 103.

| Cpds. | Structures | Yield | Spectrum Data ($^1$H NMR) |
|---|---|---|---|
| 102 | 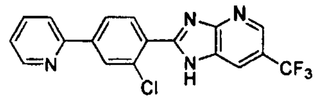 | 52 | ($CDCl_3$) δ : 8.86 (d, 1H), 8.76 (s, 1H), 8.62 (d, 1H), 8.37 (d, 1H), 8.15-8.10 (m, 2H), 8.01 (d, 1H), 7.62-7.55 (m, 2H) |
| 103 | 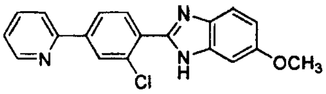 | 85 | ($CDCl_3$) δ : 8.32 (d, 1H), 7.67 (d, 2H), 7.61-7.25 (m, 1H), 7.49 (s, 1H), 7.42-7.34 (m, 4H), 7.02 (d, 1H), 3.87 (s, 3H) |

EXAMPLE 104: Preparation of 2-[4-(3-chloropyridin-2-yl)naphthalen-1-yl]-6-trifluoromethyl-1H-benzoimidazole

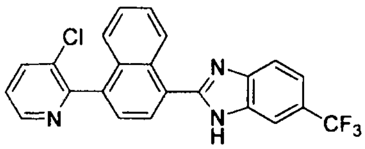

(1) Preparation of 4-carboxynaphthalene-1-bronic acid

To 5 mL of a 25% sodium hydroxide solution was slowly added 0.2g (1.08mmol) 4-methylnaphthalene-1-boronic acid. During this process, the container was kept in an ice bath. To this, a solution of 0.43g (2.70mmol) potassium permanganate in 5 mL distilled water was slowly added over 1 hour, followed by stirring for an additional 30 min. The solution was withdrawn from the ice bath and stirred at 40°C for an additional 2 hours. The resulting reaction solution was filtered through celite to remove solid matters. To the filtrate was added 10mL ethyl acetate 10mL. After the pH of the aqueous layer was adjusted to 4.0 with a 5.0N HCl solution, the ethyl acetate layer was separated, dried over anhydrous magnesium sulfate and vacuum concentrated. The residue thus obtained was dissolved in a small amount of methanol and separated using column chromatography(chloroform/methanol=10:1) to produce 0.14g of 4-carboxynaphthalene-1-bronic acid (yield 58%).
$^1$H NMR ($CDCl_3$) δ : 7.68-7.62 (m, 2H), 7.40-7.34 (m, 4H)

(2) Preparation of 4-(3-chloropyridin-2-yl)naphthalen-1-carboxylic acid

The same procedure as in (1) of Example 1 was performed to produce 4-(3-chloropyridin-2-yl)naphthalen-1-carboxylic acid (yield 60%).
$^1$H NMR ($CD_3OD$) δ : 8.97 (d, 1H), 8.63 (dd, 1H), 8.27 (d, 1H), 8.12 (d, 1H), 7.63-7.49 (m, 4H), 7.40 (d, 1H)

(3) Preparation of 2-[4-(3-chloropyridin-2-yl)naphthalen-1-yl]-6-trifluoromethyl-1H-benzoimidazole

The same procedure as in (3) of Example 1 was performed to produce 2-[4-(3-chloropyridin-2-yl)naphthalen-1-yl]-6-trifluoromethyl-1H-benzoimidazole (yield 79%).
$^1$H NMR ($CDCl_3$) δ : 8.74 (d, 1H), 8.56 (d, 1H), 8.01-7.95 (m, 2H), 7.86 (d, 1H), 7.78 (d, 1H), 7.53 (d, 1H), 7.47 (d, 2H), 7.40-7.27 (m, 3H)

EXAMPLES 105 TO 107

The same procedure as in (3) of Example 1 was performed to produce Compounds 105 to 107.

| Cpds. | Structures | Yield | Spectrum Data ($^1$H NMR) |
|---|---|---|---|
| 105 | 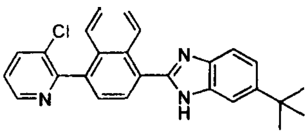 | 96 | ($CD_3OD$) δ : 8.66 (d, 1H), 8.65 (d, 1H), 8.13 (d, 1H), 7.98 (d, 1H), 7.71 (s, 1H), 7.64-7.58 (m, 5H), 7.47-7.44 (m, 2H), 1.44 (s, 9H) |

(continued)

| Cpds. | Structures | Yield | Spectrum Data ($^1$H NMR) |
|---|---|---|---|
| 106 | 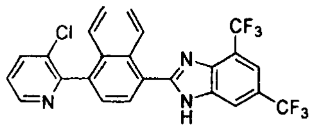 | 75 | ($CD_3OD$) δ : 8.70 (d, 1H), 8.14-8.09 (m, 1H), 8.08 (d, 1H), 8.00 (s, 1H), 7.72-7.68 (m, 5H), 7.66-7.60 (m, 1H), 7.47-7.28 (m, 1H) |
| 107 | 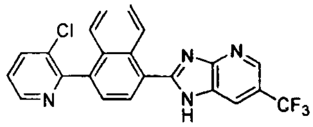 | 69 | ($CDCl_3$) δ : 8.65-8.55 (m, 4H), 8.13-8.15 (m, 1H), 7.74-7.68 (m, 3H), 7.35-7.30 (m, 2H), 7.19-7.15 (m, 1H) |

EXAMPLE 108: Preparation of 6-*tert*-butyl-2-[4-(3,5-dichloropyridin-2-yl)naphthalen-1-yl]-1H-benzoimidazole

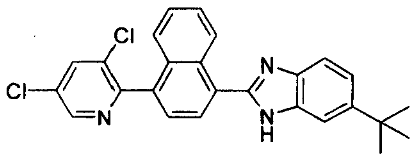

(1) Preparation of 4-(3,5-dichloropyridin-2-yl)naphthalen-1-carboxylic acid

The same procedure as in (1) of Example 1 was performed to produce 4-(3,5-dichloropyridin-2-yl)naphthalen-1-carboxylic acid (yield 56%).
$^1$H NMR ($CD_3OD$) δ : 8.92 (d, 1H), 8.63 (s, 1H), 8.19 (d, 2H), 7.60-7.55 (m, 1H), 7.49-7.42 (m, 2H), 7.36 (d, 1H)

(2) Preparation of 6-*ter*t-butyl-2-[4-(3,5-dichloropyridin-2-yl)naphthalen-1-yl]-1H-benzoimidazole

The same procedure as in (3) of Example 1 was performed to produce 6-*tert*-butyl-2-[4-(3,5-dichloropyridin-2-yl)naphthalen-1-yl]-1H-benzoimidazole (yield 79%).
$^1$H NMR (DMSO-$d_6$) δ : 7.99-7.95 (m, 1H), 7.70-7.64 (m, 1H), 7.45-7.41 (m, 1H), 7.18-7.15 (m, 1H), 6.87-6.46 (m, 7H), 0.57 (s, 9H)

EXAMPLE 109: Preparation of 2-[4-(3,5-dichloropyridin-2-yl)naphthalen-1-yl]-9,6-bistrifluoromethyl-1H-benzoimidazole

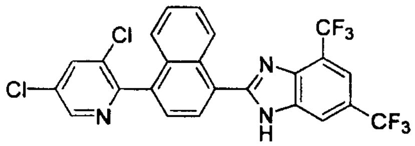

The same procedure as in (2) of Example 108 was performed to produce 2-[4-(3,5-dichloropyridin-2-yl)naphthalen-1-yl]-9,6-bistrifluoromethyl-1H-benzoimidazole (yield 60%).
$^1$H NMR (DMSO-$d_6$) δ : 8.00 (d, 1H), 7.68 (s, 1H), 7.50 (d, 1H), 7.44-7.40 (m, 1H), 7.29-7.25 (m, 1H), 6.85-6.63 (m, 5H)

EXAMPLE 110: Preparation of 6-*tert*-butyl-2-[4-(3-chloro-5-trifluoromethylpyridin-2-yl)naphthalen-1-yl]-1H-benzoimidazole

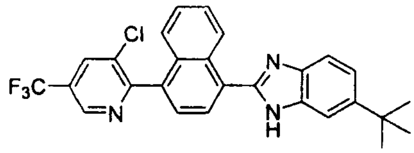

The same procedure as in Example 100 was performed to produce 6-*tert*-butyl-2-[4-(3-chloro-5-trifluoromethylpyridin-2-yl)naphthalen-1-yl]-1H-benzoimidazole (yield 56%).
$^1H$ NMR (DMSO-$d_6$) δ : 8.36-8.30 (m, 1H), 7.93-7.89 (m, 1H), 7.48-7.44 (m, 1H), 7.28-7.25 (m, 1H), 6.91-6.59 (m, 7H), 0.59 (s, 9H)

EXAMPLE 111: Preparation of 2-[4-(3-chloro-5-trifluoromethylpyridin-2-yl)naphthalen-1-yl]-4,6-bistrifluoromethyl-1H-benzoimidazole

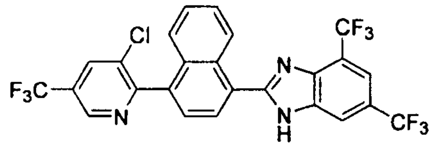

The same procedure as in Example 110 was performed to produce 2-[4-(3-chloro-5-trifluoromethylpyridin-2-yl)naphthalen-1-yl]-4,6-bistrifluoromethyl-1H-benzoimidazole (yield 55%).
$^1H$ NMR (DMSO-$d_6$) δ : 8.34 (d, 1H), 7.91 (s, 1H), 7.55 (d, 1H), 7.43-7.37 (m, 1H), 7.27 (d, 1H), 6.84-6.64 (m, 5H)

EXAMPLE 112: Preparation of N-{4-[4-(4,6-bistrifluoromethyl-1H-benzoimidazole-2-yl)naphthalen-1-yl]-2-fluorophenyl} methanesulfonamide

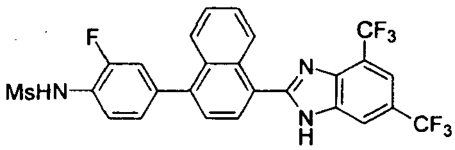

(1) Preparation of 4-(3-fluoro-4-methanesulfonylaminophenyl)naphthalen-1-carboxylic acid

The same procedure as in (1) of Example 1 was performed to produce 4-(3-fluoro-4-methanesulfonylaminophenyl)naphthalen-1-carboxylic acid (yield 43%).
$^1H$ NMR ($CD_3OD$) δ : 8.92 (d, 1H), 8.14 (d, 1H), 7.82 (s, 1H), 7.64-7.58 (m, 2H), 7.48-7.41 (m, 2H), 7.29-7.25 (m, 2H), 3.06 (s, 3H)

(2) Preparation of N-{4-[4-(4,6-bistrifluoromethyl-1H-benzoimidazole-2-yl)naphthalen-1-yl]-2-fluorophenyl}methanesulfonamide

The same procedure as in (3) of Example 1 was performed to produce N-{4-[4-(4,6-bistrifluoromethyl-1H-benzoimidazole-2-yl)naphthalen-1-yl]-2-fluorophenyl}methanesulfonamide (yield 55%).
$^1H$ NMR ($CD_3OD$) δ : 8.43 (d, 1H), 8.00-7.89 (m, 3H), 7.70-7.60 (m, 3H), 7.61-7.52 (m, 2H), 7.37-7.30 (m, 2H), 3.11 (s, 3H)

EXAMPLE 113: Preparation of 6-trifluoromethyl-2-[4-(3-trifluoromethylpyridin-2-yl)naphthalen-1-yl]-1H-benzoimidazole

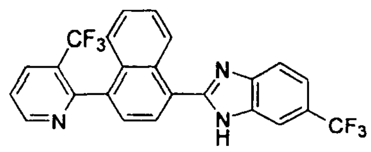

(1) Preparation of 4-(3-trifluoromethylpyridin-2-yl)naphthalen-1-carboxylic acid

The same procedure as in (1) of Example 1 was performed to produce 4-(3-trifluoromethylpyridin-2-yl)naphthalen-1-carboxylic acid (yield 60%).
$^1$H NMR ($CD_3OD$) δ : 8.75 (d, 1H), 8.35 (d, 1H), 8.26 (d, 2H), 7.74-7.70 (m, 2H), 7.59-7.54 (m, 2H), 7.25 (d, 1H)

(2) Preparation of 6-trifluoromethyl-2-[4-(3-trifluoromethylpyridin-2-yl)naphthalen-1-yl]-1H-benzoimidazole

The same procedure as in (3) of Example 1 was performed to produce 6-trifluoromethyl-2-[4-(3-trifluoromethylpyridin-2-yl)naphthalen-1-yl]-1H-benzoimidazole (yield 64%).
$^1$H NMR ($CDCl_3$) δ : 8.60-8.50 (m, 3H), 8.13-8.11 (m, 1H), 7.91-7.85 (m, 1H), 7.65-7.62 (m, 1H), 7.47-7.42 (m, 2H), 7.34-7.30 (m, 2H), 7.49-7.46 (m, 1H), 7.02-6.98 (m, 1H)

EXAMPLE 114: Preparation of 6-*tert*-butyl-2-(4-pyridin-2-ylnaphthalen-1-yl)-1H-benzoimidazole

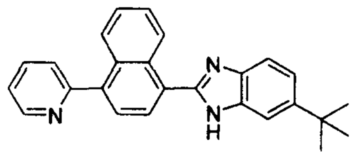

The same procedure as in Example 100 was performed to produce 6-tert-butyl-2-(4-pyridin-2-ylnaphthalen-1-yl)-1H-benzoimidazole (yield 57%).
$^1$H NMR ($CDCl_3$) δ : 8.74 (d, 1H), 7.98 (d, 1H), 7.88 (d, 1H), 7.78 (d, 2H), 7.60 (d, 2H), 7.52-7.40 (m, 6H), 1.48 (s, 9H)

EXAMPLE 115: Preparation of 2-(4-pyridin-2-ylnaphthalen-1-yl)-4,6-bistrifluoromethyl-1H-benzoimidazole

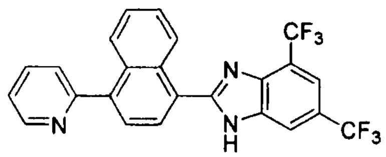

The same procedure as in Example 114 was performed to produce 2-(4-pyridin-2-ylnaphthalen-1-yl)-4,6-bistrifluoromethyl-1H-benzoimidazole (yield 47%).
$^1$H NMR ($CD_3OD$) δ : 8.74 (d, 1H), 8.44 (d, 1H), 8.03 (d, 2H), 7.94 (d, 2H), 7.72-7.66 (m, 3H), 7.64-7.58 (m, 2H), 7.05 (d, 1H)

EXAMPLE 116: Preparation of 6-tert-butyl-2-[3-chloro-4-(3-chloropyridin-2-yl)phenyl]-1H-benzoimidazole

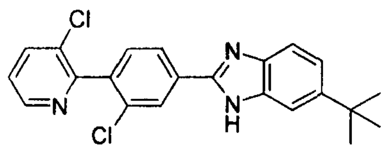

(1) Preparation of 3-chloro-4-(4,4,5,5-tetramethyl-[1,3,2]dioxabororan-2-yl)benzoic acid

The same procedure as in (1) of Example 104 was performed to produce 3-chloro-4-(4,4,5,5-tetramethyl-[1,3,2]dioxabororan-2-yl)benzoic acid (yield 50%).
$^1$H NMR ($CD_3OD$) δ : 7.89 (s, 1H), 7.80 (d, 1H), 7.65 (d, 1H), 1.37 (s, 12H)

(2) Preparation of 3-chloro-4-(3-chloropyridin-2-yl)benzoic acid

The same procedure as in (1) of Example 1 was performed to produce 3-chloro-4-(3-chloropyridin-2-yl)benzoic acid (yield 69%).
$^1$H NMR ($CD_3OD$) δ : 8.51 (dd, 1H), 8.04 (d, 1H), 7.97 (dd, 1H), 7.95 (dd, 1H), 7.45 (dd, 1H), 7.32 (d, 1H)

(3) Preparation of 6-*tert*-butyl-2-[3-chloro-4-(3-chloropyridin-2-yl)phenyl]-1H-benzoimidazole

The same procedure as in (3) of Example 1 was performed to produce 6-*tert*-butyl-2-[3-chloro-4-(3-chloropyridin-2-yl)phenyl]-1H-benzoimidazole (yield 86%).
$^1$H NMR ($CDCl_3$) δ : 8.72 (d, 1H), 8.48 (d, 1H), 8.19 (s, 1H), 8.00 (d, 1H), 7.82-7.68 (m, 1H), 7.62 (s, 1H), 7.59 (d, 1H), 7.31-7.29 (m, 2H), 1.42 (s, 9H)

EXAMPLE 117: Preparation of 2-[3-chloro-4-(3,5-dichloropyridin-2-yl)phenyl]-6-trifluoromethyl-1H-benzoimidazole

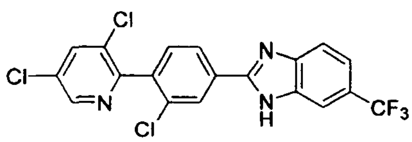

(1) Preparation of 3-chloro-4-(3,5-dichloropyridin-2-yl)benzoic acid

The same procedure as in (1) of Example 1 was performed to produce 3-chloro-4-(3,5-dichloropyridin-2-yl)benzoic acid (yield 64%).
$^1$H NMR ($CD_3OD$) δ : 8.58 (s, 1H), 8.13 (s, 1H), 8.10 (d, 1H), 8.00 (d, 1H), 7.36 (d, 1H)

(2) Preparation of 2-[3-chloro-4-(3,5-dichloropyridin-2-yl)phenyl]-6-trifluoromethyl-1H-benzoimidazole

The same procedure as in (3) of Example 1 was performed to produce 2-[3-chloro-4-(3,5-dichloropyridin-2-yl)phenyl]-6-trifluoromethyl-1H-benzoimidazole (yield 70%).
$^1$H NMR ($CD_3OD$) δ : 8.56 (s, 1H), 8.26 (s, 1H), 8.12 (dd, 2H), 7.88 (s, 1H), 7.72 (d, 1H), 7.54 (d, 1H), 7.49 (d, 1H)

EXAMPLES 118 AND 119

The same procedure as in (2) of Example 117 was performed to produce Compounds 118 and 119.

| Cpds. | Structures | Yield | Spectrum Data ($^1$H NMR) |
|---|---|---|---|
| 118 | 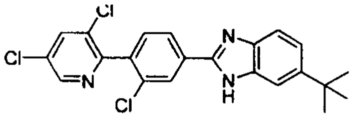 | 78 | ($CD_3OD$) δ : 8.64 (s, 1H), 8.28 (s, 1H), 8.20 (s, 1H), 8.13 (d, 1H), 7.65-7.56 (m, 3H), 7.44 (d, 1H), 1.41 (s, 9H) |

(continued)

| Cpds. | Structures | Yield | Spectrum Data ($^1$H NMR) |
|---|---|---|---|
| 119 | 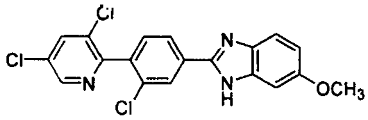 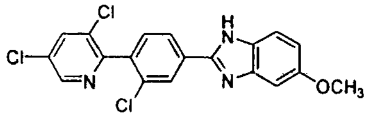 | 85 | ($CD_3OD$) δ : 8.64 (d, 0.6H), 8.61 (d, 0.4H), 8.25 (s, 0.6H), 8.20-8.15 (m, 1.0H), 8.12-8.07 (m, 1.0H), 7.58-7.50 (m, 2.4H), 7.12 (s, 0.6H), 7.10 (s, 0.4H), 6.97-6.92 (m, 1.0H), 3.87 (s, 1.8H), 3.85 (s, 1.2H) |

EXAMPLE 120: Preparation of 2-[3-chloro-4-(3-chloro-5-trifluoromethylpyridin-2-yl)phenyl]-6-trifluoromethyl-1H-benzoimidazole

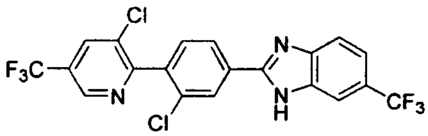

(1) Preparation of 3-chloro-4-(3-chloro-5-trifluoromethylpyridin-2-yl)benzoic acid

The same procedure as in (1) of Example 1 was performed to produce 3-chloro-4-(3-chloro-5-trifluoromethylpyridin-2-yl)benzoic acid (yield 69%).
$^1$H NMR ($CD_3OD$) δ : 8.90 (s, 1H), 8.39 (s, 1H), 8.11 (s, 1H), 8.00 (d, 1H), 7.33 (d, 1H)

(2) Preparation of 2-[3-chloro-4-(3-chloro-5-trifluoromethylpyridin-2-yl)phenyl]-6-trifluoromethyl-1H-benzoimidazole

The same procedure as in (3) of Example 1 was performed to produce 2-[3-chloro-4-(3-chloro-5-trifluoromethylpyridin-2-yl)phenyl]-6-trifluoromethyl-1H-benzoimidazole (yield 80%).
$^1$H NMR ($CD_3OD$) δ : 8.60 (s, 1H), 8.09 (s, 1H), 7.99-7.96 (m, 1H), 7.85 (s, 1H), 7.69 (d, 1H), 7.53-7.48 (m, 3H)

EXAMPLES 121 AND 122

The same procedure as in (2) of Example 120 was performed to produce Compounds 121 and 122.

| Cpds. | Structures | Yield | Spectrum Data ($^1$H NMR) |
|---|---|---|---|
| 121 | 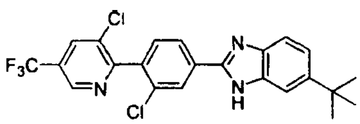 | 82 | ($CDCl_3$) δ : 8.62 (s, 1H), 8.02-7.96 (m, 1H), 7.79-7.73 (m, 2H), 7.60-7.56 (m, 1H), 7.47-7.43 (m, 1H), 7.31-7.28 (m, 1H), 7.18-7.14 (m, 1H), 1.42 (s, 9H) |
| 122 | 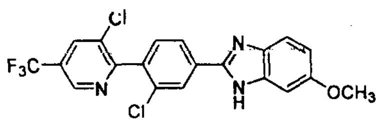 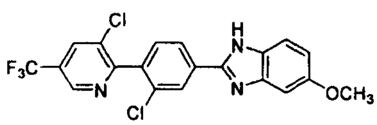 | 79 | ($CD_3OD$) δ : 8.96 (s, 0.6H), 8.94 (s, 0.4H), 8.45 (s, 0.6H), 8.43 (s, 0.4H), 8.28 (s, 0.6H), 8.15-8.10 (m, 1.4H), 7.98-7.92 (m, 1.0H), 7.63-7.58 (m, 1.0H), 7.56-7.51 (m, 1.0H), 7.13 (d, 0.6H), 7.10 (d, 0.4H), 3.88 (s, 1.8H), 3.85 (s, 1.2H) |

EXAMPLE 123: Preparation of N-[2'-chloro-4'-(4-chloro-6-trifluoromethyl-1H-benzoimidazole-2-yl)-3-fluorobiphenyl-4-yl]methanesulfonamide

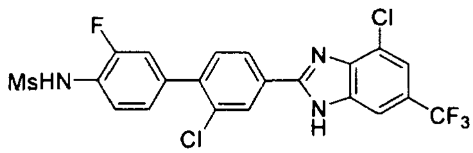

(1) Preparation of 3-chloro-4-(3-fluoro-4-methanesulfonylaminophenyl)benzoic acid

The same procedure as in (1) of Example 1 was performed to produce 3-chloro-4-(3-fluoro-4-methanesulfonylaminophenyl)benzoic acid (yield 54%).
$^1$H NMR ($CD_3OD$) δ : 8.06 (s, 1H), 7.92 (d, 1H), 7.58 (dd, 1H), 7.40 (d, 1H), 7.34 (s, 1H), 7.28-7.25 (m, 1H), 3.06 (s, 3H)

(2) Preparation of N-[2'-chloro-4'-(4-chloro-6-trifluoromethyl-1H-benzoimidazole-2-yl)-3-fluorobiphenyl-4-yl]methanesulfonamide

The same procedure as in (3) of Example 1 was performed to produce N-[2'-chloro-4'-(4-chloro-6-trifluoromethyl-1H-benzoimidazole-2-yl)-3-fluorobiphenyl-4-yl]methanesulfonamide (yield 60%).
$^1$H NMR ($CD_3OD$) δ : 8.40 (s, 1H), 8.21 (d, 1H), 7.88 (s, 1H), 7.42-7.34 (m, 3H), 7.42-7.34 (m, 2H), 3.08 (s, 3H) EXAMPLE 124: Preparation of N-[4'-(4,6-bistrifluoromethyl-1H-benzoimidazole-2-yl)-2'-chloro-3-fluorobiphenyl-4-yl]methanesulfonamide

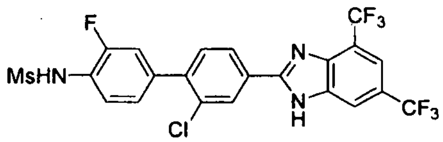

The same procedure as in (2) of Example 123 was performed to produce N-[4'-(4,6-bistrifluoromethyl-1H-benzoimidazole-2-yl)-2'-chloro-3-fluorobiphenyl-4-yl]methanesulfonamide (yield 56%).
$^1$H NMR ($CD_3OD$) δ : 8.43 (s, 1H), 8.25 (d, 1H), 8.18 (s, 1H), 7.83 (s, 1H), 7.67-7.61 (m, 2H), 7.43-7.34 (m, 2H), 3.10 (s, 3H)

EXAMPLE 125: Preparation of 2-(3-chloro-4-pyridin-2-ylphenyl)-6-trifluoromethyl-1H-benzoimidazole

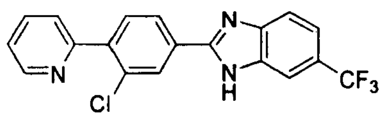

(1) Preparation of 3-chloro-4-pyridin-2-ylbenzoic acid

The same procedure as in (1) of Example 1 was performed to produce 3-chloro-4-pyridin-2-ylbenzoic acid (yield 68%.
$^1$H NMR ($CD_3OD$) δ : 8.66 (d, 1H), 8.14 (s, 1H), 8.05 (d, 1H), 7.99 (dd, 1H), 7.71 (d, 1H), 7.64 (d, 1H), 7.48 (dd, 1H)

(2) Preparation of 2-(3-chloro-4-pyridin-2-ylphenyl)-6-trifluoromethyl-1H-benzoimidazole

The same procedure as in (3) of Example 1 was performed to produce 2-(3-chloro-4-pyridin-2-ylphenyl)-6-trifluoromethyl-1H-benzoimidazole (yield 88%).
$^1$H NMR (DMSO-$d_6$) δ : 7.93-7.89 (m, 1H), 7.63-7.56 (m, 1H), 7.50-7.44 (m, 1H), 7.19-7.11 (m, 2H), 7.02-6.95 (m, 3H), 6.76 (s, 1H), 6.67-6.62 (m, 1H)

EXAMPLES 126 TO 130

The same procedure as in (2) of Example 125 was performed to produce Compounds 126 to 130.

| Cpds. | Structures | Yield | Spectrum Data ($^1H$ NMR) |
|---|---|---|---|
| 126 | 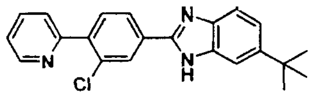 | 61 | (DMSO-$d_6$) δ : 7.92 (d, 1H), 7.52 (d, 1H), 7.42-7.38 (m, 1H), 7.15-7.11 (m, 1H), 6.97-6.92 (m, 2H), 6.87-6.83 (m, 1H), 6.79-6.73 (m, 2H), 6.67-6.63 (m, 1H), 0.57 (s, 9H) |
| 127 | 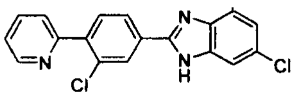 | 93 | ($CD_3OD$) δ : 8.69 (s, 1H), 8.30 (d, 1H), 8.15-8.13 (m, 1H), 7.99-7.98 (m, 1H), 7.76-7.72 (m, 2H), 7.66-7.62 (m, 2H), 7.52-7.50 (m, 1H), 7.32-7.31 (m, 1H) |
| 128 | 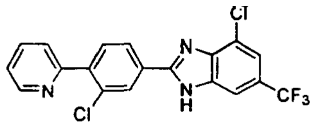 | 94 | ($CD_3OD$) δ : 8.69 (d, 1H), 8.44 (s, 1H), 8.26 (d, 1H), 8.00 (d, 1H), 7.95-7.90 (m, 1H), 7.75 (d, 2H), 7.62 (s, 1H), 7.55-7.50 (m, 1H) |
| 129 | 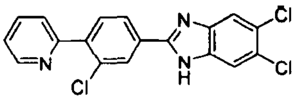 | 88 | ($CD_3OD$) δ : 8.67 (dd, 1H), 8.29 (d, 1H), 8.13 (dd, 1H), 7.97 (dd, 1H), 7.80-7.72 (m, 4H), 7.50 (dd, 1H) |
| 130 | 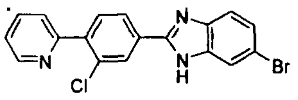 | 74 | ($CD_3OD$) δ : 8.68 (d, 1H), 8.29 (d, 1H), 8.12 (dd, 1H), 7.97 (dd, 1H), 7.80-7.70 (m, 3H), 7.55-7.48 (m, 2H), 7.43 (d, 1H) |

EXAMPLE 131: Preparation of 2-[3-chloro-4-(3-trifluoromethylpyridin-2-yl)phenyl]-6-trifluoromethyl-1H-benzoimidazole

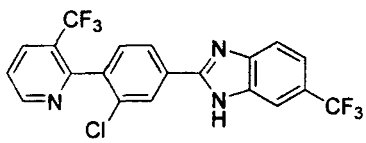

(1) Preparation of 3-chloro-4-(3-trifluoromethylpyridin-2-yl)benzoic acid

The same procedure as in (1) of Example 1 was performed to produce 3-chloro-4-(3-trifluoromethylpyridin-2-yl)benzoic acid (yield 76%).
$^1H$ NMR ($CD_3OD$) δ : 7.98-7.91 (m, 3H), 7.60-7.57 (m, 2H), 7.46 (d, 1H)

(2) Preparation of 2-[3-chloro-4-(3-trifluoromethylpyridin-2-yl)phenyl]-6-trifluoromethyl-1H-benzoimidazole

The same procedure as in (3) of Example 1 was performed to produce 2-[3-chloro-4-(3-trifluoromethylpyridin-2-yl)phenyl]-6-trifluoromethyl-1H-benzoimidazole (yield 89%).
$^1H$ NMR ($CDCl_3$) δ : 8.02 (s, 1H), 7.97-7.93 (m, 1H), 7.85 (s, 1H), 7.64 (d, 1H), 7.44 (d, 2H), 7.42-7.37 (m, 3H)

EXAMPLES 132 TO 134

The same procedure as in (2) of Example 131 was performed to produce Compounds 132 to 134.

| Cpds. | Structures | Yield | Spectrum Data ($^1H$ NMR) |
|---|---|---|---|
| 132 | 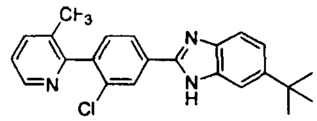 | 87 | ($CDCl_3$) δ : 8.04 (s, 1H), 8.02 (d, 1H), 7.92 (d. 1H), 7.78-7.82 (m, 1H), 7.61 (s, 1H), 7.55 (d, 1H), 7.47 (d, 1H), 7.38-7.31 (m, 2H), 1.42 (s, 9H) |
| 133 | 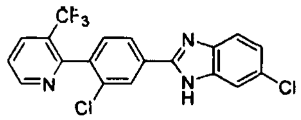 | 79 | ($CDCl_3$) δ : 7.97-7.91 (m, 3H), 7.53-7.43 (m, 3H), 7.36 (s, 2H), 7.18-7.15 (m, 1H) |
| 134 | 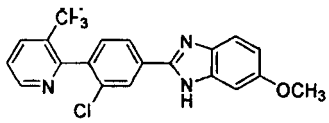 | 86 | ($CDCl_3$) δ : 7.96 (s, 1H), 7.95-7.89 (d, 1H), 7.46-7.43 (m, 2H), 7.38-7.34 (m, 3H), 7.02 (s, 1H), 6.85 (d, 1H), 3.80 (s, 3H) |

EXAMPLE 135: Preparation of 2-[3-chloro-4-(3-methylpyridin-2-yl)phenyl]-6-trifluoromethyl-1H-benzoimidazole

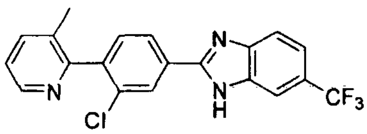

The same procedure as in Example 100 was performed to produce 2-[3-chloro-4-(3-methylpyridin-2-yl)phenyl]-6-trifluoromethyl-1H-benzoimidazole (yield 48%).
$^1H$ NMR ($CDCl_3$) δ : 8.01 (s, 1H), 7.96-7.91 (m, 1H), 7.84 (s, 1H), 7.62 (d, 1H), 7.44 (d, 1H), 7.37-7.34 (m, 4H), 2.70 (s, 3H)

EXAMPLE 136: Preparation of 3-chloro-6'-(6-trifluoromethyl-1H-benzoimidazole-2-yl)-[2,3']bipyridine

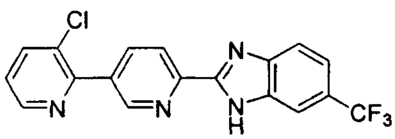

(1) Preparation of 3-chloro-2-(6-methylpyridin-3-yl)pyridine

To a solution of 0.5g (3.6mmol) 2,3-dichloropyridine in 20mL 1,2-dimethoxyethane and 20mL distilled water were added 2.2g (21.1mmol) $Na_2CO_3$, 0.41g (3.0mmol) 2-methylpyridine-5-boronic acid and 50mg $Pd(PPh_3)_4$, followed by stirring for 18 hours in a heat flux condition. After being cooled to room temperature, the solution was 50% concentrated in a vacuum condition. The aqueous layer was washed with ethyl acetate. Thereafter, the organic layer was dried over magnesium sulfate and vacuum concentrated. The concentrate was separated using column chromatography (developing solvent: ethyl acetate/hexane=1/10) to produce 0.56g of 3-chloro-2-(6-methylpyridin-3-yl)pyridine (yield 80%).
$^1H$ NMR ($CDCl_3$) δ : 8.91 (d, 1H), 8.63 (dd, 1H), 7.99 (dd, 1H), 7.83 (dd, 1H), 7.49-7.47 (m, 1H), 7.30-7.27 (m, 1H), 2.65 (s, 3H)

(2) Preparation of 5-(3-chloropyridin-2-yl)pyridin-2-carboxylic acid

The same procedure as in (1) of Example 104 was performed to produce 5-(3-chloropyridin-2-yl)pyridin-2-carboxylic acid (yield 69%).
$^1H$ NMR ($CDCl_3$) δ : 9.03 (d, 1H), 8.67 (dd, 1H), 8.36-8.32 (m, 2H), 7.88 (dd, 1H), 7.39-7.34 (m, 1H)

(3) Preparation of 3-chloro-6'-(6-trifluoromethyl-1H-benzoimidazole-2-yl)-[2,3']bipyridine

The same procedure as in (3) of Example 1 was performed to produce 3-chloro-6'-(6-trifluoromethyl-1H-benzoimidazole-2-yl)-[2,3']bipyridine (yield 92%).
$^1$H NMR ($CDCl_3$) δ : 9.04 (d, 1H), 8.65 (dd, 1H), 8.58 (d, 1H), 8.35 (dd, 1H), 8.00 (s, 1H), 7.88 (dd, 1H), 7.78 (s, 1H), 7.56 (dd, 1H), 7.32 (dd, 1H)

EXAMPLES 137 TO 145

The same procedure as in (3) of Example 136 was performed to produce Compounds 137 to 145.

| Cpds. | Structures | Yield | Spectrum Data ($^1$H NMR) |
|---|---|---|---|
| 137 | 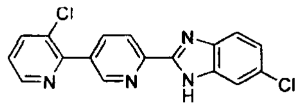 | 89 | ($CDCl_3$) δ : 9.03 (d, 1H), 8.65 (dd, 1H), 8.56 (d, 1H), 8.33 (dd, 1H), 7.86 (dd, 1H), 7.72 (d, 1H), 7.60 (d, 1H), 7.32 (dd, 1H), 7.28 (dd, 1H) |
| 138 | 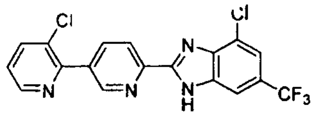 | 77 | ($CDCl_3$) δ : 9.10 (d, 1H), 8.68 (d, 1H), 8.52 (d, 1H), 8.38-8.31 (m, 1H), 8.05 (s, 1H), 7.88 (d, 1H), 7.59 (s, 1H), 7.36-7.29 (m, 1H) |
| 139 | 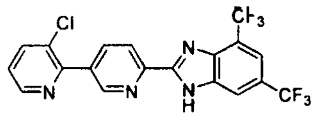 | 69 | ($CDCl_3$) δ : 9.08 (s, 1H), 8.65 (d, 1H), 8.51 (d, 1H), 8.33-8.28 (m, 2H), 7.85 (d, 1H), 7.80 (s, 1H), 7.34-7.31 (m, 1H) |
| 140 | 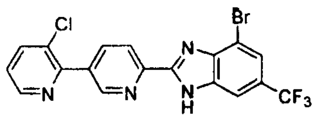 | | ($CDCl_3$) δ : 9.06 (s, 1H), 8.65-8.57 (m, 2H), 8.35 (d, 1H), 8.04 (d, 1H), 7.91 (s, 1H), 7.73 (s, 1H), 7.47 (dd, 1H) |
| 141 | 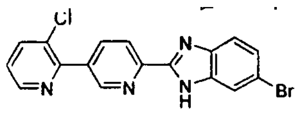 | | ($CDCl_3$) δ : 9.02 (d, 1H), 8.64 (dd, 1H), 8.52 (d, 1H), 8.30 (d, 1H), 7.83 (dd, 1H), 7.78 (s, 1H), 7.50 (d, 1H), 7.38 (d, 1H), 7.28 (dd, 1H) |
| 142 | 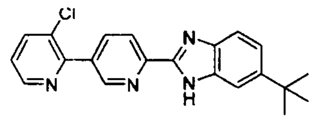 | | ($CDCl_3$) δ : 9.04 (d, 1H), 8.65 (dd, 2H), 8.30 (dd, 1H), 7.86 (dd, 1H), 7.70 (s, 1H), 7.60 (s, 1H), 7.44 (dd, 1H), 7.31 (dd, 1H), 1.41 (s, 9H) |
| 143 | 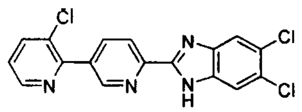 | | ($CDCl_3$) δ : 9.05 (d, 1H), 8.67 (dd, 1H), 8.57 (d, 1H), 8.34 (dd, 1H), 7.90-7.82 (m, 3H), 7.33 (dd, 1H) |
| 144 | 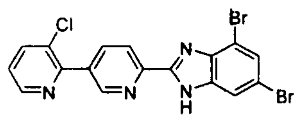 | | ($CDCl_3$) δ : 9.08 (d, 1H), 8.67 (dd, 1H), 8.52 (dd, 1H), 8.34 (dd, 1H), 7.88 (dd, 1H), 7.80 (s, 1H), 7.63 (dd, 1H), 7.34 (dd, 1H) |
| 145 | 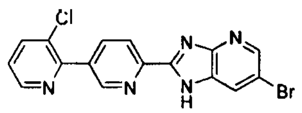 | | ($CDCl_3$) δ : 9.02 (d, 1H), 8.67 (dd, 1H), 8.37 (s, 2H), 8.23 (d, 1H), 7.97 (d, 1H), 7.89 (dd, 1H), 7.36 (dd, 1H) |

EXAMPLE 146: Preparation of 3-chloro-6'-(6-morpholin-4-yl-1H-benzoimidazole-2-yl)-[2,3']bipyridine

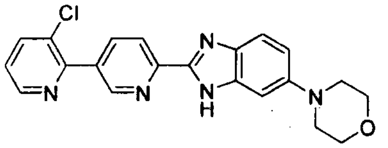

The same procedure as in Example 17 was performed to produce 3-chloro-6'-(6-morpholin-4-yl-1H-benzoimidazole-2-yl)-[2,3']bipyridine (yield 72%).
$^1$H NMR ($CD_3OD$) δ : 9.21 (dd, 1H), 8.69 (d, 1H), 8.48 (dd, 1H), 8.35 (d, 1H), 8.09 (d, 1H), 7.73 (d, 1H), 7.55-7.50 (m, 1H), 7.45 (dd, 1H), 7.23 (d, 1H), 3.92-3.88 (m, 4H), 3.32-3.29 (m, 4H)

EXAMPLES 147 TO 150

The same procedure as in Example 146 was performed to produce Compounds 147 to 150.

| Cpds. | Structures | Yield | Spectrum Data ($^1$H NMR) |
|---|---|---|---|
| 147 | 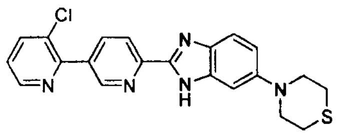 | 65 | ($CD_3OD$) δ : 9.19 (dd, 1H), 8.68 (d, 1H), 8.47 (dd, 1H), 8.36 (d, 1H), 8.08 (dd, 1H), 7.72 (d, 1H), 7.54-7.50 (m, 1H), 7.40 (dd, 1H), 7.24 (d, 1H), 3.74-3.70 (m, 4H), 2.84-2.80 (m, 4H) |
| 148 | 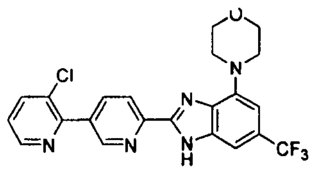 | 69 | ($CD_3OD$) δ : 9.09 (d, 1H), 8.65 (dd, 1H), 8.46 (d, 1H), 8.37 (dd, 1H), 8.05(d, 1H), 7.59 (s, 1H), 7.50-7.46 (m, 1H), 7.04 (s, 1H), 4.01-3.97 (m, 4H), 3.53-3.49 (m, 4H) |
| 149 | 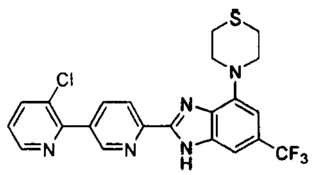 | 58 | ($CD_3OD$) δ : 9.08 (d, 1H), 8.66 (dd, 1H), 8.52 (d, 1H), 8.37(dd, 1H), 8.07 (d, 1H), 7.54-7.49 (m, 2H), 7.02 (s, 1H), 3.86-3.82 (m, 4H), 2.96-2.92 (m, 4H) |
| 150 | 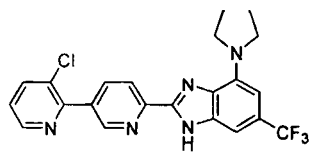 | 72 | ($CD_3OD$) δ : 9.04 (d, 1H), 8.64 (dd, 1H), 8.43 (d, 1H), 8.32 (dd, 1H), 8.04 (d, 1H), 7.49-7.45 (m, 2H), 7.32 (s, 1H), 3.89-3.84 (m, 4H), 2.20-2.15 (m, 4H) |

EXAMPLE 151: Preparation of 3,5-dichloro-6'-(6-trifluoromethyl-1H-benzoimidazole-2-yl)-[2,3']bipyridine

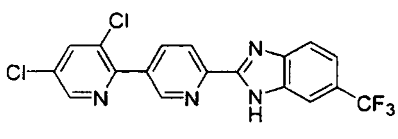

(1) Preparation of 3,5-dichloro-2-(6-methylpyridin-3-yl)pyridine

The same procedure as in (1) of Example 136 was performed to produce 3,5-dichloro-2-(6-methylpyridin-3-yl)pyridine (yield 72%).
$^1$H NMR ($CD_3OD$) δ : 8.76 (s, 1H), 8.67 (s, 1H), 8.20-8.11 (m, 2H), 7.47-7.43 (m, 1H), 2.61 (s, 3H)

(2) The same procedure as in Example 100 was performed to produce 3,5-dichloro-6'-(6-trifluoromethyl-1H-benzoimidazole-2-yl)-[2,3']bipyridine (yield 65%).

$^1$H NMR ($CDCl_3$) $\delta$ : 9.05 (s, 1H), 8.81 (d, 1H), 8.62 (s, 1H), 8.37 (d, 1H), 8.10 (s, 1H), 7.90 (s, 1H), 7.86 (d, 1H), 7.64 (d, 1H)

EXAMPLES 152 TO 155

The same procedure as in (2) of Example 151 was performed to produce Compounds 152 to 155.

| Cpds. | Structures | Yield | Spectrum Data ($^1$H NMR) |
|---|---|---|---|
| 152 | 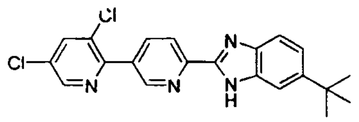 | 91 | ($CDCl_3$) $\delta$ : 9.02-8.98 (m, 1H), 8.61 (s, 1H), 8.45-8.28 (m, 2H), 8.08 (s, 1H), 7.96-7.80 (m, 1H), 7.68-7.60 (m, 2H), 1.42 (s, 9H) |
| 153 | 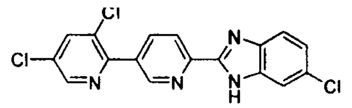 | 92 | ($CDCl_3$) $\delta$ : 8.99 (s, 1H), 8.64 (d, 1H), 8.59 (s, 1H), 8.29 (d, 1H), 7.89 (s, 1H), 7.70 (s, 1H), 7.65 (d, 1H), 7.31-7.28 (m, 1H) |
| 154 | 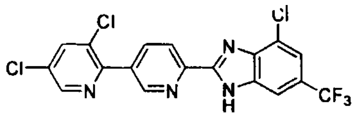 | 78 | ($CDCl_3$) $\delta$ : 9.08 (s, 1H), 8.64 (s, 1H), 8.59 (d, 1H), 8.33 (d, 1H), 7.91 (d, 2H), 7.60 (s, 1H) |
| 155 | 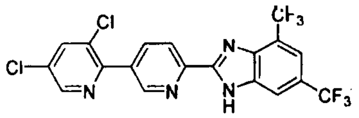 | 71 | $^1$H NMR (DMSO-$d_6$) $\delta$ : 9.09 (s, 1H), 8.64 (s, 1H), 8.59 (d, 1H), 8.35 (d, 1H), 8.27 (s, 1H), 7.92 (s, 1H), 7.84 (s, 1H) |

EXAMPLE 156: Preparation of 6'-(9,6-bistrifluoromethyl-1H-benzoimidazole-2-yl)-3-chloro-5-trifluoromethyl-[2,3']bipyridine

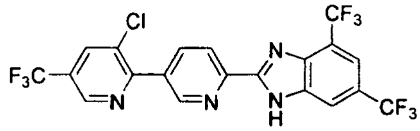

(1) Preparation of 3-chloro-5-trifluoromethyl-2-(6-methylpyridin-3-yl)pyridine

The same procedure as in (1) of Example 136 was performed to produce 3-chloro-5-trifluoromethyl-2-(6-methylpyridin-3-yl)pyridine (yield 79%).
$^1$H NMR ($CD_3OD$) $\delta$ : 8.94 (s, 1H), 8.82 (s, 1H), 8.39 (s, 1H), 8.16 (d, 1H), 7.48 (d, 1H), 2.63 (s, 3H)

(2) Preparation of 5-(3-chloro-5-trifluoromethylpyridin-2-yl)pyridin-2-carboxylic acid

The same procedure as in (1) of Example 104 was performed to produce 5-(3-chloro-5-trifluoromethylpyridin-2-yl)pyridin-2-carboxylic acid (yield 70%).
$^1$H NMR ($CD_3OD$) $\delta$ : 9.05-8.95 (m, 2H), 8.42-8.38 (m, 2H), 7.66-7.62 (m, 1H)

(3) Preparation of 6'-(4,6-bistrifluoromethyl-1H-benzoimidazole-2-yl)-3-chloro-5-trifluoromethyl-[2,3']bipyridine

The same procedure as in (3) of Example 1 was performed to produce 6'-(4,6-bistrifluoromethyl-1H-benzoimidazole-2-yl)-3-chloro-5-trifluoromethyl-[2,3']bipyridine (yield 73%).

$^1H$ NMR ($CDCl_3$) δ : 9.15 (s, 1H), 8.93 (s, 1H), 8.56 (d, 1H), 8.39-8.33 (m, 2H), 8.13 (s, 1H), 7.85 (s, 1H)

EXAMPLE 157: Preparation of 6'-(6-methoxy-1H-benzoimidazole-2-yl)-3-chloro-5-trifluoromethyl-[2,3']bipyridine

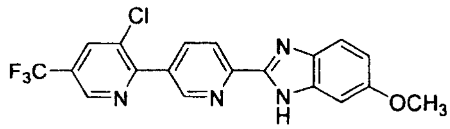

The same procedure as in (3) of Example 156 was performed to produce 6'-(6-methoxy-1H-benzoimidazole-2-yl)-3-chloro-5-trifluoromethyl-[2,3']bipyridine (yield 91%).
$^1H$ NMR ($CDCl_3$) δ : 8.91 (s, 1H), 8.25-8.20 (m, 1H), 8.12 (s, 1H), 7.57 (d, 1H), 7.36 (s, 1H), 7.36-7.28 (m, 3H), 3.94 (s, 3H)

EXAMPLE 158: Preparation of 3-trifluoromethyl-6'-(6-trifluoromethyl-1H-benzoimidazole-2-yl)-[2,3']bipyridine

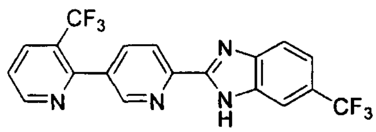

(1) Preparation of 3-trifluoromethyl-2-(6-methylpyridin-3-yl)pyridine

The same procedure as in (1) of Example 136 was performed to produce 3-trifluoromethyl-2-(6-methylpyridin-3-yl)pyridine (yield 82%).
$^1H$ NMR ($CDCl_3$) δ : 8.88 (d, 1H), 8.67 (s, 1H), 8.12 (dd, 1H), 7.76 (dd, 1H), 7.54-7.49 (m, 1H), 7.29-7.28 (m, 1H), 2.66 (s, 3H)

(2) Preparation of 5-(3-trifluoromethylpyridin-2-yl)pyridin-2-ylcarboxylic acid

The same procedure as in (1) of Example 104 was performed to produce 5-(3-trifluoromethylpyridin-2-yl)pyridin-2-ylcarboxylic acid (yield 75%).
$^1H$ NMR ($CD_3OD$) δ: 8.92-8.89 (m, 1H), 8.79 (s, 1H), 8.34-8.27 (m, 2H), 8.15 (s, 1H), 7.73-7.68 (m, 1H)

(3) Preparation of 3-trifluoromethyl-6'-(6-trifluoromethyl-1H-benzoimidazole-2-yl)-[2,3']bipyridine

The same procedure as in (3) of Example 1 was performed to produce 3-trifluoromethyl-6'-(6-trifluoromethyl-1H-benzoimidazole-2-yl)-[2,3']bipyridine (yield 94%).
$^1H$ NMR ($CDCl_3$) δ: 8.94 (d, 1H), 8.84 (s, 1H), 8.61 (d, 1H), 8.19 (d, 1H), 8.11 (d, 1H), 8.10 (s, 1H), 8.08 (d, 1H), 7.61-7.54 (m, 2H)

EXAMPLES 159 TO 168

The same procedure as in (3) of Example 158 was performed to produce Compounds 159 to 168.

| Cpds. | Structures | Yield | Spectrum Data ($^1H$ NMR) |
|---|---|---|---|
| 159 | 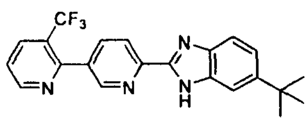 | 93 | ($CDCl_3$) δ: 8.91 (dd, 1H), 8.78 (d, 1H), 8.77-8.72 (br, 1H), 8.15 (dd, 1H), 8.05 (d, 1H), 7.75-7.65 (br, 2H), 7.53 (dd, 1H), 7.43 (d, 1H), 1.39 (s, 9H) |

(continued)

| Cpds. | Structures | Yield | Spectrum Data ($^1$H NMR) |
|---|---|---|---|
| 160 | 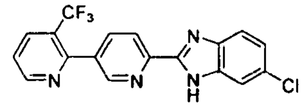 | 88 | ($CDCl_3$) δ: 8.92 (d, 1H), 8.80 (d, 1H), 8.53 (d, 1H), 8.16 (dd, 1H), 8.05 (dd, 1H), 7.65-7.56 (br, 2H), 7.54 (dd, 1H), 7.30 (dd, 1H) |
| 161 | 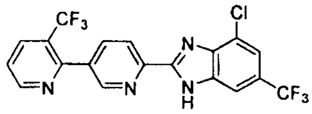 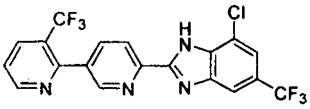 | 73 | ($CDCl_3$) δ: 8.89 (d, 1.2H), 8.79 (d, 0.8H), 8.62 (d, 0.4H), 8.50 (d, 0.6H), 8.13 (d, 1.0H), 8.07-8.01 (m, 1.6H), 7.70 (s, 0.4H), 7.54-7.49 (m, 2.0H) |
| 162 | 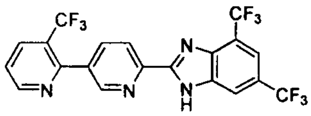 | 75 | ($CDCl_3$) δ: 8.95 (d, 1H), 8.86 (s, 1H), 8.60 (d, 1H), 8.25 (s, 1H), 8.19 (d, 1H), 8.13 (d, 1H), 7.86 (s, 1H), 7.60-7.56 (m, 1H) |
| 163 | 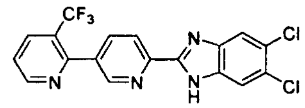 | 92 | ($CD_3OD$) δ: 8.92 (d, 1H), 8.83 (s, 1H), 8.41 (d, 1H), 8.33 (d, 1H), 8.11 (dd, 1H), 7.82 (s, 2H), 7.71 (dd, 1H) |
| 164 | 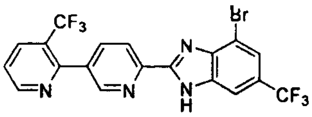 | 70 | ($CD_3OD$) δ: 8.93 (d, 1H), 8.87 (s, 1H), 8.59 (s, 1H), 8.35 (d, 1H), 8.14 (d, 1H), 7.94 (s, 1H), 7.76-7.69 (m, 2H) |
| 165 | 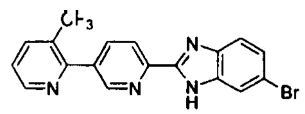 | 66 | ($CD_3OD$) δ: 8.90 (dd, 1H), 8.81 (d, 1H), 8.35 (dd, 1H), 8.29 (dd, 1H), 8.07 (dd, 1H), 7.80-7.70 (br, 1H), 7.67 (dd, 1H), 7.65-7.55 (br, 1H), 7.41 (dd, 1H) |
| 166 | 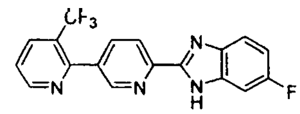 | 85 | ($CD_3OD$) δ: 8.91 (dd, 1H), 8.82 (d, 1H), 8.39-8.31 (m, 2H), 8.09 (dd, 1H), 7.71-7.65 (m, 2H), 7.35 (br, 1H), 7.09 (dd, 1H) |
| 167 | 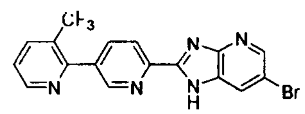 | 52 | ($CDCl_3$) δ: 9.79 (s, 1H), 8.91 (dd, 1H), 8.74 (d, 1H), 8.35 (dd, 1H), 8.15 (dd, 1H), 8.10 (dd, 1H), 8.03 (d, 1H), 7.54 (dd, 1H) |
| 168 | 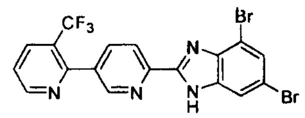 | 62 | ($CD_3OD$) δ: 8.90 (d, 1H), 8.82 (s, 1H), 8.52 (d, 1H), 8.32 (d, 1H), 8.09 (dd, 1H), 7.72 (d, 1H), 7.67 (dd, 1H), 7.59 (s, 1H) |

EXAMPLE 169: Preparation of 6'-(6-morpholin-4-yl-1H-benzoimidazo-2-yl)-3-trifluoromethyl-[2,3']bipyridine

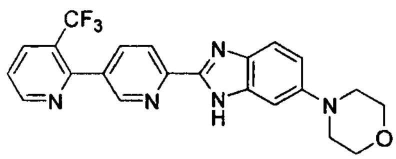

The same procedure as in Example 17 was performed to produce 6'-(6-morpholin-4-yl-1H-benzoimidazo-2-yl)-3-trifluoromethyl-[2,3']bipyridine (yield 67%).
$^1$H NMR ($CD_3OD$) δ: 8.90 (d, 1H), 8.80 (s, 1H), 8.33 (dd, 1H), 8.27 (dd, 1H), 8.07 (dd, 1H), 7.75-7.81 (br, 1H), 7.66 (dd, 1H), 7.65-7.55 (br, 1H), 7.41 (dd, 1H), 3.88-3.92 (m, 4H), 3.32-3.29 (m, 4H)

EXAMPLE 170: Preparation of 6'-(4-morpholin-4-yl-6-trifluoromethyl-1H-benzoimidazole-2-yl)-3-trifluoromethyl-[2,3']bipyridine

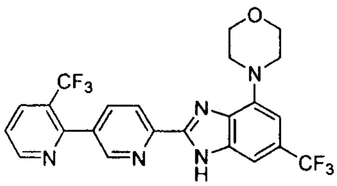

The same procedure as in Example 169 was performed to produce 6'-(4-morpholin-4-yl-6-trifluoromethyl-1H-benzoimidazole-2-yl)-3-trifluoromethyl-[2,3']bipyridine (yield 67%).
$^1$H NMR ($CD_3OD$) δ: 8.92 (d, 1H), 8.86 (s, 1H), 8.62-8.55 (br, 1H), 8.35 (d, 1H), 8.14 (d, 1H), 7.92 (s, 1H), 7.75-7.69 (m, 2H), 3.99-3.96 (m, 4H), 3.52-3.48 (m, 4H)

EXAMPLE 171: Preparation of 6'-(6-chloro-1H-benzoimidazole-2-yl)-[2,3']bipyridine

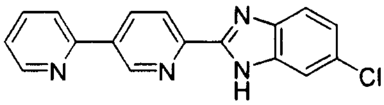

(1) Preparation of 2-(6-methylpyridin-3-yl)pyridine

The same procedure as in (1) of Example 136 was performed to produce 2-(6-methylpyridin-3-yl)pyridine (yield 67%).
$^1$H NMR ($CDCl_3$) δ: 9.08 (d, 1H), 8.72 (dd, 1H), 8.24 (dd, 1H), 7.79-7.75 (m, 2H), 7.62-7.45 (m, 1H), 7.30-7.27 (m, 1H), 2.64 (s, 3H)

(2) Preparation of 5-(pyridin-2-yl)pyridin-2-carboxylic acid

The same procedure as in (1) of Example 104 was performed to produce 5-(pyridin-2-yl)pyridin-2-carboxylic acid (yield 77%).
$^1$H NMR ($CD_3OD$) δ: 9.30 (s, 1H), 8.73 (d, 1H), 8.62 (d, 1H), 8.30 (d, 1H), 8.07-7.98 (m, 2H), 7.49 (d, 1H)

(3) Preparation of 6'-(6-chloro-1H-benzoimidazole-2-yl)-[2,3']bipyridine

The same procedure as in (3) of Example 1 was performed to produce 6'-(6-chloro-1H-benzoimidazole-2-yl)-[2,3']bipyridine (yield 90%).
$^1$H NMR ($CDCl_3$) δ: 7.80-7.78 (m, 1H), 7.70-7.67 (m, 1H), 7.62-7.58 (m, 1H), 7.46-7.45 (m, 1H), 7.32-7.13 (m, 6H)

EXAMPLES 172 AND 173

The same procedure as in (3) of Example 171 was performed to produce Compounds 172 and 173.

| Cpds. | Structures | Yield | Spectrum Data ($^1$H NMR) |
| --- | --- | --- | --- |
| 172 | 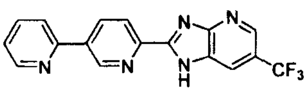 | 68 | ($CDCl_3$) δ: 8.91 (d, 1H), 7.74 (s, 1H), 7.67-7.63 (m, 2H) 7.61 (d, 1H), 7.56-7.43 (m, 4H) |

(continued)

| Cpds. | Structures | Yield | Spectrum Data ($^1$H NMR) |
|---|---|---|---|
| 173 | 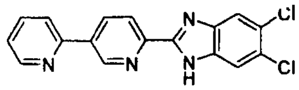 | 81 | ($CD_3OD$) δ: 9.35 (d, 1H), 8.71 (d, 1H), 8.55 (dd, 1H), 8.38 (d, 1H), |
| | | | 8.05-7.96 (m, 2H), 7.86 (s, 1H), 7.77 (s, 1H), 7.46 (dd, 1H) |

EXAMPLE 174: Preparation of 6-*tert*-butyl-2-(6-naphthalen-1-ylpyridin-3-yl)-1H-benzoimidazole

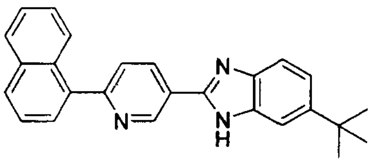

The same procedure as in Example 100 was performed to produce 6-*tert*-butyl-2-(6-naphthalen-1-ylpyridin-3-yl)-1H-benzoimidazole (yield 53%).
$^1$H NMR ($CD_3OD$) δ: 9.40 (s, 1H), 8.62 (d, 1H), 8.00-7.95 (m, 5H), 7.58-7.46 (m, 6H), 1.28 (s, 9H)

EXAMPLES 175 TO 177

The same procedure as in Example 174 was performed to produce Compounds 175 to 177.

| Cpds. | Structures | Yield | Spectrum Data ($^1$H NMR) |
|---|---|---|---|
| 175 | 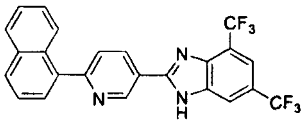 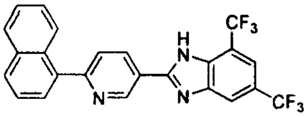 | 80 | ($CD_3OD$) δ: 9.53 (d, 0.8H), 9.19 (d, 0.2H), 8.81 (dd, 0.8H), 8.45 (dd, 0.2H), 8.23 (s, 0.8H), 8.20 (s, 0.2H), 8.04-8.00 (m, 3.0H), 7.94-7.90 (m, 1.0H), 7.85 (s, 0.8H), 7.82 (s, 0.2H), 7.70-7.63 (m, 2.0H), 7.58-7.50 (m, 2.0H) |
| 176 | 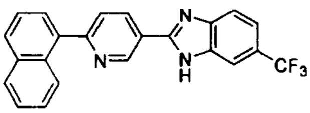 | 89 | ($CD_3OD$) δ: 9.45 (d, 1H), 8.66 (dd, 1H), 8.03-7.96 (m, 4H), 7.89 (dd, 1H), 7.69-7.53 (m, 6H) |
| 177 | 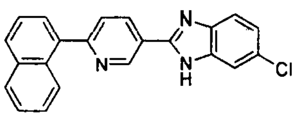 | 90 | ($CD_3OD$) δ: 9.41 (d, 1H), 8.64 (dd, 1H), 8.00-7.95 (m, 3H), 7.89 (d, 1H), 7.68-7.53 (m, 6H), 7.31 (d, 1H) |

EXAMPLE 178: Preparation of 2-(4-pyrrol-1-ylphenyl]-6-trifluoromethyl-1H-benzoimidazole

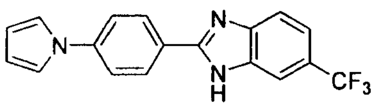

The same procedure as in (3) of Example 1 was performed to produce 2-(4-pyrrol-1-ylphenyl]-6-trifluoromethyl-1H-benzoimidazole (yield 77%).
$^1$H NMR ($CDCl_3$) δ: 8.12 (d, 2H, *J*=8.7Hz), 7.92 (s, 1H), 7.69 (d, 1H, *J*=8.6Hz), 7.54-7.49 (m, 3H), 7.15-7.14 (m, 2H), 6.40-6.38 (m, 2H)

EXAMPLES 179 TO 200

The same procedure as in Example 178 was performed to produce Compounds 179 to 200.

| Cpds. | Structures | Yield | Spectrum Data ($^1$H NMR) |
|---|---|---|---|
| 179 | 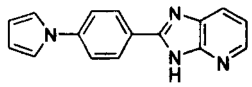 | 48 | ($CDCl_3$) δ: 8.37-8.36 (m, 1H), 8.25 (d, 1H, |
| | | | *J*=8.2Hz), 8.15 (d, 2H, *J*=8.1Hz), 7.47 (d, 2H, *J*=8.1Hz), 7.30-7.28(m, 1H), 7.16-7.14 (m, 2H), 6.39-6.36(m, 2H) |
| 180 | 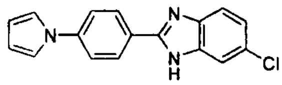 | 81 | ($CDCl_3$) δ: 8.09 (d, 2H, *J*=8.7Hz), 7.63 (s, 1H), 7.57 (d, 1H, *J*=8.6Hz), 7.51 (d, 2H, *J*=8.7Hz), 7.27-7.24 (m, 1H), 7.16-7.15 (m, 2H), 6.40-6.38 (m, 2H) |
| 181 | 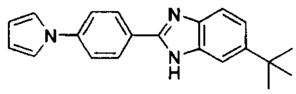 | 69 | ($CDCl_3$) δ: 8.10 (d, 2H, *J*=8.7Hz), 7.64 (s, 1H), 7.56 (d, 1H, *J*=8.6Hz), 7.45 (d, 2H, *J*=8.7Hz), 7.35 (d, 1H, *J*=8.5Hz), 7.12-7.10 (m, 2H), 6.37-6.36 (m, 2H), 1.37 (s, 9H) |
| 182 | 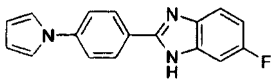 | 68 | ($CDCl_3$) δ: 8.08 (d, 2H, *J*=8.6Hz), 7.52 (d, 2H, *J*=8.6Hz), 7.42 (s, 1H), 7.34 (d, 1H, *J*=8.5Hz), 7.16-7.15 (m, 2H), 7.07-7.02 (m, 1H), 6.40-6.37 (m, 2H) |
| 183 | 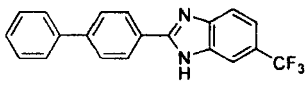 | 92 | ($CDCl_3$) δ: 8.15 (d, 2H, *J*=8.4Hz), 7.93 (s, 1H), 7.74-7.69 (m, 3H), 7.63 (d, 2H, *J*=7.0Hz), 7.53 (d, 1H, *J*=8.4Hz), 7.49-7.39 (m, 3H) |
| 184 | 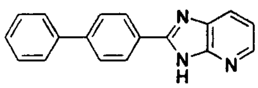 | 69 | ($CDCl_3$) δ: 8.38 (m, 1H), 8.31 (d, 2H, *J*=8.2Hz), 7.83 (d, 2H, *J*=8.2Hz), |
| | | | 7.73-7.64 (m, 4H), 7.51-7.43 (m, 2H), 7.34-7.30 (m, 1H) |
| 185 | 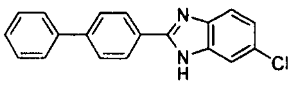 | 85 | ($CDCl_3$) δ: 8.11 (d, 2H, *J*=8.5Hz), 7.72 (d, 2H, *J*=8.5Hz), 7.64-7.57 (m, 4H), 7.50-7.36 (m, 3H), 7.27-7.24 (m, 1H) |
| 186 | 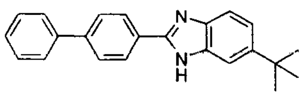 | 79 | ($CDCl_3$) δ: 8.12 (d, 2H, *J*=8.4Hz), 7.57 (d, 1H, *J*=1.5Hz), 7.52-7.47 (m, 5H), 7.42-7.33 (m, 3H), 7.28-7.25 (m, 1H), 1.29 (s, 9H) |
| 187 | 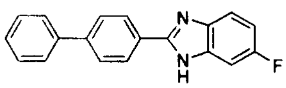 | 88 | ($CDCl_3$) δ: 8.10 (d, 2H, *J*=8.3Hz), 7.72 (d, 2H, *J*=8.3Hz), 7.60 (d, 2H, *J*=7.5Hz), 7.59-7.56 (m, 1H), 7.45 (d, 2H, *J*=7.5Hz), 7.41-7.33 (m, 2H), 7.07-7.00 (m, 1H) |
| 188 | 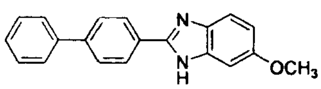 | 90 | ($CDCl_3$) δ: 8.10 (d, 2H, *J*=8.4Hz), 7.73-7.69 (m, 2H), 7.64-7.57 (m, 3H), 7.46-7.34 (m, 4H), 6.95-6.92 (m, 1H), 3.86 (s, 3H) |

(continued)

| Cpds. | Structures | Yield | Spectrum Data ($^1$H NMR) |
|---|---|---|---|
| 189 | 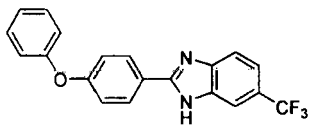 | 76 | ($CDCl_3$) δ: 8.00 (d, 2H, *J*=8.8Hz), 7.86 (s, 1H), 7.64 (d, 1H, *J*=8.4Hz), 7.49 (d, 1H, *J*=8.4Hz), 7.47-7.36 (m, 2H), 7.21-7.16 (m, 1H), 7.07-7.04 (m, 4H) |
| 190 | 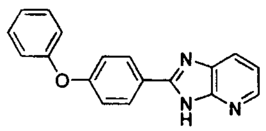 | 60 | ($CDCl_3$) δ: 8.30 (br, 1H), 8.18-8.14 (m, 3H), 7.44-7.39 (m, 2H), 7.31-7.29 (m, 1H), 7.22 (d, *J*=7.4Hz, 1H), 7.18-7.10 (m, 4H) |
| 191 | 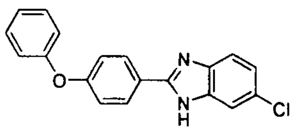 | 76 | ($CDCl_3$) δ: 8.00 (d, 2H, *J*=8.8Hz), 7.54 (d, 1H, *J*=1.5Hz), 7.49 (d, 1H, *J*=8.6Hz), 7.40-7.34 (m, 2H), 7.23-7.14 (m, 2H), 7.06-6.97 (m, 4H) |
| 192 | 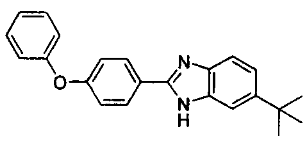 | 77 | ($CDCl_3$) δ: 8.02 (d, 2H, *J*=8.8Hz), 7.62 (s, 1H), 7.54 (d, 1H, *J*=8.6Hz), 7.40-7.31 (m, 3H), 7.16 (t, 1H, *J*=7.4Hz), 7.08-7.02 (m, 4H), 1.37 (s, 9H) |
| 193 | 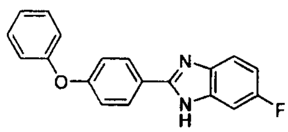 | 83 | ($CDCl_3$) δ: 8.00 (d, 2H, *J*=5.0Hz), 7.50-7.45 (m, 1H), 7.41-7.36 (m, 2H), 7.30-7.26 (m, 1H), 7.20-7.16 (m, 1H), 7.08-7.01 (m, 5H) |
| 194 | 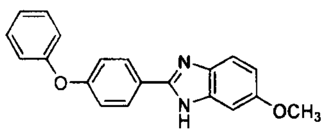 | 83 | ($CDCl_3$) δ: 7.98 (d, 2H, *J*=8.8Hz), 7.50 (d, 1H, *J*=8.8Hz), 7.42-7.35 (m, 2H), 7.18-7.13 (m, 1H), 7.08-7.01 (m, 5H), 6.89 (dd, 1H, *J*=8.8, 2.4Hz), 3.83 (s, 3H) |
| 195 | 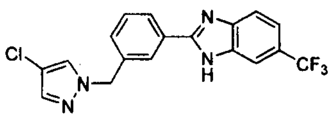 | 72 | ($CD_3OD$) δ: 8.19-8.15 (m, 2H), 8.03-7.96 (m, 2H), 7.87-7.81 (m, 1H), 7.68-7.56 (m, 3H), 7.55-7.52 (m, 1H), 5.52 (d, 2H) |
| 196 | 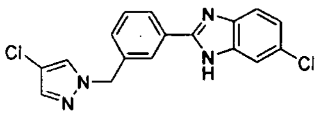 | 75 | ($CD_3OD$) δ: 8.15-8.11 (m, 2H), 7.99 (s, 1H), 7.71-7.62 (m, 4H), 7.52 (d, 1H), 7.36 (d, 1H), 5.53 (s, 2H) |
| 197 | 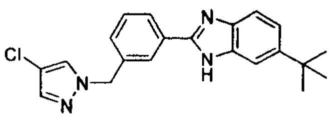 | 80 | ($CD_3OD$) δ: 8.15-8.12 (m, 2H), 7.99 (s, 1H), 7.73 (s, 1H), 7.68-7.60 (m, 3H), 7.50-7.48 (m, 2H), 5.54 (s, 2H), 1.53 (s, 9H) |
| 198 | 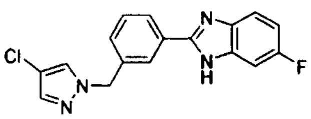 | 71 | ($CD_3OD$) δ: 8.15-8.11 (m, 2H), 7.99 (s, 1H), 7.72-7.63 (m, 3H), 7.52 (d, 1H), 7.41 (d, 1H), 7.21-7.12 (m, 1H), 5.54 (s, 2H) |
| 199 | 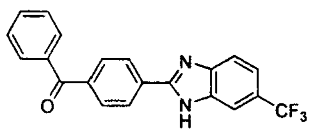 | 86 | ($CD_3OD$) δ: 8.39 (d, 2H), 8.10-8.06 (m, 3H), 7.94 (d, 2H), 7.90 (s, 1H), 7.79 (d, 1H), 7.72-7.68 (m, 3H) |

(continued)

| Cpds. | Structures | Yield | Spectrum Data ($^1H$ NMR) |
|---|---|---|---|
| 200 | 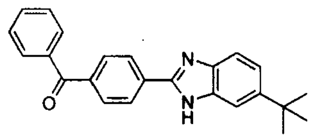 | 82 | ($CD_3OD$) δ: 8.35 (d, 2H), 8.05 (d, 2H), 7.94 (d, 2H), 7.82-7.77 (m, 2H), 7.71-7.66 (m, 3H), 7.54 (d, 1H), 1.54 (s, 9H) |

EXAMPLE 201: Preparation of 2-[4-(3-chloropyridin-2-yl)phenyl]-6-trifluoromethyl-1H-benzoimidazole chloride

500mg (1.34mmol) of 2-[4-(3-chloropyridin-2-yl)phenyl]-6-trifluoromethyl-1H-benzoimidazole was dissolved in 10mL of diethylether, followed by stirring for 30 min at a subzero temperature. After saturation with gaseous hydrochloric acid, the solution was stirred for an additional one hour at a subzero temperature. A white precipitate thus obtained was filtered and dried for 24 hours in a vacuum condition to give 513mg of 2-[4-(3-chloropyridin-2-yl)phenyl]-6-trifluoromethyl-1H-benzoimidazole chloride.
$^1H$ NMR ($CD_3OD$) δ: 8.75 (d, 1H), 8.38 (d, 2H), 8.29 (d, 1H), 8.23 (s, 1H), 8.13 (d, 2H), 8.10 (d, 1H), 7.97 (d, 1H), 7.70(dd, 1H)
mp : 291 - 293°C

EXAMPLE 202: Preparation of 6-tert-butyl-2-[4-(3-chloropyridin-2-yl)phenyl]-1H-benzoimidazole chloride

The same procedure as in Example 201 was performed to produce 500 mg of 6-tert-butyl-2-[4-(3-chloropyridin-2-yl)phenyl]-1H-benzoimidazole chloride.
$^1H$ NMR ($CD_3OD$) δ: 8.73 (d, 1H), 8.30 (d, 2H), 8.26 (d, 1H), 8.10 (d, 2H), 7.83 (d, 2H), 7.81 (s, 1H), 7.67 (dd, 1H), 1.47 (s, 9H)
mp : 189 ~ 190°C

EXAMPLE 203: Preparation of 6-tert-butyl-2-[4-(3-chloropyridin-2-yl)naphthalen-1-yl]-1H-benzoimidazole chloride

The same procedure as in Example 201 was performed to produce 510 mg of 6-*tert*-butyl-2-[4-(3-chloropyridin-2-yl)naphthalen-1-yl]-1H-benzoimidazole chloride.
$^1H$ NMR ($CD_3OD$) δ: 8.68 (d, 1H), 8.20 (d, 1H), 8.16-8.11 (m, 2H), 7.83-7.72 (m, 5H), 7.66-7.62 (m, 2H), 7.54 (d, 1H), 1.41 (s, 9H)
mp: 208 - 214°C

EXAMPLE 204: Preparation of 6'-(4,6-bistrifluoromethyl-1H-benzoimidazole-2-yl)-3-chloro[2,3']bipyridine chloride

The same procedure as in Example 201 was performed to produce 520 mg of 6'-(4,6-bistrifluoromethyl-1H-benzoimidazole-2-yl)-3-chloro[2,3']bipyridine chloride.
$^1H$ NMR ($CD_3OD$) δ: 9.03 (s, 1H), 8.61-8.53 (m, 2H), 8.30 (d, 1H), 8.15 (s, 1H), 8.03 (d, 1H), 7.77 (s, 1H), 7.47-7.43 (m, 1H)
mp: 133 - 135°C

EXAMPLE 205: Preparation of 3-trifluoromethyl-6'-(6-trifluoromethyl-1H-benzoimidazole-2-yl)-[2,3']bipyridine chloride

The same procedure as in Example 201 was performed to produce 495 mg of 3-trifluoromethyl-6'-(6-trifluoromethyl-1H-benzoimidazole-2-yl)-[2,3']bipyridine chloride.
$^1H$ NMR ($CD_3OD$) δ: 9.00 (s, 1H), 8.91 (d, 1H), 8.46 (d, 1H), 8.35-8.28 (m, 2H), 8.17 (s, 1H), 8.04 (d, 1H), 7.90 (d, 1H), 7.73-7.69 (m, 1H)
mp: 234 - 240°C

EXAMPLE 206: Preparation of 6-*tert*-butyl-2-[4-(3-chloropyridin-2-yl)phenyl]-1H-benzoimidazole sodium salt

A solution of 100mg (0.28mmol) of 6-*tert*-butyl-2-[4-(3-chloropyridin-2-yl)phenyl]-1H-benzoimidazole in 2.8mL of 0.1N sodium hydroxide and 0.5mL of methanol was stirred at room temperature for 30 min and vacuum concentrated to produce 107 mg of 6-*tert*-butyl-2-[4-(3-chloropyridin-2-yl)phenyl]-1H-benzoimidazole sodium salt as a pale yellow precipitate.

$^1$H NMR ($CD_3OD$) δ: 8.60 (d, 1H), 8.22 (d, 2H), 8.04 (d, 1H), 7.85 (d, 2H), 7.65 (s, 1H), 7.57 (d, 1H), 7.48-7.38 (m, 2H), 1.43 (s, 9H).
mp: 317 - 324°C

EXAMPLE 207: Preparation of 3-trifluoromethyl-6'-(6-trifluoromethyl-1H-benzoimidazole-2-yl)-[2,3']bipyridine sulfate

1.0g (2.45mmol) of a pyridine compound was dissolved in 30 mL of acetone and stirred for 30 min at a subzero temperature. To this, a dilution of 0.16 mL (2.94mmol) of sulfate in 20 mL of acetone was dropwisely added over 10 min. Stirring for one hour at a subzero temperature produced a white precipitate which was then vacuum dried to produce 1.22 g of 3-trifluoromethyl-6'-(6-trifluoromethyl-1H-benzoimidazole-2-yl)-[2,3']bipyridine sulfate.
$^1$H NMR ($CD_3OD$) δ: 9.07 (s, 1H), 8.99 (d, 1H), 8.55 (d, 1H), 8.46 (d, 1H), 8.37 (dd, 1H), 8.26 (s, 1H), 8.12 (d, 1H), 7.97 (d, 1H), 7.80-7.74 (m, 1H)

EXAMPLE 208: Preparation of 3-trifluoromethyl-6'-(6-trifluoromethyl-1H-benzoimidazole-2-yl)-[2,3']bipyridine phosphate

1.0g (2.45mmol) of a bipyridine compound was dissolved in 30mL of acetone and stirred for 30 min at a freezing temperature. To this, a dilution of 0.16 mL (2.94mmol) of phosphoric acid in 20 mL of acetone was added dropwise over 10 min. Stirring for one hour at a freezing temperature produced a white precipitate which was then vacuum dried to produce 1.23 g of 3-trifluoromethyl-6'-(6-trifluoromethyl-1H-benzoimidazole-2-yl)-[2,3']bipyridine phosphate.
$^1$H NMR ($CD_3OD$) δ: 8.92 (dd, 1H), 8.85 (s, 1H), 8.45 (d, 1H), 8.34 (d, 1H), 8.14 (dd, 1H), 8.00 (s, 1H), 7.83 (d, 1H), 7.72-7.70 (m, 1H), 7.59 (d, 1H)

EXAMPLE 209: Preparation of 3-trifluoromethyl-6'-(6-trifluoromethyl-1H-benzoimidazole-2-yl)-[2,3']bipyridine methanesulfonate

1.0g (2.45mmol) of a bipyridine compound was dissolved in 30mL of diethylether and stirred for 30 min under a glacial condition. To this, a dilution of 0.19mL (2.94mmol) of methanesulfonic acid in 20 mL of diethylether was added dropwise over 10 min. Stirring for one hour at a freezing temperature produced a white precipitate which was then vacuum dried to produce 1.25 g of 3-trifluoromethyl-6'-(6-trifluoromethyl-1H-benzoimidazole-2-yl)-[2,3']bipyridine methanesulfonate.
$^1$H NMR ($CD_3OD$) δ: 9.05 (s, 1H), 8.96 (d, 1H), 8.52 (d, 1H), 8.41-8.32 (m, 2H), 8.24 (s, 1H), 8.10 (d, 1H), 7.95 (d, 1H), 7.76 (dd, 1H), 2.72 (s, 3H)

EXAMPLE 210: Preparation of 3-trifluoromethyl-6'-(6-trifluoromethyl-1H-benzoimidazole-2-yl)-[2,3']bipyridine benzenesulfonate

1.0g (2.45mmol) of a bipyridine compound was dissolved in 30mL of diethylether and stirred for 30 min at a freezing temperature. To this, a dilution of 0.85 mg (5.39mmol) of benzenesulfonic acid in 20 mL of diethylether was added dropwise over 10 min. Stirring for one hour at a freezing temperature produced a white precipitate which was then vacuum dried to produce 1.39 g of 3-trifluoromethyl-6'-(6-trifluoromethyl-1H-benzoimidazole-2-yl)-[2,3']bipyridine benzenesulfonate.
$^1$H NMR ($CD_3OD$) δ: 9.07 (s, 1H), 9.00 (d, 1H), 8.53 (d, 1H), 8.49 (d, 1H), 8.31 (d, 2H), 8.23 (s, 1H), 8.10 (d, 1H), 7.97 (d, 1H), 7.84-7.80 (m, 6H), 7.44-7.38 (m, 7.5H)

EXPERIMENTAL EXAMPLE 1: Assay for Inhibitory Activity against Vanilloid Receptor

1) Preparation of test compounds

Each of the compounds synthesized in the examples were dissolved in dimethyl sulfoxide (DMSO) at a concentration of 10mM. For use in the assay for inhibitory activity against patch clamp activity, the solutions were diluted to a concentration of 1 μM with the same physiological saline as in the pipette solution of Table 1, below.

2) Assay for inhibitory activity against VR1 patch clamp activity

In this experiment, changes in VR1 activity were recorded in current using an electrophysiological technique.
For use in this experiment, a rat TRPV1 cDNA was transfected into a subcultured HEK293 strain with the aid of lipofectamin.

The rat TRPV1 gene-transfected HEK293 cells were detached from the cell culture dishes with trypsin and distributed on a patch clamp recording chamber.

A cover glass slip on the bottom of the patch clamp recording chamber was treated for 15 min or longer with a 50% poly-L-lysine solution so that the HEK cells would readily adhere thereto. Cellular membranes wer ruptured at room temperature (~25°C) from randomly selected HEK cells to perform an inside-out patch clamp technique. 10 μM of capsaicin was fed into the chamber (cytoplasmic side) to activate a capsaicin current through the VR1. When the capsaicin current reached a stable stage, each compound was added along with 10 μM of capsaicin to record the inhibitory activity of the compounds with regard to the current. The inhibition was expressed as percentages. Because the capsaicin current showed rapid run-down in the presence of calcium ions across the membrane, experimental solutions comprising the ion compositions of Table 1 were used to prevent the run-down and record the inhibitory activity of effective compounds.

TABLE 1. Experimental Solution Composition for Capsaicin Current Recordings

| (in mM) | Pipette Solution | Bath Solution |
|---|---|---|
| NaCl | 140 | 140 |
| HEPES | 10 | 10 |
| EGTA | 0 | 2 |
| pH 7.3 adjusted with | NMG | NMG |

Detected current was amplified using a Digidata 1200B A/D converter and an Axopatch 1D amplifier (Axon instruments Inc.), and the data were recorded and analyzed with the aid of a computer. The inhibitory activity of each compound was expressed as a percentage relative to the capsaicin current immediately before the administration of each compound.

TABLE 2. Assay for Inhibitory Activity of Test Compounds Against VR1 Activity

| Cpds | Inhibition % (1μ M) | Cpds | Inhibition % (1μ M) |
|---|---|---|---|
| 1 | 100 | 116 | 30 |
| 2 | 45 | 117 | 80 |
| 3 | 100 | 118 | 93 |
| 5 | 10 | 121 | 76 |
| 6 | 94 | 126 | 87 |
| 8 | 41 | 138 | 73 |
| 10 | 80 | 139 | 89 |
| 29 | 50 | 151 | 84 |
| 30 | 96 | 158 | 69 |
| 31 | 66 | 161 | 58 |
| 32 | 78 | 162 | 99 |
| 33 | 10 | 178 | 20 |
| 34 | 16 | 179 | 100 |
| 67 | 80 | 181 | 40 |
| 70 | 45 | 183 | 17 |
| 104 | 86 | 189 | 90 |
| 105 | 78 | 191 | 60 |
| 107 | 97 | 193 | 50 |
| 115 | 71 | 194 | 30 |

EXPERIMENTAL EXAMPLE 2: Calcium Influx of Vanilloid Receptor

Experiments for calcium influx were performed to measure the activity of the compounds as antagonists.

1) Cell culture

The cell strain hVR1-HEK293 is the human embryonic kidney(HEK) 293 Tet-on cell transfected with a human vanilloid-1 gene (pTRE2hyg-hVR1, 7.8kb), which is capable of controlling the expression of VR1 according to the administration of deoxycycline, a tetracycline derivative analog. In a medium supplemented with deoxycycline two days before calcium influx experiments, the cell strain was cultured to induce the expression of VR1. After being grown to a confluency of about 80% in T75 flasks, hVR1-HEK293 was detached from the flask using trypsin. Cells harvested by centrifugation were suspended in a medium supplemented with 1μg/mL of deoxycycline and diluted to a density of 2-4 x $10^5$ cells/mL, after which a 100 μL aliquot of the cell suspension was added to each well of 96 well black plates. Before the measurement of calcium influx, the cells were incubated for two days at 37°C in a 5% $CO_2$ incubator.

2) Preparation of compound samples

For use in calcium influx experiments, each compound was dissolved in dimethyl sulfoxide (DMSO).

3) Measurement of calcium influx

To measure an intracellular calcium influx, cells were incubated at 37°C for 90 min in a medium containing the calcium indicator Fluo-3/AM so as to allow the fluorescent dye to penetrate into the cells. Then, the cells were washed three times with D-PBS (Dulbecco's phosphate buffered saline) containing 10mM HEPES to remove the fluorescent dye which remained outside. 193μL aliquot of D-PBS was added to each well, followed by the addition of various concentrations of compounds thereto. In order to measure the antagonistic activity of the compounds, calcium influx was induced by treatment with 1μM capsaicin. The inhibitory effect of each compound on 1μM capsaicin-induced intracellular calcium influx according to compound concentration was measured using a fluorescent analyzer and the data thus obtained were analyzed on the basis of the Hill's equation.

4) Measurement results

TABLE 3: Inhibitory Activity of Compounds Against Calcium Influx

| Cpds | Inhibitory Activity $IC_{50}$ (nM) | Cpds | Inhibitory Activity $IC_{50}$ (nM) |
|---|---|---|---|
| 1 | 130 | 82 | 263 |
| 2 | 8570 | 97 | 644 |
| 3 | 61 | 104 | 31 |
| 5 | 6038 | 105 | 24 |
| 6 | 11 | 106 | 275 |
| 8 | 31 | 107 | 17 |
| 10 | 114 | 113 | 363 |
| 12 | 70 | 117 | 525 |
| 13 | 289 | 118 | 313 |
| 14 | 168 | 126 | 105 |
| 25 | 100 | 129 | 3963 |
| 29 | 4 | 130 | 436 |
| 30 | 23 | 136 | 952 |
| 31 | 8 | 137 | 569 |

(continued)

| Cpds | Inhibitory Activity $IC_{50}$ (nM) | Cpds | Inhibitory Activity $IC_{50}$ (nM) |
|---|---|---|---|
| 32 | 13 | 138 | 16 |
| 33 | 10 | 139 | 38 |
| 34 | 26 | 140 | 66 |
| 35 | 39 | 141 | 2402 |
| 36 | 94 | 151 | 97 |
| 37 | 75 | 158 | 14 |
| 38 | 454 | 159 | 3946 |
| 39 | 19 | 160 | 258 |
| 47 | 82 | 161 | 23 |
| 48 | 160 | 162 | 14 |
| 49 | 284 | 163 | 15 |
| 50 | 292 | 164 | 9 |
| 51 | 246 | 165 | 28 |
| 52 | 1404 | 166 | 3901 |
| 53 | 145 | 173 | 46 |
| 56 | 22 | 174 | 282 |
| 58 | 145 | 176 | 229 |
| 59 | 296 | 177 | 151 |
| 60 | 150 | 181 | 221 |
| 67 | 122 | 195 | 1405 |

EXPERIMENTAL EXAMPLE 3: Assay for Analgesic Effect

A PBQ-induced writhing test was conducted in mice to assay for the analgesic effect of the novel compounds. Five-week-old ICR male mice were treated with PBQ (phenyl-p-quinone, 0.02%). A suspension of 20 mg of test material in 10 mL of physiological saline was used per kg of weight of mice. Test materials in combination with a carrier were orally administered one hour before the intraabdominal injection of PBQ in a dose of 10 mL per kg weight. From 5 min after the injection, individual mice in each test group were measured for numbers of writhes at regular intervals of 10 min. Analgesic effects of test compounds were expressed as percentages of the decrease in number of writhes compared to a control.

[% Inhibition=(Control administered with carrier alone - Group administerd with test material)/control administered with carrier alone X 100]

TABLE 4. Analgesic Effects of Test Compounds

| Cpds. | % Inhibition | Cpds. | % Inhibition |
|---|---|---|---|
| 1 | 33 | 105 | 50 |
| 3 | 58 | 107 | 46 |
| 6 | 33 | 138 | 22 |

(continued)

| Cpds. | % Inhibition | Cpds. | % Inhibition |
|---|---|---|---|
| 12 | 35 | 139 | 26 |
| 25 | 23 | 158 | 53 |
| 29 | 41 | 162 | 28 |
| 31 | 35 | 163 | 41 |
| 33 | 31 | 164 | 53 |
| 36 | 70 | 165 | 60 |
| 37 | 50 | 167 | 55 |
| 67 | 23 | 169 | 57 |

EXPERIMENTAL TEST 4: Toxicity Test

The novel compounds were assayed for acute oral toxicity in mice.

To four groups of 10 ICR lineage male mice, the compounds of the present invention, 2-[4-(3-chloropyridin-2-yl)-phenyl]-6-trifluoromethyl-1H-benzoimidazole(1), 6-*tert*-butyl-2-[4-(3-chloropyridin-2-yl)phenyl]-1H-benzoimidazole(3), N-[4'-(4,6-bistrifluoromethyl-1H-benzoimidazole-2-yl)-3-fluorobiphenyl-4-yl]methanesulfonamide(29), 4-bromo-6-(trifluoromethyl)-2-(4-(3-(trifluoromethyl)pyridin-2-yl)phenyl)-1H-benzoimidazole(36), 6-*tert*-butyl-2-[4-(3-chloropyridin-2-yl)naphthalen-1-yl]-1H-benzoimidazole(105), 3-trifluoromethyl-6'-(6-trifluoromethyl-1H-benzoimidazole-2-yl)-[2,3']bipyridine(158), 6-bromo-2-(5-(3-(trifluoromethyl)pyridin-2-yl)pyridin-2-yl)-1H-benzoimidazole(165) and 6'-(6-morpholin-4-yl-1H-benzoimidazole-2-yl)-3-trifluoromethyl-[2,3']bipyridine(169) were orally administered at doses of zero mg/kg for control, and 125, 250 and 500 mg/kg for test groups, and observations were made of the death, clinical symptoms, and weight changes, and microscopic diagnosis of the animals for 14 days.

None of the mice to which the compounds of interest were administered died or differed from the control with respect to clinical symptoms, weight change, and microscopic diagnosis, indicating that the compounds of the present invention are safe.

Industrial Applicability

As described hereinbefore, the compounds of the present invention are useful in the prevention and treatment of pain, acute pain, chronic pain, neuropathic pain, postoperative pain, migraines, arthralgia, neuropathy, nerve injury, diabetic neuropathy, neurological illness, neurodermatitis, strokes, bladder hypersensitivity, irritable bowel syndrome, respiratory disorders such as asthma, chronic obstructive pulmonary disease, etc., burns, psoriasis, itching, vomiting, irritation of the skin, eyes, and mucous membranes, gastric-duodenal ulcers, inflammatory intestinal diseases, and inflammatory diseases.

Although the preferred embodiments of the present invention have been disclosed for illustrative purposes, those skilled in the art will appreciate that various modifications, additions and substitutions are possible, without departing from the scope of the invention as disclosed in the accompanying claims.

## Claims

**1.** A compound, or a pharmaceutically acceptable salt thereof, selected from the group consisting of

2-[4-(3-chloropyridin-2-yl)phenyl]-6-trifluoromethyl-1H-benzoimidazole,
6-chloro-2-[4-(3-chloropyridin-2-yl)phenyl]-1H-benzoimidazole,
6-*tert*-butyl-2-[4-(3-chloropyridin-2-yl)phenyl]-1H-benzoimidazole,
2-[4-(3-chloropyridin-2-yl)phenyl]-6-fluoro-1H-benzoimidazole,
2-[4-(3-chloropyridin-2-yl)phenyl]-6-methoxy-1H-benzoimidazole,
4-chloro-2-[4-(3-chloropyridin-2-yl)phenyl]-6-trifluoromethyl-1H-benzoimidazole,
2-[4-(3-chloropyridin-2-yl)phenyl]-4,6-bistrifluoromethyl-1H-benzoimidazole,
2-[4-(3-chloropyridin-2-yl)phenyl]-6-trifluoromethyl-1H-imidazo[4,5-b]pyridine,
2-[4-(3-chloropyridin-2-yl)phenyl]-3H-imidazo[4,5-b]pyridine,

2-[4-(3-chloropyridin-2-yl)phenyl]-6,7-dimethyl-1H-benzoimidazole,
2-[4-(3-chloropyridin-2-yl)phenyl]-1H-benzoimidazole,
2-[4-(3-chloropyridin-2-yl)phenyl]-5,6-dichloro-1H-benzoimidazole,
6-bromo-2-(4-(3-chloropyridin-2-yl)phenyl)-1H-benzoimidazole,
4-bromo-2-(4-(3-chloropyridin-2-yl)phenyl)-6-(trifluoromethyl)-1H-benzoimidazole,
6-bromo-2-(4-(3-chloropyridin-2-yl)phenyl-1H-imidazo[4,5-b]pyridine,
4,6-dibromo-2-[4-(3-chloropyridin-2-yl)phenyl]-1H-benzoimidazole,
2-[4-(3-chloropyridin-2-yl)phenyl]-6-morpholin-4-yl-1H-benzoimidazole,
2-[4-(3-chloropyridin-2-yl)phenyl]-6-thiomorpholin-4-yl-1H-benzoimidazole,
2-[4-(3-chloropyridin-2-yl)phenyl]-4-morpholin-4-yl-6-trifluoromethyl-1H-benzoimidazole,
2-[4-(3-chloropyridin-2-yl)phenyl]-4-thiomorpholin-4-yl-6-trifluoromethyl-1H-benzoimidazole,
2-[4-(3-chloropyridin-2-yl)phenyl]-4-pyrrolidin-1-yl-6-trifluoromethyl-1H-benzoimidazole,
{2-[4-(3-chloropyridin-2-yl)phenyl]-6-trifluoromethyl-1H-benzoimidazole-4-yl}diethylamine,
2-[4-(3-chloropyridin-2-yl)phenyl]-6-morpholin-4-yl-1H-imidazo[4,5-b]pyridine,
2-[4-(3-chloropyridin-2-yl)phenyl]-6-pyrrolidin-1-yl-1H-imidazo[4,5-b]pyridine,
6-tert-butyl-2-[4-(3,5-dichloropyridin-2-yl)phenyl]-1H-benzoimidazole,
2-[4-(3,5-dichloropyridin-2-yl)phenyl]-4,6-bistrifluoromethyl-1H-benzoimidazole,
6-*tert*-butyl-2-[4-(3-chloro-5-trifluoromethyl-pyridin-2-yl)phenyl]-1H-benzoimidazole,
2-[4-(3-chloro-5-trifluoromethylpyridin-2-yl)phenyl]-4,6-bistrifluoromethyl-1H-benzoimidazole,
2-[4-(3-chloro-5-trifluoromethylpyridin-2-yl)phenyl]-5,7-bistrifluoromethyl-1H-benzoimidazole,
N-[4'-(4,6-bistrifluoromethyl-1H-benzoimidazole-2-yl)-3-fluorobiphenyl-4-yl]methanesulfonamide,
6-*tert*-butyl-2-[4-(3-trifluoromethylpyridin-2-yl)phenyl]-1H-benzoimidazole,
4-chloro-6-trifluoromethyl-2-[4-(3-trifluoromethylpyridin-2-yl)phenyl]-1H-benzoimidazole,
6-chloro-2-[4-(3-trifluoromethylpyridin-2-yl)phenyl]-1H-benzoimidazole,
4,6-bistrifluoromethyl-2-[4-(3-trifluoromethyl pyridin-2-yl)phenyl]-1H-benzoimidazole,
6-trifluoromethyl-2-[4-(3-trifluoromethylpyridin-2-yl)phenyl]-1H-imidazo[4,5-b]pyridine,
5,6-dichloro-2-[4-(3-trifluoromethylpyridin-2-yl)phenyl]-1H-benzoimidazole,
4-bromo-6-(trifluoromethyl)-2-(4-(3-(trifluoro-methyl)pyridin-2-yl)phenyl)-1H-benzoimidazole,
6-bromo-2-(4-(3-(trifluoromethyl)pyridin-2-yl)phenyl)-1H-benzoimidazole,
6-fluoro-2-(4-(3-(trifluoromethyl)pyridin-2-yl)phenyl)-1H-benzoimidazole,
6-bromo-2-(4-(3-(trifluoromethyl)pyridin-2-yl)phenyl-1H-imidazo[4,5-b]pyridine,
4,6-dibromo-2-[4-(3-trifluoromethylpyridin-2-yl)phenyl]-1H-benzoimidazole,
6-morpholin-4-yl-2-[4-(3-trifluoromethylpyridin-2-yl)phenyl]-1H-benzoimidazole,
6-thiomorpholin-4-yl-2-[4-(3-trifluoromethyl-pyridin-2-yl)phenyl]-1H-benzoimidazole,
diethyl-{2-[4-(3-trifluoromethylpyridin-2-yl)phenyl]-3H-benzoimidazo-5-yl}amine,
4-morpholin-4-yl-6-trifluoromethyl-2-[4-(3-trifluoromethylpyridin-2-yl)phenyl]-1H-benzoimidazole,
4-thiomorpholin-4-yl-6-trifluoromethyl-2-[4-(3-trifluoromethylpyridin-2-yl)phenyl]-1H-benzoimidazole,
4-pyrrolidin-1-yl-6-trifluoromethyl-2-[4-(3-trifluoromethylpyridin-2-yl)phenyl]-1H-benzoimidazole,
6-*tert*-butyl-2-(4-pyridin-2-ylphenyl)-1H-benzoimidazole,
6-((trifluoromethyl)-2-(4-(pyridin-2-yl)phenyl)-1H-benzoimidazole,
4-chloro-6-(trifluoromethyl)-2-(4-(pyridin-2-yl)phenyl)-1H-benzoimidazole,
5,6-dichloro-2-(4-(pyridin-2-yl)phenyl)-1H-benzoimidazole,
4,6-bis(trifluoromethyl)-2-(4-(pyridin-2-yl)phenyl)-1H-benzoimidazole,
6-fluoro-2-(4-(pyridin-2-yl)phenyl)-1H-benzoimidazole,
6-bromo-2-(4-(pyridin-2-yl)phenyl)-1H-imidazo[4,5-b]pyridine,
4,6-dibromo-2-(4-pyridin-2-ylphenyl)-1H-benzoimidazole,
2-[4-(6-chloro-5-methylpyridazin-3-yl)phenyl]-4,6-bistrifluoromethyl-1H-benzoimidazole,
2-[4-(6-chloro-5-methylpyridazin-3-yl)phenyl]-5,7-bistrifluoromethyl-1H-benzoimidazole,
4-bromo-6-(trifluoromethyl)-2-(4-(4-(trifluoro-methyl)pyrimidin-2-yl)phenyl)-1H-benzoimidazole,
4,6-dibromo-2-[4-(4-trifluoromethylpyrimidin-2-yl)phenyl]-1H-benzoimidazole,
6-*tert*-butyl-2-(4-(pyrimidin-2-yl)phenyl)-1H-benzoimidazole,
2-((4-(6-(trifluoromethyl)-1H-benzoimidazole-2-yl)phenyl)pyridin-3-carbonitrile,
2-((4-(6-(*tert*-butyl-1H-benzoimidazole-2-yl)phenyl)pyridin-3-carbonitrile,
6'-(4,6-dibromo-1H-benzoimidazole-2-yl)-[2,3']bipyridyl-3-carbonitrile,
2-[4-(3-chloropyrazin-2-yl)phenyl]-6-trifluoromethyl-1H-benzoimidazole,
6-*tert*-butyl-2-[4-(3-chloropyrazin-2-yl)phenyl]-1H-benzoimidazole,
6-bromo-2-[4-(3-chloropyrazin-2-yl)phenyl]-1H-benzoimidazole,
5,6-dichloro-2-[4-(3-chloropyrazin-2-yl)phenyl]-1H-benzoimidazole,

6-bromo-2-[4-(3-chloropyrazin-2-yl)phenyl]-1H-imidazo[4,5-b]pyridine,
6-tert-butyl-2-[4-(3-chloropyridin-2-yl)-2-fluorophenyl]-1H-benzoimidazole,
4-chloro-2-[4-(3-chloropyridin-2-yl)-2-fluorophenyl]-6-trifluoromethyl-1H-benzoimidazole,
2-[4-(3-chloropyridin-2-yl)-2-fluorophenyl]-6-methoxy-1H-benzoimidazole,
6-*tert*-butyl-2-[4-(3,5-dichloropyridin-2-yl)-2-fluorophenyl]-1H-benzoimidazole,
4-chloro-2-[4-(3,5-dichloropyridin-2-yl)-2-fluorophenyl]-6-trifluoromethyl-1H-benzoimidazole,
2-[4-(3,5-dichloropyridin-2-yl)-2-fluorophenyl]-4,6-bistrifluoromethyl-1H-benzoimidazole,
4-chloro-2-[4-(3-chloro-5-trifluoromethylpyridin-2-yl)-2-fluorophenyl]-6-trifluoromethyl-1H-benzoimidazole,
2-[4-(3-chloro-5-trifluoromethylpyridin-2-yl)-2-fluorophenyl]-6-trifluoromethyl-1H-imidazo[4,5-b]pyridine,
2-[4-(3-chloro-5-trifluoromethylpyridin-2-yl)-2-fluorophenyl]-6-methoxy-1H-benzoimidazole,
N-[3,3'-difluoro-4'-(6-methoxy-1H-benzoimidazole-2-yl)biphenyl-4-yl]methanesulfonamide,
2-[2-fluoro-4-(3-trifluoromethylpyridin-2-yl)phenyl]-6-trifluoromethyl-1H-benzoimidazole,
6-tert-butyl-2-[2-fluoro-4-(3-trifluoromethyl-pyridin-2-yl)phenyl]-1H-benzoimidazole,
6-chloro-2-[2-fluoro-4-(3-trifluoromethylpyridin-2-yl)phenyl]-1H-benzoimidazole,
5-chloro-2-[2-fluoro-4-(3-trifluoromethylpyridin-2-yl)phenyl]-1H-benzoimidazole,
4-chloro-2-[2-fluoro-4-(3-trifluoromethylpyridin-2-yl)phenyl]-6-trifluoromethyl-1H-benzoimidazole,
7-chloro-2-[2-fluoro-4-(3-trifluoromethylpyridin-2-yl)phenyl]-5-trifluoromethyl-1H-benzoimidazole,
2-((2-fluoro-4-pyridin-2-yl)phenyl-6-trifluoromethyl-1H-benzoimidazole,
6-*tert*-butyl-2-(2-fluoro-4-pyridin-2-yl)phenyl-1H-benzoimidazole,
6-chloro-2-(2-fluoro-4-pyridin-2-yl)phenyl-1H-benzoimidazole,
2-((2-fluoro-4-pyridin-2-yl)phenyl-4,6-bistrifluoro-methyl-1H-benzoimidazole,
2-[2-chloro-4-(3-chloropyridin-2-yl)phenyl]-6-trifluoromethyl-1H-benzoimidazole,
4-chloro-2-[2-chloro-4-(3-chloropyridin-2-yl)phenyl]-6-trifluoromethyl-1H-benzoimidazole,
2-[2-chloro-4-(3-chloropyridin-2-yl)phenyl]-6-trifluoromethyl-1H-imidazo[4,5-b]pyridine,
2-[2-chloro-4-(3,5-dichloropyridin-2-yl)phenyl]-6-trifluoromethyl-1H-benzoimidazole,
4-chloro-2-[2-chloro-4-(3,5-dichloropyridin-2-yl)phenyl]-6-trifluoromethyl-1H-benzoimidazole,
2-[2-chloro-4-(3,5-dichloropyridin-2-yl)phenyl]-4,6-bistrifluoromethyl-1H-benzoimidazole,
2-[2-chloro-4-(3,5-dichloropyridin-2-yl)phenyl]-6-trifluoromethyl-1H-imidazo[4,5-b]pyridine,
2-[2-chloro-4-(3-chloro-5-trifluoromethylpyridin-2-yl)phenyl]-6-trifluoromethyl-1H-benzoimidazole,
4-chloro-2-[2-chloro-4-(3-chloro-5-trifluoro-methylpyridin-2-yl)phenyl]-6-trifluoromethyl-1H-benzoimidazole,
N-[4'-(6-*tert*-butyl-1H-benzoimidazole-2-yl)-3'-chloro-3-fluorobiphenyl-4-yl]methanesulfonamide,
N-[3'-chloro-4'-(4-chloro-6-trifluoromethyl-1H-benzoimidazole-2-yl)-3-fluorobiphenyl-4-yl]metharie-sulfonamide,
N-[4'-(4,6-bistrifluoromethyl-1H-benzoimidazole-2-yl)-3'-chloro-3-fluorobiphenyl-4-yl]methanesulfonamide,
2-[2-chloro-4-(3-trifluoromethylpyridin-2-yl)phenyl]-6-trifluoromethyl-1H-benzoimidazole,
4-chloro-2-[2-chloro-4-(3-trifluoromethylpyridin-2-yl)phenyl]-6-trifluoromethyl-1H-benzoimidazole,
2-[2-chloro-4-(3-trifluoromethylpyridin-2-yl)phenyl]-4,6-bistrifluoromethyl-1H-benzoimidazole,
6-*tert*-butyl-2-[2-chloro-4-(3-methylpyridin-2-yl)phenyl]-1H-benzoimidazole,
6-chloro-2-(2-chloro-4-pyridin-2-ylphenyl)-1H-benzoimidazole,
2-((2-chloro-4-pyridin-2-ylphenyl)-6-trifluoromethyl-1H-imidazo[4,5-b]pyridine,
2-((2-chloro-4-pyridin-2-ylphenyl)-6-methoxy-1H-benzoimidazole,
2-[4-(3-chloropyridin-2-yl)naphthalen-1-yl]-6-trifluoromethyl-1H-benzoimidazole,
6-*tert*-butyl-2-[4-(3-chloropyridin-2-yl)naphthalen-1-yl]-1H-benzoimidazole,
2-[4-(3-chloropyridin-2-yl)naphthalen-1-yl]-4,6-bistrifluoromethyl-1H-benzoimidazole,
2-[4-(3-chloropyridin-2-yl)naphthalen-1-yl]-6-trifluoromethyl-1H-imidazo[4,5-b]pyridine,
6-*tert*-butyl-2-[4-(3,5-dichloropyridin-2-yl)naphthalen-1-yl]-1H-benzoimidazole,
2-[4-(3,5-dichloropyridin-2-yl)naphthalen-1-yl]-4,6-bistrifluoromethyl-1H-benzoimidazole,
6-*tert*-butyl-2-[4-(3-chloro-5-trifluoromethyl-pyridin-2-yl)naphthalen-1-yl]-1H-benzoimidazole,
2-[4-(3-chloro-5-trifluoromethylpyridin-2-yl)naphthalen-1-yl]-4,6-bistrifluoromethyl-1H-benzoimidazole,
N-{4-[4-(4,6-bistrifluoromethyl-1H-benzoimidazole-2-yl)naphthalen-1-yl]-2-fluorophenyl}methanesulfonamide,
6-trifluoromethyl-2-[4-(3-trifluoromethylpyridin-2-yl)naphthalen-1-yl]-1H-benzoimidazole,
6-*tert*-butyl-2-(4-pyridin-2-ylnaphthalen-1-yl)-1H-benzoimidazole,
2-((4-pyridin-2-ylnaphthalen-1-yl)-4,6-bistrifluoromethyl-1H-benzoimidazole,
6-*tert*-butyl-2-[3-chloro-4-(3-chloropyridin-2-yl)phenyl]-1H-benzoimidazole,
2-[3-chloro-4-(3,5-dichloropyridin-2-yl)phenyl]-6-trifluoromethyl-1H-benzoimidazole,
6-tert-butyl-2-[3-chloro-4-(3,5-dichloropyridin-2-yl)phenyl]-1H-benzoimidazole,
2-[3-chloro-4-(3,5-dichloropyridin-2-yl)phenyl]-6-methoxy-1H-benzoimidazole,
2-[3-chloro-4-(3,5-dichloropyridin-2-yl)phenyl]-5-methoxy-1H-benzoimidazole,

2-[3-chloro-4-(3-chloro-5-trifluoromethylpyridin-2-yl)phenyl]-6-trifluoromethyl-1H-benzoimidazole,
6-*tert*-butyl-2-[3-chloro-4-(3-chloro-5-trifluoromethylpyridin-2-yl)phenyl]-1H-benzoimidazole,
2-[3-chloro-4-(3-chloro-5-trifluoromethylpyridin-2-yl)phenyl]-6-methoxy-1H-benzoimidazole,
2-[3-chloro-4-(3-chloro-5-trifluoromethylpyridin-2-yl)phenyl]-5-methoxy-1H-benzoimidazole,
N-[2'-chloro-4'-(4-chloro-6-trifluoromethyl-1H-benzoimidazole-2-yl)-3-fluorobiphenyl-4-yl]methane-sulfonamide,
N-[4'-(4,6-bistrifluoromethyl-1H-benzoimidazole-2-yl)-2'-chloro-3-fluorobiphenyl-4-yl]methanesulfonamide,
2-((3-chloro-4-pyridin-2-ylphenyl)-6-trifluoromethyl-1H-benzoimidazole,
6-*tert*-butyl-2-(3-chloro-4-pyridin-2-ylphenyl)-1H-benzoimidazole,
6-chloro-2-(3-chloro-4-pyridin-2-ylphenyl)-1H-benzoimidazole,
4-chloro-2-(3-chloro-4-pyridin-2-ylphenyl)-6-trifluoromethyl-1H-benzoimidazole,
5,6-dichloro-2-(3-chloro-4-(pyridin-2-yl)phenyl)-1H-benzoimidazole,
6-bromo-2-(3-chloro-4-(pyridin-2-yl)phenyl)-1H-benzoimidazole,
2-[3-chloro-4-(3-trifluoromethylpyridin-2-yl)phenyl]-6-trifluoromethyl-1H-benzoimidazole,
6-*tert*-butyl-2-[3-chloro-4-(3-trifluoromethyl-pyridin-2-yl)phenyl]-1H-benzoimidazole,
6-chloro-2-[3-chloro-4-(3-trifluoromethylpyridin-2-yl)phenyl]-1H-benzoimidazole,
2-[3-chloro-4-(3-trifluoromethylpyridin-2-yl)phenyl]-6-methoxy-1H-benzoimidazole,
2-[3-chloro-4-(3-methylpyridin-2-yl)phenyl]-6-trifluoromethyl-1H-benzoimidazole,
3-chloro-6'-(6-trifluoromethyl-1H-benzoimidazole-2-yl)-[2,3']bipyridine,
3-chloro-6'-(6-chloro-1H-benzoimidazole-2-yl)-[2,3']bipyridine,
3-chloro-6'-(4-chloro-6-trifluoromethyl-1H-benzoimidazole-2-yl)-[2,3']bipyridine,
6'-(4,6-bistrifluoromethyl-1H-benzoimidazole-2-yl)-3-chloro-[2,3']bipyridine,
4-bromo-2-(5-(3-chloropyridin-2-yl)pyridin-2-yl)-6-(trifluoromethyl)-1H-benzoimidazole,
6-bromo-2-(5-(3-chloropyridin-2-yl)pyridin-2-yl)-1H-benzoimidazole,
6'-(6-*tert*-butyl-1H-benzoimidazole-2-yl)-3-chloro-[2,3']bipyridine,
3-chloro-6'-(5,6-dichloro-1H-benzoimidazole-2-yl)-[2,3']bipyridine,
3-chloro-6'-(4,6-dibromo-1H-benzoimidazole-2-yl)-[2,3']bipyridine,
6'-(6-bromo-1H-imidazo[4,5-b]pyridine-2-yl)-3-chloro-[2,3']bipyridine,
3-chloro-6'-(6-morpholin-4-yl-1H-benzoimidazole-2-yl)-[2,3']bipyridine,
3-chloro-6'-(6-thiomorpholin-4-yl-1H-benzoimidazole-2-yl)-[2,3']bipyridine,
3-chloro-6'-(4-morpholin-4-yl-6-trifluoromethyl-1H-benzoimidazole-2-yl)-[2,3']bipyridine,
3-chloro-6'-(4-thiomorpholin-4-yl-6-trifluoromethyl-1H-benzoimidazole-2-yl)-[2,3']bipyridine,
3-chloro-6'-(4-pyrrolidin-1-yl-6-trifluoromethyl-1H-benzoimidazole-2-yl)-[2,3']bipyridine,
3,5-dichloro-6'-(6-trifluoromethyl-1H-benzoimidazole-2-yl)-[2,3']bipyridine,
6'-(6-*tert*-butyl-1H-benzoimidazole-2-yl)-3,5-dichloro-[2,3']bipyridine,
3,5-dichloro-6'-(6-chloro-1H-benzoimidazole-2-yl)-[2,3']bipyridine,
3,5-dichloro-6'-(4-chloro-6-trifluoromethyl-1H-benzoimidazole-2-yl)-[2,3']bipyridine,
6'-(4,6-bistrifluoromethyl-1H-benzoimidazole-2-yl)-3,5-dichloro-[2,3']bipyridine,
6'-(4,6-bistrifluoromethyl-1H-benzoimidazole-2-yl)-3-chloro-5-trifluoromethyl-[2,3']bipyridine,
6'-(6-methoxy-1H-benzoimidazole-2-yl)-3-chloro-5-trifluoromethyl-[2,3']bipyridine,
3-trifluoromethyl-6'-(6-trifluoromethyl-1H-benzoimidazole-2-yl)-[2,3']bipyridine,
6'-(6-*tert*-butyl-1H-benzoimidazole-2-yl)-3-trifluoromethyl-[2,3']bipyridine,
6'-(6-chloro-1H-benzoimidazole-2-yl)-3-trifluoromethyl-[2,3']bipyridine,
6'-(4-chloro-6-trifluoromethyl-1H-benzoimidazole-2-yl)-3-trifluoromethyl-[2,3']bipyridine,
6'-(5-trifluoromethyl-7-chloro-1H-benzoimidazole-2-yl)-3-trifluoromethyl-[2,3']bipyridine,
6'-(4,6-bistrifluoromethyl-1H-benzoimidazole-2-yl)-3-trifluoromethyl-[2,3']bipyridine,
6'-(5,6-dichloro-1H-benzoimidazole-2-yl)-3-trifluoromethyl-[2,3']bipyridine,
4-bromo-6-(trifluoromethyl)-2-(5-(3-(trifluoro-methyl)pyridin-2-yl)pyridin-2-yl)-1H-benzoimidazole,
6-bromo-2-(5-(3-(trifluoromethyl)pyridin-2-yl)pyridin-2-yl)-1H-benzoimidazole,
6-fluoro-2-(5-(3-(trifluoromethyl)pyridin-2-yl)pyridin-2-yl)-1H-benzoimidazole,
6'-(6-bromo-1H-imidazo[4,5-b]pyridine-2-yl)-3-trifluoromethyl[2,3']bipyridine,
6'-(4,6-dibromo-1H-benzoimidazole-2-yl)-3-trifluoromethyl-[2,3']bipyridine,
6'-(6-morpholin-4-yl-1H-benzoimidazo-2-yl)-3-trifluoromethyl-[2,3']bipyridine,
6'-(4-morpholin-4-yl-6-trifluoromethyl-1H-benzoimidazole-2-yl)-3-trifluoromethyl-[2,3']bipyridine,
6'-(6-chloro-1H-benzoimidazole-2-yl)-[2,3']bipyridine,
6'-(6-trifluoromethyl-1H-imidazo[4,5-b]pyridine-2-yl)-[2,3']bipyridine,
5,6-dichloro-2-(5-(pyridin-2-yl)pyridin-2-yl)-1H-benzoimidazole,
6-*tert*-butyl-2-(6-naphthalen-1-ylpyridin-3-yl)-1H-benzoimidazole,

2-((6-naphthalen-1-ylpyridin-3-yl)-4,6-bistrifluoromethyl-1H-benzoimidazole,
2-((6-naphthalen-1-ylpyridin-3-yl)-5,7-bistrifluoromethyl-1H-benzoimidazole,
6-((trifluoromethyl)-2-(6-(naphthalen-1-yl)pyridin-3-yl)-1H-benzoimidazole,
6-chloro-2-(6-(naphthalen-1-yl)pyridin-3-yl)-1H-benzoimidazole,
2-((4-pyrrol-1-ylphenyl]-6-trifluoromethyl-1H-benzoimidazole,
2-((4-pyrrol-1-ylphenyl)-3H-imidazo[4,5-b]pyridine, 6-chloro-2-(4-pyrrol-1-ylphenyl]-1H-benzoimidazole,
6-tert-butyl-2-(4-pyrrol-1-ylphenyl]-1H-benzoimidazole,
6-fluoro-2-(4-pyrrol-1-ylphenyl]-1H-benzoimidazole,
2-biphenyl-4-yl-6-trifluoromethyl-1H-benzoimidazole,
2-biphenyl-4-yl-3H-imidazo[4,5-b]pyridine,
2-biphenyl-4-yl-6-chloro-1H-benzoimidazole,
2-biphenyl-4-yl-6-tert-butyl-1H-benzoimidazole,
2-biphenyl-4-yl-6-fluoro-1H-benzoimidazole,
2-biphenyl-4-yl-6-methoxy-1H-benzoimidazole,
2-((4-phenoxyphenyl-6-trifluoromethyl)-1H-benzoimidazole,
2-((4-phenoxyphenyl)-3H-imidazo[4,5-b]pyridine,
6-chloro-2-(4-phenoxyphenyl)-1H-benzoimidazole,
6-*tert*-butyl-2-(4-phenoxyphenyl)-1H-benzoimidazole,
6-fluoro-2-(4-phenoxyphenyl)-1H-benzoimidazole,
6-methoxy-2-(4-phenoxyphenyl)-1H-benzoimidazole,
2-[3-(4-chloropyrazol-1-ylmethyl)phenyl]-6-trifluoromethyl-1H-benzoimidazole,
6-chloro-2-[3-(4-chloropyrazol-1-ylmethyl)phenyl]-1H-benzoimidazole,
6-*tert*-butyl-2-[3-(4-chloropyrazol-1-ylmethyl)phenyl]-1H-benzoimidazole,
2-[3-(4-chloropyrazol-1-ylmethyl)phenyl]-6-fluoro-1H-benzoimidazole,
phenyl-[4-(6-trifluoromethyl-1H-benzoimidazole-2-yl)phenyl]methanone,
[4-((6-*tert*-butyl-1H-benzoimidazole-2-yl)phenyl]phenylmethanone,
2-[4-(3-chloropyridin-2-yl)phenyl]-6-trifluoromethyl-1H-benzoimidazole chloride,
6-*tert*-butyl-2-[4-(3-chloropyridin-2-yl)phenyl]-1H-benzoimidazole chloride,
6-*tert*-butyl-2-[4-(3-chloropyridin-2-yl)naphthalen-1-yl]-1H-benzoimidazole chloride,
6'-(4,6-bistrifluoromethyl-1H-benzoimidazole-2-yl)-3-chloro[2,3']bipyridine chloride,
3-trifluoromethyl-6'-(6-trifluoromethyl-1H-benzoimidazole-2-yl)-[2,3']bipyridine chloride,
6-*tert*-butyl-2-[4-(3-chloropyridin-2-yl)phenyl]-1H-benzoimidazole sodium salt,
3-trifluoromethyl-6'-(6-trifluoromethyl-1H-benzoimidazole-2-yl)-[2,3']bipyridine sulfate,
3-trifluoromethyl-6'-(6-trifluoromethyl-1H-benzoimidazole-2-yl)-[2,3']bipyridine phosphate,
3-trifluoromethyl-6'-(6-trifluoromethyl-1H-benzoimidazole-2-yl)-[2,3']bipyridine methanesulfonate,
and 3-trifluoromethyl-6'-(6-trifluoromethyl-1H-benzoimidazole-2-yl)-[2,3']bipyridine benzenesulfonate.

**2.** A pharmaceutical composition, comprising a compound of claim 1, or a pharmaceutically acceptable salt thereof as an active ingredient, and a pharmaceutically acceptable carrier.

**3.** A compound of claim 1, or a pharmaceutically acceptable salt thereof, for use in the prevention and treatment of pain, acute pain, chronic pain, neuropathic pain, postoperative pain, migraines, arthralgia, neuropathy, nerve injury, diabetic neuropathy, neurological illness, neurodermatitis, strokes, bladder hypersensitivity, irritable bowel syndrome, respiratory disorders, asthma, chronic obstructive pulmonary disease, burns, psoriasis, itching, vomiting, irritation of the skin, eyes, and mucous membranes, gastric-duodenal ulcers, inflammatory intestinal diseases, inflammatory diseases, and combinations thereof.

## Patentansprüche

**1.** Eine Verbindung oder ein pharmazeutisch akzeptables Salz davon, ausgewählt aus der Gruppe, bestehend aus

2-[4-(3-Chlorpyridin-2-yl)phenyl]-6-trifluormethyl-1H-benzoimidazol,
6-Chlor-2-[4-(3-chlorpyridin-2-yl)phenyl]-1H-benzoimidazol,
6-*tert*-Butyl-2-[4-(3-chlorpyridin-2-yl)phenyl]-1H-benzoimidazol,
2-[4-(3-Chlorpyridin-2-yl)phenyl]-6-fluor-1H-benzoimidazol,
2-[4-(3-Chlorpyridin-2-yl)phenyl]-6-methoxy-1H-benzoimidazol,
4-Chlor-2-[4-(3-chlorpyridin-2-yl)phenyl]-6-trifluormethyl-1 H-benzoimidazol,

2-[4-(3-Chlorpyridin-2-yl)phenyl]-4,6-bistrifluormethyl-1 H-benzoimidazol,
2-[4-(3-Chlorpyridin-2-yl)phenyl]-6-trifluormethyl-1H-imidazo[4,5-b]pyridin,
2-[4-(3-Chlorpyridin-2-yl)phenyl]-3H-imidazo[4,5-b]pyridin,
2-[4-(3-Chlorpyridin-2-yl)phenyl]-6,7-dimethyl-1 H-benzoimidazol,
2-[4-(3-Chlorpyridin-2-yl)phenyl]-1 H-benzoimidazol,
2-[4-(3-Chlorpyridin-2-yl)phenyl]-5,6-dichlor-1 H-benzoimidazol,
6-Brom-2-(4-(3-chlorpyridin-2-yl)phenyl)-1 H-benzoimidazol,
4-Brom-2-(4-(3-chlorpyridin-2-yl)phenyl)-6-(trifluormethyl)-1H-benzoimidazol,
6-Brom-2-(4-(3-chlorpyridin-2-yl)phenyl-1H-imidazo[4,5-b]pyridin,
4,6-Dibrom-2-[4-(3-chlorpyridin-2-yl)phenyl]-1 H-benzoimidazol,
2-[4-(3-Chlorpyridin-2-yl)phenyl]-6-morpholin-4-yl-1 H-benzoimidazol,
2-[4-(3-Chlorpyridin-2-yl)phenyl]-6-thiomorpholin-4-yl-1H-benzoimidazol,
2-[4-(3-Chlorpyridin-2-yl)phenyl]-4-morpholin-4-yl-6-trifluormethyl-1 H-benzoimidazol,
2-[4-(3-Chlorpyridin-2-yl)phenyl]-4-thiomorpholin-4-yl-6-trifluormethyl-1H-benzoimidazol,
2-[4-(3-Chlorpyridin-2-yl)phenyl]-4-pyrrolidin-1-yl-6-trifluormethyl-1 H-benzoimidazol,
{2-[4-(3-Chlorpyridin-2-yl)phenyl]-6-trifluormethyl-1H-benzoimidazol-4-yl}diethylamin,
2-[4-(3-Chlorpyridin-2-yl)phenyl]-6-morpholin-4-yl-1H-imidazo[4,5-b]pyridin,
2-[4-(3-Chlorpyridin-2-yl)phenyl]-6-pyrrolidin-1-yl-1H-imidazo[4,5-b]pyridin,
6-*tert*-Butyl-2-[4-(3,5-dichlorpyridin-2-yl)phenyl]-1H-benzoimidazol,
2-[4-(3,5-Dichlorpyridin-2-yl)phenyl]-4,6-bistrifluormethyl-1H-benzoimidazol,
6-*tert*-Butyl-2-[4-(3-chlor-5-trifluormethyl-pyridin-2-yl)phenyl]-1H-benzoimidazol,
2-[4-(3-Chlor-5-trifluormethylpyridin-2-yl)phenyl]-4,6-bistrifluormethyl-1 H-benzoimidazol,
2-[4-(3-Chlor-5-trifluormethylpyridin-2-yl)phenyl]-5,7-bistrifluormethyl-1 H-benzoimidazol,
N-[4'-(4,6-Bistrifluormethyl-1 H-benzoimidazol-2-yl)-3-fluorbiphenyl-4-yl]methansulfonamid,
6-*tert*-Butyl-2-[4-(3-trifluormethylpyridin-2-yl)phenyl]-1H-benzoimidazol,
4-Chlor-6-trifluormethyl-2-[4-(3-trifluormethylpyridin-2-yl)phenyl]-1H-benzoimidazol,
6-Chlor-2-[4-(3-trifluormethylpyridin-2-yl)phenyl]-1H-benzoimidazol,
4,6-Bistrifluormethyl-2-[4-(3-trifluormethylpyridin-2-yl)phenyl]-1H-benzoimidazol,
6-Trifluormethyl-2-[4-(3-trifluormethylpyridin-2-yl)phenyl]-1H-imidazo[4,5-b]pyridin,
5,6-Dichlor-2-[4-(3-trifluormethylpyridin-2-yl)phenyl]-1H-benzoimidazol,
4-Brom-6-(trifluormethyl)-2-(4-(3-(trifluormethyl)pyridin-2-yl)phenyl)-1H-benzoimidazol,
6-Brom-2-(4-(3-(trifluormethyl)pyridin-2-yl)phenyl)-1H-benzoimidazol,
6-Fluor-2-(4-(3-(trifluormethyl)pyridin-2-yl)phenyl)-1H-benzoimidazol,
6-Brom-2-(4-(3-(trifluormethyl)pyridin-2-yl)phenyl-1H-imidazo[4,5-b]pyridin,
4,6-Dibrom-2-[4-(3-trifluormethylpyridin-2-yl)phenyl]-1H-benzoimidazol,
6-Morpholin-4-yl-2-[4-(3-trifluormethylpyridin-2-yl)phenyl]-1H-benzoimidazol,
6-Thiomorpholin-4-yl-2-[4-(3-trifluormethylpyridin-2-yl)phenyl]-1H-benzoimidazol,
Diethyl-{2-[4-(3-trifluormethylpyridin-2-yl)phenyl]-3H-benzoimidazo-5-yl}amin,
4-Morpholin-4-yl-6-trifluormethyl-2-[4-(3-trifluormethylpyridin-2-yl)phenyl]-1H-benzoimidazol,
4-Thiomorpholin-4-yl-6-trifluormethyl-2-[4-(3-trifluormethylpyridin-2-yl)phenyl]-1H-benzoimidazol,
4-Pyrrolidin-1-yl-6-trifluormethyl-2-[4-(3-trifluormethylpyridin-2-yl)phenyl]-1H-benzoimidazol,
6-*tert*-Butyl-2-(4-pyridin-2-ylphenyl)-1H-benzoimidazol,
6-((Trifluormethyl)-2-(4-(pyridin-2-yl)phenyl)-1H-benzoimidazol,
4-Chlor-6-(trifluormethyl)-2-(4-(pyridin-2-yl)phenyl)-1H-benzoimidazol,
5,6-Dichlor-2-(4-(pyridin-2-yl)phenyl)-1 H-benzoimidazol,
4,6-Bis(trifluormethyl)-2-(4-(pyridin-2-yl)phenyl)-1H-benzoimidazol,
6-Fluor-2-(4-(pyridin-2-yl)phenyl)-1H-benzoimidazol,
6-Brom-2-(4-(pyridin-2-yl)phenyl)-1H-imidazo[4,5-b]pyridin,
4,6-Dibrom-2-(4-pyridin-2-ylphenyl)-1 H-benzoimidazol,
2-[4-(6-Chlor-5-methylpyridazin-3-yl)phenyl]-4,6-bistrifluormethyl-1H-benzoimidazol,
2-[4-(6-Chlor-5-methylpyridazin-3-yl)phenyl]-5,7-bistrifluormethyl-1H-benzoimidazol,
4-Brom-6-(trifluormethyl)-2-(4-(4-(trifluormethyl)pyrimidin-2-yl)phenyl)-1H-benzoimidazol,
4,6-Dibrom-2-[4-(4-trifluormethylpyrimidin-2-yl)phenyl]-1H-benzoimidazol,
6-*tert*-Butyl-2-(4-(pyrimidin-2-yl)phenyl)-1H-benzoimidazol,
2-((4-(6-(Trifluormethyl)-1H-benzoimidazol-2-yl)phenyl)pyridin-3-carbonitril,
2-((4-(6-(*tert*-Butyl-1H-benzoimidazol-2-yl)phenyl)pyridin-3-carbonitril,
6'-(4,6-Dibrom-1 H-benzoimidazol-2-yl)-[2,3']bipyridyl-3-carbonitril,
2-[4-(3-Chlorpyrazin-2-yl)phenyl]-6-trifluormethyl-1H-benzoimidazol,

6-*tert*-Butyl-2-[4-(3-chlorpyrazin-2-yl)phenyl]-1H-benzoimidazol,
6-Brom-2-[4-(3-chlorpyrazin-2-yl)phenyl]-1H-benzoimidazol,
5,6-Dichlor-2-[4-(3-chlorpyrazin-2-yl)phenyl]-1H-benzoimidazol,
6-Brom-2-[4-(3-chlorpyrazin-2-yl)phenyl]-1H-imidazo[4,5-b]pyridin,
6-*tert*-Butyl-2-[4-(3-chlorpyridin-2-yl)-2-fluorphenyl]-1H-benzoimidazol,
4-Chlor-2-[4-(3-chlorpyridin-2-yl)-2-fluorphenyl]-6-trifluormethyl-1H-benzoimidazol,
2-[4-(3-Chlorpyridin-2-yl)-2-fluorphenyl]-6-methoxy-1H-benzoimidazol,
6-*tert*-Butyl-2-[4-(3,5-dichlorpyridin-2-yl)-2-fluorphenyl]-1H-benzoimidazol,
4-Chlor-2-[4-(3,5-dichlorpyridin-2-yl)-2-fluorphenyl]-6-trifluormethyl-1H-benzoimidazol,
2-[4-(3,5-Dichlorpyridin-2-yl)-2-fluorphenyl]-4,6-bistrifluormethyl-1H-benzoimidazol,
4-Chlor-2-[4-(3-chlor-5-trifluormethylpyridin-2-yl)-2-fluorphenyl]-6-trifluormethyl-1H-benzoimidazol,
2-[4-(3-Chlor-5-trifluormethylpyridin-2-yl)-2-fluorphenyl]-6-trifluormethyl-1H-imidazo[4,5-b]pyridin,
2-[4-(3-Chlor-5-trifluormethylpyridin-2-yl)-2-fluorphenyl]-6-methoxy-1H-benzoimidazol,
N-[3,3'-Difluor-4'-(6-methoxy-1H-benzoimidazol-2-yl)biphenyl-4-yl]methansulfonamid,
2-[2-Fluor-4-(3-trifluormethylpyridin-2-yl)phenyl]-6-trifluormethyl-1H-benzoimidazol,
6-*tert*-Butyl-2-[2-fluor-4-(3-trifluormethylpyridin-2-yl)phenyl]-1H-benzoimidazol,
6-Chlor-2-[2-fluor-4-(3-trifluormethylpyridin-2-yl)phenyl]-1H-benzoimidazol,
5-Chlor-2-[2-fluor-4-(3-trifluormethylpyridin-2-yl)phenyl]-1H-benzoimidazol,
4-Chlor-2-[2-fluor-4-(3-trifluormethylpyridin-2-yl)phenyl]-6-trifluormethyl-1H-benzoimidazol,
7-Chlor-2-[2-fluor-4-(3-trifluormethylpyridin-2-yl)phenyl]-5-trifluormethyl-1H-benzoimidazol,
2-((2-Fluor-4-pyridin-2-yl)phenyl-6-trifluormethyl-1H-benzoimidazol,
6-*tert*-Butyl-2-(2-fluor-4-pyridin-2-yl)phenyl-1H-benzoimidazol,
6-Chlor-2-(2-fluor-4-pyridin-2-yl)phenyl-1H-benzoimidazol,
2-((2-Fluor-4-pyridin-2-yl)phenyl-4,6-bistrifluor-methyl-1H-benzoimidazol,
2-[2-Chlor-4-(3-chlorpyridin-2-yl)phenyl]-6-trifluormethyl-1H-benzoimidazol,
4-Chlor-2-[2-chlor-4-(3-chlorpyridin-2-yl)phenyl]-6-trifluormethyl-1H-benzoimidazol,
2-[2-Chlor-4-(3-chlorpyridin-2-yl)phenyl]-6-trifluormethyl-1H-imidazo[4,5-b]pyridin,
2-[2-Chlor-4-(3,5-dichlorpyridin-2-yl)phenyl]-6-trifluormethyl-1H-benzoimidazol,
4-Chlor-2-[2-chlor-4-(3,5-dichlorpyridin-2-yl)phenyl]-6-trifluormethyl-1H-benzoimidazol,
2-[2-Chlor-4-(3,5-dichlorpyridin-2-yl)phenyl]-4,6-bistrifluormethyl-1H-benzoimidazol,
2-[2-Chlor-4-(3,5-dichlorpyridin-2-yl)phenyl]-6-trifluormethyl-1H-imidazo[4,5-b]pyridin,
2-[2-Chlor-4-(3-chlor-5-trifluormethylpyridin-2-yl)phenyl]-6-trifluormethyl-1H-benzoimidazol,
4-Chlor-2-[2-chlor-4-(3-chlor-5-trifluormethylpyridin-2-yl)phenyl]-6-trifluormethyl-1H-benzoimidazol,
N-[4'-(6-*tert*-Butyl-1H-benzoimidazol-2-yl)-3'-chlor-3-fluorbiphenyl-4-yl]methansulfonamid,
N-[3'-Chlor-4'-(4-chlor-6-trifluormethyl-1 H-benzoimidazol-2-yl)-3-fluorbiphenyl-4-yl]methansulfonamid,
N-[4'-(4,6-Bistrifluormethyl-1H-benzoimidazol-2-yl)-3'-chlor-3-fluorbiphenyl-4-yl]methansulfonamid,
2-[2-Chlor-4-(3-trifluormethylpyridin-2-yl)phenyl]-6-trifluormethyl-1H-benzoimidazol,
4-Chlor-2-[2-chlor-4-(3-trifluormethylpyridin-2-yl)phenyl]-6-trifluormethyl-1H-benzoimidazol,
2-[2-Chlor-4-(3-trifluormethylpyridin-2-yl)phenyl]-4,6-bistrifluormethyl-1H-benzoimidazol,
6-*tert*-Butyl-2-[2-chlor-4-(3-methylpyridin-2-yl)phenyl]-1H-benzoimidazol,
6-Chlor-2-(2-chlor-4-pyridin-2-ylphenyl)-1 H-benzoimidazol,
2-((2-Chlor-4-pyridin-2-ylphenyl)-6-trifluormethyl-1H-imidazo[4,5-b]pyridin,
2-((2-Chlor-4-pyridin-2-ylphenyl)-6-methoxy-1 H-benzoimidazol,
2-[4-(3-Chlorpyridin-2-yl)naphthalen-1-yl]-6-trifluormethyl-1H-benzoimidazol,
6-*tert*-Butyl-2-[4-(3-chlorpyridin-2-yl)naphthalen-1-yl]-1H-benzoimidazol,
2-[4-(3-Chlorpyridin-2-yl)naphthalen-1-yl]-4,6-bistrifluormethyl-1 H-benzoimidazol,
2-[4-(3-Chlorpyridin-2-yl)naphthalen-1-yl]-6-trifluormethyl-1H-imidazo[4,5-b]pyridin,
6-*tert*-Butyl-2-[4-(3,5-dichlorpyridin-2-yl)naphthalen-1-yl]-1H-benzoimidazol,
2-[4-(3,5-Dichlorpyridin-2-yl)naphthalen-1-yl]-4,6-bistrifluormethyl-1 H-benzoimidazol,
6-*tert*-Butyl-2-[4-(3-chlor-5-trifluormethylpyridin-2-yl)naphthalen-1-yl]-1H-benzoimidazol,
2-[4-(3-Chlor-5-trifluormethylpyridin-2-yl)naphthalen-1-yl]-4,6-bistrifluormethyl-1H-benzoimidazol,
N-{4-[4-(4,6-Bistrifluormethyl-1H-benzoimidazol-2-yl)naphthalen-1-yl]-2-fluorphenyl}methansulfonamid,
6-Trifluormethyl-2-[4-(3-trifluormethylpyridin-2-yl)naphthalen-1-yl]-1H-benzoimidazol,
6-*tert*-Butyl-2-(4-pyridin-2-ylnaphthalen-1-yl)-1H-benzoimidazol,
2-((4-Pyridin-2-ylnaphthalen-1-yl)-4,6-bistrifluormethyl-1H-benzoimidazol,
6-*tert*-Butyl-2-[3-chlor-4-(3-chlorpyridin-2-yl)phenyl]-1H-benzoimidazol,
2-[3-Chlor-4-(3,5-dichlorpyridin-2-yl)phenyl]-6-trifluormethyl-1H-benzoimidazol,
6-*tert*-Butyl-2-[3-chlor-4-(3,5-dichlorpyridin-2-yl)phenyl]-1H-benzoimidazol,

2-[3-Chlor-4-(3,5-dichlorpyridin-2-yl)phenyl]-6-methoxy-1H-benzoimidazol,
2-[3-Chlor-4-(3,5-dichlorpyridin-2-yl)phenyl]-5-methoxy-1H-benzoimidazol,
2-[3-Chlor-4-(3-chlor-5-trifluormethylpyridin-2-yl)phenyl]-6-trifluormethyl-1H-benzoimidazol,
6-*tert*-Butyl-2-[3-chlor-4-(3-chlor-5-trifluormethylpyridin-2-yl)phenyl]-1H-benzoimidazol,
2-[3-Chlor-4-(3-chlor-5-trifluormethylpyridin-2-yl)phenyl]-6-methoxy-1H-benzoimidazol,
2-[3-Chlor-4-(3-chlor-5-trifluormethylpyridin-2-yl)phenyl]-5-methoxy-1H-benzoimidazol,
N-[2'-Chlor-4'-(4-chlor-6-trifluormethyl-1H-benzoimidazol-2-yl)-3-fluorbiphenyl-4-yl]methansulfonamid,
N-[4'-(4,6-Bistrifluormethyl-1H-benzoimidazol-2-yl)-2'-chlor-3-fluorbiphenyl-4-yl]methansulfonamid,
2-((3-Chlor-4-pyridin-2-ylphenyl)-6-trifluormethyl-1H-benzoimidazol,
6-*tert*-Butyl-2-(3-chlor-4-pyridin-2-ylphenyl)-1H-benzoimidazol,
6-Chlor-2-(3-chlor-4-pyridin-2-ylphenyl)-1 H-benzoimidazol,
4-Chlor-2-(3-chlor-4-pyridin-2-ylphenyl)-6-trifluormethyl-1H-benzoimidazol,
5,6-Dichlor-2-(3-chlor-4-(pyridin-2-yl)phenyl)-1H-benzoimidazol,
6-Brom-2-(3-chlor-4-(pyridin-2-yl)phenyl)-1 H-benzoimidazol,
2-[3-Chlor-4-(3-trifluormethylpyridin-2-yl)phenyl]-6-trifluormethyl-1H-benzoimidazol,
6-*tert*-Butyl-2-[3-chlor-4-(3-trifluormethylpyridin-2-yl)phenyl]-1H-benzoimidazol,
6-Chlor-2-[3-chlor-4-(3-trifluormethylpyridin-2-yl)phenyl]-1H-benzoimidazol,
2-[3-Chlor-4-(3-trifluormethylpyridin-2-yl)phenyl]-6-methoxy-1H-benzoimidazol,
2-[3-Chlor-4-(3-methylpyridin-2-yl)phenyl]-6-trifluormethyl-1H-benzoimidazol,
3-Chlor-6'-(6-trifluormethyl-1H-benzoimidazol-2-yl)-[2,3']bipyridin,
3-Chlor-6'-(6-chlor-1 H-benzoimidazol-2-yl)-[2,3']bipyridin,
3-Chlor-6'-(4-chlor-6-trifluormethyl-1 H-benzoimidazol-2-yl)-[2,3']bipyridin,
6'-(4,6-Bistrifluormethyl-1H-benzoimidazol-2-yl)-3-chlor-[2,3']bipyridin,
4-Brom-2-(5-(3-chlorpyridin-2-yl)pyridin-2-yl)-6-(trifluormethyl)-1H-benzoimidazol,
6-Brom-2-(5-(3-chlorpyridin-2-yl)pyridin-2-yl)-1H-benzoimidazol,
6'-(6-*tert*-Butyl-1H-benzoimidazol-2-yl)-3-chlor-[2,3']bipyridin,
3-Chlor-6'-(5,6-dichlor-1H-benzoimidazol-2-yl)-[2,3']bipyridin,
3-Chlor-6'-(4,6-dibrom-1H-benzoimidazol-2-yl)-[2,3']bipyridin,
6'-(6-Brom-1H-imidazo[4,5-b]pyridin-2-yl)-3-chlor-[2,3']bipyridin,
3-Chlor-6'-(6-morpholin-4-yl-1H-benzoimidazol-2-yl)-[2,3']bipyridin,
3-Chlor-6'-(6-thiomorpholin-4-yl-1H-benzoimidazol-2-yl)-[2,3']bipyridin,
3-Chlor-6'-(4-morpholin-4-yl-6-trifluormethyl-1H-benzoimidazol-2-yl)-[2,3']bipyridin,
3-Chlor-6'-(4-thiomorpholin-4-yl-6-trifluormethyl-1H-benzoimidazol-2-yl)-[2,3']bipyridin,
3-Chlor-6'-(4-pyrrolidin-1-yl-6-trifluormethyl-1 H-benzoimidazol-2-yl)-[2,3']bipyridin,
3,5-Dichlor-6'-(6-trifluormethyl-1H-benzoimidazol-2-yl)-[2,3']bipyridin,
6'-(6-*tert*-Butyl-1H-benzoimidazol-2-yl)-3,5-dichlor-[2,3']bipyridin,
3,5-Dichlor-6'-(6-chlor-1H-benzoimidazol-2-yl)-[2,3']bipyridin,
3,5-Dichlor-6'-(4-chlor-6-trifluormethyl-1H-benzoimidazol-2-yl)-[2,3']bipyridin,
6'-(4,6-Bistrifluormethyl-1H-benzoimidazol-2-yl)-3,5-dichlor-[2,3']bipyridin,
6'-(4,6-Bistrifluormethyl-1H-benzoimidazol-2-yl)-3-chlor-5-trifluormethyl-[2,3']bipyridin,
6'-(6-Methoxy-1H-benzoimidazol-2-yl)-3-chlor-5-trifluormethyl-[2,3']bipyridin,
3-Trifluormethyl-6'-(6-trifluormethyl-1H-benzoimidazol-2-yl)-[2,3']bipyridin,
6'-(6-*tert*-Butyl-1H-benzoimidazol-2-yl)-3-trifluormethyl-[2,3']bipyridin,
6'-(6-Chlor-1H-benzoimidazol-2-yl)-3-trifluormethyl-[2,3']bipyridin,
6'-(4-Chlor-6-trifluormethyl-1H-benzoimidazol-2-yl)-3-trifluormethyl-[2,3']bipyridin,
6'-(5-Trifluormethyl-7-chlor-1H-benzoimidazol-2-yl)-3-trifluormethyl-[2,3']bipyridin,
6'-(4,6-Bistrifluormethyl-1H-benzoimidazol-2-yl)-3-trifluormethyl-[2,3']bipyridin,
6'-(5,6-Dichlor-1H-benzoimidazol-2-yl)-3-trifluormethyl-[2,3']bipyridin,
4-Brom-6-(trifluormethyl)-2-(5-(3-(trifluormethyl)pyridin-2-yl)pyridin-2-yl)-1H-benzoimidazol,
6-Brom-2-(5-(3-(trifluormethyl)pyridin-2-yl)pyridin-2-yl)-1H-benzoimidazol,
6-Fluor-2-(5-(3-(trifluormethyl)pyridin-2-yl)pyridin-2-yl)-1H-benzoimidazol,
6'-(6-Brom-1H-imidazo[4,5-b]pyridin-2-yl)-3-trifluormethyl[2,3']bipyridin,
6'-(4,6-Dibrom-1H-benzoimidazol-2-yl)-3-trifluormethyl-[2,3']bipyridin,
6'-(6-Morpholin-4-yl-1H-benzoimidazo-2-yl)-3-trifluormethyl-[2,3']bipyridin,
6'-(4-Morpholin-4-yl-6-trifluormethyl-1H-benzoimidazol-2-yl)-3-trifluormethyl-[2,3']bipyridin,
6'-(6-Chlor-1H-benzoimidazol-2-yl)-[2,3']bipyridin,
6'-(6-Trifluormethyl-1H-imidazo[4,5-b]pyridin-2-yl)-[2,3']bipyridin,
5,6-Dichlor-2-(5-(pyridin-2-yl)pyridin-2-yl)-1H-benzoimidazol,

6-*tert*-Butyl-2-(6-naphthalen-1-ylpyridin-3-yl)-1H-benzoimidazol,
2-((6-Naphthalen-1-ylpyridin-3-yl)-4,6-bistrifluormethyl-1H-benzoimidazol,
2-((6-Naphthalen-1-ylpyridin-3-yl)-5,7-bistrifluormethyl-1H-benzoimidazol,
6-((Trifluormethyl)-2-(6-(naphthalen-1-yl)pyridin-3-yl)-1H-benzoimidazol,
6-Chlor-2-(6-(naphthalen-1-yl)pyridin-3-yl)-1H-benzoimidazol,
2-((4-Pyrrol-1-ylphenyl]-6-trifluormethyl-1 H-benzoimidazol,
2-((4-Pyrrol-1-ylphenyl)-3H-imidazo[4,5-b]pyridin,
6-Chlor-2-(4-pyrrol-1-ylphenyl]-1H-benzoimidazol,
6-*tert*-Butyl-2-(4-pyrrol-1-ylphenyl]-1H-benzoimidazol,
6-Fluor-2-(4-pyrrol-1-ylphenyl]-1H-benzoimidazol,
2-Biphenyl-4-yl-6-trifluormethyl-1H-benzoimidazol,
2-Biphenyl-4-yl-3H-imidazo[4,5-b]pyridin,
2-Biphenyl-4-yl-6-chlor-1H-benzoimidazol,
2-Biphenyl-4-yl-6-*tert*-butyl-1H-benzoimidazol,
2-Biphenyl-4-yl-6-fluor-1H-benzoimidazol,
2-Biphenyl-4-yl-6-methoxy-1H-benzoimidazol,
2-((4-Phenoxyphenyl-6-trifluormethyl)-1 H-benzoimidazol,
2-((4-Phenoxyphenyl)-3H-imidazo[4,5-b]pyridin,
6-Chlor-2-(4-phenoxyphenyl)-1H-benzoimidazol,
6-*tert*-Butyl-2-(4-phenoxyphenyl)-1H-benzoimidazol,
6-Fluor-2-(4-phenoxyphenyl)-1H-benzoimidazol,
6-Methoxy-2-(4-phenoxyphenyl)-1H-benzoimidazol,
2-[3-(4-Chlorpyrazol-1-ylmethyl)phenyl]-6-trifluormethyl-1H-benzoimidazol,
6-Chlor-2-[3-(4-chlorpyrazol-1-ylmethyl)phenyl]-1 H-benzoimidazol,
6-*tert*-Butyl-2-[3-(4-chlorpyrazol-1-ylmethyl)phenyl]-1H-benzoimidazol,
2-[3-(4-Chlorpyrazol-1-ylmethyl)phenyl]-6-fluor-1 H-benzoimidazol,
Phenyl-[4-(6-trifluormethyl-1H-benzoimidazol-2-yl)phenyl]methanon,
[4-((6-*tert*-Butyl-1H-benzoimidazol-2-yl)phenyl]phenylmethanon,
2-[4-(3-Chlorpyridin-2-yl)phenyl]-6-trifluormethyl-1 H-benzoimidazolchlorid,
6-*tert*-Butyl-2-[4-(3-chlorpyridin-2-yl)phenyl]-1H-benzoimidazolchlorid,
6-*tert*-Butyl-2-[4-(3-chlorpyridin-2-yl)naphthalen-1-yl]-1H-benzoimidazolchlorid,
6'-(4,6-Bistrifluormethyl-1 H-benzoimidazol-2-yl)-3-chlor[2,3']bipyridinchlorid,
3-Trifluormethyl-6'-(6-trifluormethyl-1 H-benzoimidazol-2-yl)-[2,3']bipyridinchlorid,
6-*tert*-Butyl-2-[4-(3-chlorpyridin-2-yl)phenyl]-1H-benzoimidazolnatriumsalz,
3-Trifluormethyl-6'-(6-trifluormethyl-1H-benzoimidazol-2-yl)-[2,3']bipyridinsulfat,
3-Trifluormethyl-6'-(6-trifluormethyl-1H-benzoimidazol-2-yl)-[2,3']bipyridinphosphat,
3-Trifluormethyl-6'-(6-trifluormethyl-1 H-benzoimidazol-2-yl)-[2,3']bipyridinmethansulfonat,
und 3-Trifluormethyl-6'-(6-trifluormethyl-1 H-benzoimidazol-2-yl)-[2,3']bipyridinbenzensulfonat.

**2.** Eine pharmazeutische Zusammensetzung, die eine Verbindung gemäß Anspruch 1 oder ein pharmazeutisch akzeptables Salz davon als Wirkstoff und einen pharmazeutisch akzeptablen Träger beinhaltet.

**3.** Verbindung gemäß Anspruch 1 oder ein pharmazeutisch akzeptables Salz davon zur Verwendung bei der Prävention und Behandlung von Schmerz, akutem Schmerz, chronischem Schmerz, neuropathischem Schmerz, postoperativem Schmerz, Migränen, Arthralgie, Neuropathie, Nervenverletzung, diabetischer Neuropathie, neurologischer Krankheit, Neurodermitis, Schlaganfällen, Blasenüberempfindlichkeit, Reizdarmsyndrom, Atemwegserkrankungen, Asthma, chronisch obstruktiver Lungenerkrankung, Verbrennungen, Schuppenflechte, Jucken, Erbrechen, Irritation der Haut, Augen und Schleimhäute, Magen-Zwölffingerdarm-Geschwüren, entzündlichen Darmerkrankungen, entzündlichen Erkrankungen und Kombinationen davon.

## Revendications

**1.** Un composé, ou un sel acceptable d'un point de vue pharmaceutique de celui-ci, sélectionné dans le groupe consistant en

2-[4-(3-chloropyridin-2-yl)phényl]-6-trifluorométhyl-1 H-benzoimidazole,
6-chloro-2-[4-(3-chloropyridin-2-yl)phényl]-1 H-benzoimidazole,

6-*tert*-butyl-2-[4-(3-chloropyridin-2-yl)phényl]-1H-benzoimidazole,
2-[4-(3-chloropyridin-2-yl)phényl]-6-fluoro-1H-benzoimidazole,
2-[4-(3-chloropyridin-2-yl)phényl]-6-méthoxy-1 H-benzoimidazole,
4-chloro-2-[4-(3-chloropyridin-2-yl)phényl]-6-trifluorométhyl-1 H-benzoimidazole,
2-[4-(3-chloropyridin-2-yl)phényl]-4,6-bistrifluorométhyl-1H-benzoimidazole,
2-[4-(3-chloropyridin-2-yl)phényl]-6-trifluorométhyl-1 H-imidazo[4,5-b]pyridine,
2-[4-(3-chloropyridin-2-yl)phényl]-3H-imidazo[4,5-b]pyridine,
2-[4-(3-chloropyridin-2-yl)phényl]-6,7-diméthyl-1 H-benzoimidazole,
2-[4-(3-chloropyridin-2-yl)phényl]-1 H-benzoimidazole,
2-[4-(3-chloropyridin-2-yl)phényl]-5,6-dichloro-1 H-benzoimidazole,
6-bromo-2-(4-(3-chloropyridin-2-yl)phényl)-1H-benzoimidazole,
4-bromo-2-(4-(3-chloropyridin-2-yl)phényl)-6-(trifluorométhyl)-1H-benzoimidazole,
6-bromo-2-(4-(3-chloropyridin-2-yl)phényl-1 H-imidazo[4,5-b]pyridine,
4,6-dibromo-2-[4-(3-chloropyridin-2-yl)phényl]-1 H-benzoimidazole,
2-[4-(3-chloropyridin-2-yl)phényl]-6-morpholin-4-yl-1 H-benzoimidazole,
2-[4-(3-chloropyridin-2-yl)phényl]-6-thiomorpholin-4-yl-1 H-benzoimidazole,
2-[4-(3-chloropyridin-2-yl)phényl]-4-morpholin-4-yl-6-trifluorométhyl-1 H-benzoimidazole,
2-[4-(3-chloropyridin-2-yl)phényl]-4-thiomorpholin-4-yl-6-trifluorométhyl-1H-benzoimidazole,
2-[4-(3-chloropyridin-2-yl)phényl]-4-pyrrolidin-1-yl-6-trifluorométhyl-1H-benzoimidazole,
{2-[4-(3-chloropyridin-2-yl)phényl]-6-trifluorométhyl-1H-benzoimidazole-4-yl}diéthylamine,
2-[4-(3-chloropyridin-2-yl)phényl]-6-morpholin-4-yl-1 H-imidazo[4,5-b]pyridine,
2-[4-(3-chloropyridin-2-yl)phényl]-6-pyrrolidin-1-yl-1H-imidazo[4,5-b]pyridine,
6-*tert*-butyl-2-[4-(3,5-dichloropyridin-2-yl)phényl]-1H-benzoimidazole,
2-[4-(3,5-dichloropyridin-2-yl)phényl]-4,6-bistrifluorométhyl-1 H-benzoimidazole,
6-*tert*-butyl-2-[4-(3-chloro-5-trifluorométhyl-pyridin-2-yl)phényl]-1H-benzoimidazole,
2-[4-(3-chloro-5-trifluorométhylpyridin-2-yl)phényl]-4,6-bistrifluorométhyl-1H-benzoimidazole,
2-[4-(3-chloro-5-trifluorométhylpyridin-2-yl)phényl]-5,7-bistrifluorométhyl-1H-benzoimidazole,
N-[4'-(4,6-bistrifluorométhyl-1 H-benzoimidazole-2-yl)-3-fluorobiphényl-4-yl]méthanesulfonamide,
6-*tert*-butyl-2-[4-(3-trifluorométhylpyridin-2-yl)phényl]-1H-benzoimidazole,
4-chloro-6-trifluorométhyl-2-[4-(3-trifluorométhylpyridin-2-yl)phényl]-1H-benzoimidazole,
6-chloro-2-[4-(3-trifluorométhylpyridin-2-yl)phényl]-1H-benzoimidazole,
4,6-bistrifluorométhyl-2-[4-(3-trifluorométhylpyridin-2-yl)phényl]-1H-benzoimidazole,
6-trifluorométhyl-2-[4-(3-trifluorométhylpyridin-2-yl)phényl]-1H-imidazo[4,5-b]pyridine,
5,6-dichloro-2-[4-(3-trifluorométhylpyridin-2-yl)phényl]-1H-benzoimidazole,
4-bromo-6-(trifluorométhyl)-2-(4-(3-(trifluorométhyl)pyridin-2-yl)phényl)-1H-benzoimidazole,
6-bromo-2-(4-(3-(trifluorométhyl)pyridin-2-yl)phényl)-1H-benzoimidazole,
6-fluoro-2-(4-(3-(trifluorométhyl)pyridin-2-yl)phényl)-1H-benzoimidazole,
6-bromo-2-(4-(3-(trifluorométhyl)pyridin-2-yl)phényl-1H-imidazo[4,5-b]pyridine,
4,6-dibromo-2-[4-(3-trifluorométhylpyridin-2-yl)phényl]-1H-benzoimidazole,
6-morpholin-4-yl-2-[4-(3-trifluorométhylpyridin-2-yl)phényl]-1H-benzoimidazole,
6-thiomorpholin-4-yl-2-[4-(3-trifluorométhyl-pyridin-2-yl)phényl]-1H-benzoimidazole,
diéthyl-{2-[4-(3-trifluorométhylpyridin-2-yl)phényl]-3H-benzoimidazo-5-yl}amine,
4-morpholin-4-yl-6-trifluorométhyl-2-[4-(3-trifluorométhylpyridin-2-yl)phényl]-1H-benzoimidazole,
4-thiomorpholin-4-yl-6-trifluorométhyl-2-[4-(3-trifluorométhylpyridin-2-yl)phényl]-1H-benzoimidazole,
4-pyrrolidin-1-yl-6-trifluorométhyl-2-[4-(3-trifluorométhylpyridin-2-yl)phényl]-1H-benzoimidazole,
6-*tert*-butyl-2-(4-pyridin-2-ylphényl)-1H-benzoimidazole,
6-((trifluorométhyl)-2-(4-(pyridin-2-yl)phényl)-1H-benzoimidazole,
4-chloro-6-(trifluorométhyl)-2-(4-(pyridin-2-yl)phényl)-1 H-benzoimidazole,
5,6-dichloro-2-(4-(pyridin-2-yl)phényl)-1H-benzoimidazole,
4,6-bis(trifluorométhyl)-2-(4-(pyridin-2-yl)phényl)-1H-benzoimidazole,
6-fluoro-2-(4-(pyridin-2-yl)phényl)-1H-benzoimidazole,
6-bromo-2-(4-(pyridin-2-yl)phényl)-1H-imidazo[4,5-b]pyridine,
4,6-dibromo-2-(4-pyridin-2-ylphényl)-1H-benzoimidazole,
2-[4-(6-chloro-5-méthylpyridazin-3-yl)phényl]-4,6-bistrifluorométhyl-1H-benzoimidazole,
2-[4-(6-chloro-5-méthylpyridazin-3-yl)phényl]-5,7-bistrifluorométhyl-1H-benzoimidazole,
4-bromo-6-(trifluorométhyl)-2-(4-(4-(trifluorométhyl)pyrimidin-2-yl)phényl)-1H-benzoimidazole,
4,6-dibromo-2-[4-(4-trifluorométhylpyrimidin-2-yl)phényl]-1H-benzoimidazole,
6-*tert*-butyl-2-(4-(pyrimidin-2-yl)phényl)-1H-benzoimidazole,

2-((4-(6-(trifluorométhyl)-1H-benzoimidazole-2-yl)phényl)pyridin-3-carbonitrile,
2-((4-(6-(*tert*-butyl-1H-benzoimidazole-2-yl)phényl)pyridin-3-carbonitrile,
6'-(4,6-dibromo-1H-benzoimidazole-2-yl)-[2,3']bipyridyl-3-carbonitrile,
2-[4-(3-chloropyrazin-2-yl)phényl]-6-trifluorométhyl-1 H-benzoimidazole,
6-*tert*-butyl-2-[4-(3-chloropyrazin-2-yl)phényl]-1H-benzoimidazole,
6-bromo-2-[4-(3-chloropyrazin-2-yl)phényl]-1H-benzoimidazole,
5,6-dichloro-2-[4-(3-chloropyrazin-2-yl)phényl]-1H-benzoimidazole,
6-bromo-2-[4-(3-chloropyrazin-2-yl)phényl]-1H-imidazo[4,5-b]pyridine,
6-*tert*-butyl-2-[4-(3-chloropyridin-2-yl)-2-fluorophényl]-1H-benzoimidazole,
4-chloro-2-[4-(3-chloropyridin-2-yl)-2-fluorophényl]-6-trifluorométhyl-1H-benzoimidazole,
2-[4-(3-chloropyridin-2-yl)-2-fluorophényl]-6-méthoxy-1H-benzoimidazole,
6-*tert*-butyl-2-[4-(3,5-dichloropyridin-2-yl)-2-fluorophényl]-1H-benzoimidazole,
4-chloro-2-[4-(3,5-dichloropyridin-2-yl)-2-fluorophényl]-6-trifluorométhyl-1H-benzoimidazole,
2-[4-(3,5-dichloropyridin-2-yl)-2-fluorophényl]-4,6-bistrifluorométhyl-1H-benzoimidazole,
4-chloro-2-[4-(3-chloro-5-trifluorométhylpyridin-2-yl)-2-fluorophényl]-6-trifluorométhyl-1H-benzoimidazole,
2-[4-(3-chloro-5-trifluorométhylpyridin-2-yl)-2-fluorophényl]-6-trifluorométhyl-1H-imidazo[4,5-b]pyridine,
2-[4-(3-chloro-5-trifluorométhylpyridin-2-yl)-2-fluorophényl]-6-méthoxy-1H-benzoimidazole,
N-[3,3'-difluoro-4'-(6-méthoxy-1H-benzoimidazole-2-yl)biphényl-4-yl]méthanesulfonamide,
2-[2-fluoro-4-(3-trifluorométhylpyridin-2-yl)phényl]-6-trifluorométhyl-1H-benzoimidazole,
6-*tert*-butyl-2-[2-fluoro-4-(3-trifluorométhyl-pyridin-2-yl)phényl]-1H-benzoimidazole,
6-chloro-2-[2-fluoro-4-(3-trifluorométhylpyridin-2-yl)phényl]-1H-benzoimidazole,
5-chloro-2-[2-fluoro-4-(3-trifluorométhylpyridin-2-yl)phényl]-1H-benzoimidazole,
4-chloro-2-[2-fluoro-4-(3-trifluorométhylpyridin-2-yl)phényl]-6-trifluorométhyl-1H-benzoimidazole,
7-chloro-2-[2-fluoro-4-(3-trifluorométhylpyridin-2-yl)phényl]-5-trifluorométhyl-1H-benzoimidazole,
2-((2-fluoro-4-pyridin-2-yl)phényl-6-trifluorométhyl-1H-benzoimidazole,
6-*tert*-butyl-2-(2-fluoro-4-pyridin-2-yl)phényl-1H-benzoimidazole,
6-chloro-2-(2-fluoro-4-pyridin-2-yl)phényl-1H-benzoimidazole,
2-((2-fluoro-4-pyridin-2-yl)phényl-4,6-bistrifluoro-méthyl-1H-benzoimidazole,
2-[2-chloro-4-(3-chloropyridin-2-yl)phényl]-6-trifluorométhyl-1H-benzoimidazole,
4-chloro-2-[2-chloro-4-(3-chloropyridin-2-yl)phényl]-6-trifluorométhyl-1H-benzoimidazole,
2-[2-chloro-4-(3-chloropyridin-2-yl)phényl]-6-trifluorométhyl-1H-imidazo[4,5-b]pyridine,
2-[2-chloro-4-(3,5-dichloropyridin-2-yl)phényl]-6-trifluorométhyl-1H-benzoimidazole,
4-chloro-2-[2-chloro-4-(3,5-dichloropyridin-2-yl)phényl]-6-trifluorométhyl-1 H-benzoimidazole,
2-[2-chloro-4-(3,5-dichloropyridin-2-yl)phényl]-4,6-bistrifluorométhyl-1H-benzoimidazole,
2-[2-chloro-4-(3,5-dichloropyridin-2-yl)phényl]-6-trifluorométhyl-1H-imidazo[4,5-b]pyridine,
2-[2-chloro-4-(3-chloro-5-trifluorométhylpyridin-2-yl)phényl]-6-trifluorométhyl-1H-benzoimidazole,
4-chloro-2-[2-chloro-4-(3-chloro-5-trifluorométhylpyridin-2-yl)phényl]-6-trifluorométhyl-1H-benzoimidazole,
N-[4'-(6-*tert*-butyl-1H-benzoimidazole-2-yl)-3'-chloro-3-fluorobiphényl-4-yl]méthanesulfonamide,
N-[3'-chloro-4'-(4-chloro-6-trifluorométhyl-1H-benzoimidazole-2-yl)-3-fluorobiphényl-4-yl]méthanesulfonamide,
N-[4'-(4,6-bistrifluorométhyl-1H-benzoimidazole-2-yl)-3'-chloro-3-fluorobiphényl-4-yl]méthanesulfonamide,
2-[2-chloro-4-(3-trifluorométhylpyridin-2-yl)phényl]-6-trifluorométhyl-1 H-benzoimidazole,
4-chloro-2-[2-chloro-4-(3-trifluorométhylpyridin-2-yl)phényl]-6-trifluorométhyl-1H-benzoimidazole,
2-[2-chloro-4-(3-trifluorométhylpyridin-2-yl)phényl]-4,6-bistrifluorométhyl-1H-benzoimidazole,
6-*tert*-butyl-2-[2-chloro-4-(3-méthylpyridin-2-yl)phényl]-1H-benzoimidazole,
6-chloro-2-(2-chloro-4-pyridin-2-ylphényl)-1H-benzoimidazole,
2-((2-chloro-4-pyridin-2-ylphényl)-6-trifluorométhyl-1 H-imidazo[4,5-b]pyridine,
2-((2-chloro-4-pyridin-2-ylphényl)-6-méthoxy-1 H-benzoimidazole,
2-[4-(3-chloropyridin-2-yl)naphtalén-1-yl]-6-trifluorométhyl-1H-benzoimidazole,
6-*tert*-butyl-2-[4-(3-chloropyridin-2-yl)naphtalén-1-yl]-1H-benzoimidazole,
2-[4-(3-chloropyridin-2-yl)naphtalén-1-yl]-4,6-bistrifluorométhyl-1H-benzoimidazole,
2-[4-(3-chloropyridin-2-yl)naphtalén-1-yl]-6-trifluorométhyl-1H-imidazo[4,5-b]pyridine,
6-*tert*-butyl-2-[4-(3,5-dichloropyridin-2-yl)naphtalén-1-yl]-1H-benzoimidazole,
2-[4-(3,5-dichloropyridin-2-yl)naphtalén-1-yl]-4,6-bistrifluorométhyl-1H-benzoimidazole,
6-*tert*-butyl-2-[4-(3-chloro-5-trifluorométhyl-pyridin-2-yl)naphtalén-1-yl]-1H-benzoimidazole,
2-[4-(3-chloro-5-trifluorométhylpyridin-2-yl)naphtalén-1-yl]-4,6-bistrifluorométhyl-1H-benzoimidazole,
N-{4-[4-(4,6-bistrifluorométhyl-1H-benzoimidazole-2-yl)naphtalén-1-yl]-2-fluorophényl}méthanesulfonamide,
6-trifluorométhyl-2-[4-(3-trifluorométhylpyridin-2-yl)naphtalén-1-yl]-1H-benzoimidazole,

6-*tert*-butyl-2-(4-pyridin-2-ylnaphtalén-1-yl)-1H-benzoimidazole,
2-((4-pyridin-2-ylnaphtalén-1-yl)-4,6-bistrifluorométhyl-1H-benzoimidazole,
6-*tert*-butyl-2-[3-chloro-4-(3-chloropyridin-2-yl)phényl]-1H-benzoimidazole,
2-[3-chloro-4-(3,5-dichloropyridin-2-yl)phényl]-6-trifluorométhyl-1H-benzoimidazole,
6-*tert*-butyl-2-[3-chloro-4-(3,5-dichloropyridin-2-yl)phényl]-1H-benzoimidazole,
2-[3-chloro-4-(3,5-dichloropyridin-2-yl)phényl]-6-méthoxy-1H-benzoimidazole,
2-[3-chloro-4-(3,5-dichloropyridin-2-yl)phényl]-5-méthoxy-1H-benzoimidazole,
2-[3-chloro-4-(3-chloro-5-trifluorométhylpyridin-2-yl)phényl]-6-trifluorométhyl-1H-benzoimidazole,
6-*tert*-butyl-2-[3-chloro-4-(3-chloro-5-trifluorométhylpyridin-2-yl)phényl]-1H-benzoimidazole,
2-[3-chloro-4-(3-chloro-5-trifluorométhylpyridin-2-yl)phényl]-6-méthoxy-1H-benzoimidazole,
2-[3-chloro-4-(3-chloro-5-trifluorométhylpyridin-2-yl)phényl]-5-méthoxy-1H-benzoimidazole,
N-[2'-chloro-4'-(4-chloro-6-trifluorométhyl-1 H-benzoimidazole-2-yl)-3-fluorobiphényl-4-yl]méthanesulfonamide,
N-[4'-(4,6-bistrifluorométhyl-1H-benzoimidazole-2-yl)-2'-chloro-3-fluorobiphényl-4-yl]méthanesulfonamide,
2-((3-chloro-4-pyridin-2-ylphényl)-6-trifluorométhyl-1 H-benzoimidazole,
6-*tert*-butyl-2-(3-chloro-4-pyridin-2-ylphényl)-1H-benzoimidazole,
6-chloro-2-(3-chloro-4-pyridin-2-ylphényl)-1H-benzoimidazole,
4-chloro-2-(3-chloro-4-pyridin-2-ylphényl)-6-trifluorométhyl-1H-benzoimidazole,
5,6-dichloro-2-(3-chloro-4-(pyridin-2-yl)phényl)-1H-benzoimidazole,
6-bromo-2-(3-chloro-4-(pyridin-2-yl)phényl)-1H-benzoimidazole,
2-[3-chloro-4-(3-trifluorométhylpyridin-2-yl)phényl]-6-trifluorométhyl-1 H-benzoimidazole,
6-*tert*-butyl-2-[3-chloro-4-(3-trifluorométhylpyridin-2-yl)phényl]-1H-benzoimidazole,
6-chloro-2-[3-chloro-4-(3-trifluorométhylpyridin-2-yl)phényl]-1H-benzoimidazole,
2-[3-chloro-4-(3-trifluorométhylpyridin-2-yl)phényl]-6-méthoxy-1H-benzoimidazole,
2-[3-chloro-4-(3-méthylpyridin-2-yl)phényl]-6-trifluorométhyl-1H-benzoimidazole,
3-chloro-6'-(6-trifluorométhyl-1H-benzoimidazole-2-yl)-[2,3']bipyridine,
3-chloro-6'-(6-chloro-1H-benzoimidazole-2-yl)-[2,3']bipyridine,
3-chloro-6'-(4-chloro-6-trifluorométhyl-1H-benzoimidazole-2-yl)-[2,3']bipyridine,
6'-(4,6-bistrifluorométhyl-1H-benzoimidazole-2-yl)-3-chloro-[2,3']bipyridine,
4-bromo-2-(5-(3-chloropyridin-2-yl)pyridin-2-yl)-6-(trifluorométhyl)-1H-benzoimidazole,
6-bromo-2-(5-(3-chloropyridin-2-yl)pyridin-2-yl)-1H-benzoimidazole,
6'-(6-*tert*-butyl-1H-benzoimidazole-2-yl)-3-chloro-[2,3']bipyridine,
3-chloro-6'-(5,6-dichloro-1H-benzoimidazole-2-yl)-[2,3']bipyridine,
3-chloro-6'-(4,6-dibromo-1H-benzoimidazole-2-yl)-[2,3']bipyridine,
6'-(6-bromo-1H-imidazo[4,5-b]pyridin-2-yl)-3-chloro-[2,3']bipyridine,
3-chloro-6'-(6-morpholin-4-yl-1H-benzoimidazole-2-yl)-[2,3']bipyridine,
3-chloro-6'-(6-thiomorpholin-4-yl-1H-benzoimidazole-2-yl)-[2,3']bipyridine,
3-chloro-6'-(4-morpholin-4-yl-6-trifluorométhyl-1 H-benzoimidazole-2-yl)-[2,3']bipyridine,
3-chloro-6'-(4-thiomorpholin-4-yl-6-trifluorométhyl-1H-benzoimidazole-2-yl)-[2,3']bipyridine,
3-chloro-6'-(4-pyrrolidin-1-yl-6-trifluorométhyl-1 H-benzoimidazole-2-yl)-[2,3']bipyridine,
3,5-dichloro-6'-(6-trifluorométhyl-1H-benzoimidazole-2-yl)-[2,3']bipyridine,
6'-(6-*tert*-butyl-1H-benzoimidazole-2-yl)-3,5-dichloro-[2,3']bipyridine,
3,5-dichloro-6'-(6-chloro-1H-benzoimidazole-2-yl)-[2,3']bipyridine,
3,5-dichloro-6'-(4-chloro-6-trifluorométhyl-1 H-benzoimidazole-2-yl)-[2,3']bipyridine,
6'-(4,6-bistrifluorométhyl-1H-benzoimidazole-2-yl)-3,5-dichloro-[2,3']bipyridine,
6'-(4,6-bistrifluorométhyl-1H-benzoimidazole-2-yl)-3-chloro-5-trifluorométhyl-[2,3']bipyridine,
6'-(6-méthoxy-1H-benzoimidazole-2-yl)-3-chloro-5-trifluorométhyl-[2,3']bipyridine,
3-trifluorométhyl-6'-(6-trifluorométhyl-1H-benzoimidazole-2-yl)-[2,3']bipyridine,
6'-(6-*tert*-butyl-1H-benzoimidazole-2-yl)-3-trifluorométhyl-[2,3']bipyridine,
6'-(6-chloro-1H-benzoimidazole-2-yl)-3-trifluorométhyl-[2,3']bipyridine,
6'-(4-chloro-6-trifluorométhyl-1H-benzoimidazole-2-yl)-3-trifluorométhyl-[2,3']bipyridine,
6'-(5-trifluorométhyl-7-chloro-1H-benzoimidazole-2-yl)-3-trifluorométhyl-[2,3']bipyridine,
6'-(4,6-bistrifluorométhyl-1H-benzoimidazole-2-yl)-3-trifluorométhyl-[2,3']bipyridine,
6'-(5,6-dichloro-1 H-benzoimidazole-2-yl)-3-trifluorométhyl-[2,3']bipyridine,
4-bromo-6-(trifluorométhyl)-2-(5-(3-(trifluorométhyl)pyridin-2-yl)pyridin-2-yl)-1H-benzoimidazole,
6-bromo-2-(5-(3-(trifluorométhyl)pyridin-2-yl)pyridin-2-yl)-1H-benzoimidazole,
6-fluoro-2-(5-(3-(trifluorométhyl)pyridin-2-yl)pyridin-2-yl)-1H-benzoimidazole,
6'-(6-bromo-1H-imidazo[4,5-b]pyridin-2-yl)-3-trifluorométhyl[2,3']bipyridine,

6'-(4,6-dibromo-1H-benzoimidazole-2-yl)-3-trifluorométhyl-[2,3']bipyridine,
6'-(6-morpholin-4-yl-1H-benzoimidazo-2-yl)-3-trifluorométhyl-[2,3']bipyridine,
6'-(4-morpholin-4-yl-6-trifluorométhyl-1H-benzoimidazole-2-yl)-3-trifluorométhyl-[2,3']bipyridine,
6'-(6-chloro-1H-benzoimidazole-2-yl)-[2,3']bipyridine,
6'-(6-trifluorométhyl-1H-imidazo[4,5-b]pyridin-2-yl)-[2,3']bipyridine,
5,6-dichloro-2-(5-(pyridin-2-yl)pyridin-2-yl)-1H-benzoimidazole,
6-*tert*-butyl-2-(6-naphtalén-1-ylpyridin-3-yl)-1H-benzoimidazole,
2-((6-naphtalén-1-ylpyridin-3-yl)-4,6-bistrifluorométhyl-1H-benzoimidazole,
2-((6-naphtalén-1-ylpyridin-3-yl)-5,7-bistrifluorométhyl-1H-benzoimidazole,
6-((trifluorométhyl)-2-(6-(naphtalén-1-yl)pyridin-3-yl)-1H-benzoimidazole,
6-chloro-2-(6-(naphtalén-1-yl)pyridin-3-yl)-1 H-benzoimidazole,
2-((4-pyrrol-1-ylphényl]-6-trifluorométhyl-1H-benzoimidazole,
2-((4-pyrrol-1-ylphényl)-3H-imidazo[4,5-b]pyridine,
6-chloro-2-(4-pyrrol-1-ylphényl]-1 H-benzoimidazole,
6-*tert*-butyl-2-(4-pyrrol-1-ylphényl]-1H-benzoimidazole,
6-fluoro-2-(4-pyrrol-1-ylphényl]-1 H-benzoimidazole,
2-biphényl-4-yl-6-trifluorométhyl-1 H-benzoimidazole,
2-biphényl-4-yl-3H-imidazo[4,5-b]pyridine,
2-biphényl-4-yl-6-chloro-1 H-benzoimidazole,
2-biphényl-4-yl-6-*tert*-butyl-1H-benzoimidazole,
2-biphényl-4-yl-6-fluoro-1 H-benzoimidazole,
2-biphényl-4-yl-6-méthoxy-1 H-benzoimidazole,
2-((4-phénoxyphényl-6-trifluorométhyl)-1H-benzoimidazole,
2-((4-phénoxyphényl)-3H-imidazo[4,5-b]pyridine,
6-chloro-2-(4-phénoxyphényl)-1H-benzoimidazole,
6-*tert*-butyl-2-(4-phénoxyphényl)-1H-benzoimidazole,
6-fluoro-2-(4-phénoxyphényl)-1 H-benzoimidazole,
6-méthoxy-2-(4-phénoxyphényl)-1 H-benzoimidazole,
2-[3-(4-chloropyrazol-1-ylméthyl)phényl]-6-trifluorométhyl-1 H-benzoimidazole,
6-chloro-2-[3-(4-chloropyrazol-1-ylméthyl)phényl]-1 H-benzoimidazole,
6-*tert*-butyl-2-[3-(4-chloropyrazol-1-ylméthyl)phényl]-1H-benzoimidazole,
2-[3-(4-chloropyrazol-1-ylméthyl)phényl]-6-fluoro-1 H-benzoimidazole,
phényl-[4-(6-trifluorométhyl-1H-benzoimidazole-2-yl)phényl]méthanone,
[4-((6-*tert*-butyl-1H-benzoimidazole-2-yl)phényl]phénylméthanone,
chlorure de 2-[4-(3-chloropyridin-2-yl)phényl]-6-trifluorométhyl-1H-benzoimidazole,
chlorure de 6-*tert*-butyl-2-[4-(3-chloropyridin-2-yl)phényl]-1H-benzoimidazole,
chlorure de 6-*tert*-butyl-2-[4-(3-chloropyridin-2-yl)naphtalén-1-yl]-1H-benzoimidazole,
chlorure de 6'-(4,6-bistrifluorométhyl-1 H-benzoimidazole-2-yl)-3-chloro[2,3']bipyridine,
chlorure de 3-trifluorométhyl-6'-(6-trifluorométhyl-1H-benzoimidazole-2-yl)-[2,3']bipyridine,
sel de sodium de 6-*tert*-butyl-2-[4-(3-chloropyridin-2-yl)phényl]-1H-benzoimidazole,
sulfate de 3-trifluorométhyl-6'-(6-trifluorométhyl-1H-benzoimidazole-2-yl)-[2,3']bipyridine,
phosphate de 3-trifluorométhyl-6'-(6-trifluorométhyl-1H-benzoimidazole-2-yl)-[2,3']bipyridine,
méthanesulfonate de 3-trifluorométhyl-6'-(6-trifluorométhyl-1H-benzoimidazole-2-yl)-[2,3']bipyridine,
et benzènesulfonate de 3-trifluorométhyl-6'-(6-trifluorométhyl-1H-benzoimidazole-2-yl)-[2,3']bipyridine.

**2.** Une composition pharmaceutique, comprenant un composé de la revendication 1, ou un sel acceptable d'un point de vue pharmaceutique de celui-ci comme principe actif, et un véhicule acceptable d'un point de vue pharmaceutique.

**3.** Un composé de la revendication 1, ou un sel acceptable d'un point de vue pharmaceutique de celui-ci, destiné à être utilisé dans la prévention et le traitement de la douleur, de la douleur aiguë, de la douleur chronique, de la douleur neuropathique, de la douleur post-opératoire, des migraines, de l'arthralgie, d'une neuropathie, d'une lésion nerveuse, de la neuropathie diabétique, d'une affection neurologique, de la neurodermatite, des accidents vasculaires cérébraux, de l'hypersensibilité de la vessie, du syndrome du côlon irritable, de troubles respiratoires, de l'asthme, de la bronchopneumopathie chronique obstructive, des brûlures, du psoriasis, des prurits, des vomissements, d'une irritation de la peau, des yeux et des membranes muqueuses, des ulcères gastro-duodénaux, de maladies intestinales inflammatoires, de maladies inflammatoires, et de combinaisons de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020040034804
- WO 0208221 A
- WO 03062209 A
- WO 2004055003 A
- WO 2004055004 A
- WO 2004002983 A
- WO 0216317 A
- WO 2004035549 A
- WO 2004014871 A
- WO 03099284 A
- WO 03022809 A
- WO 02090326 A
- WO 02072536 A
- WO 03068749 A
- WO 2004033435 A
- WO 02076946 A
- WO 0095420 A
- WO 03055484 A
- WO 03014064 A
- WO 03080578 A
- WO 03097586 A
- WO 03070247 A
- WO 03029199 A

### Non-patent literature cited in the description

- **CATERINA et al.** *Nature,* 1997, vol. 389, 816
- **TOMINAGA et al.** *Neuron,* 1998, vol. 21, 531
- **HWANG.** *PNAS,* 2000, vol. 97, 3655
- **PREMKUMAR et al.** *Nature,* 2000, vol. 408, 985
- **CATERINA et al.** *Science,* 2000, vol. 288, 306
- **NOZAWA et al.** *Neuroscience Letter,* 2001, vol. 309, 33
- **YIANGOU et al.** Lancet. North America Edition, 2001, vol. 357, 1338
- **BIRDER et al.** *PNAS,* 2001, vol. 98, 13396
- *Pharmacol. Rev.,* 1986, vol. 38, 179
- **SZALLASI.** *J. Med chem.,* 2004, vol. 47, 2717
- **TAPOLCSANYI.** *Tetrahedron,* 2002, vol. 58, 10137
- **SHARMA.** *Bioorg. Med. Chem. Lett.,* 1998, vol. 8, 3459-3464
- **ROGLIC.** *Pharmazie,* 2001, vol. 56, 803
- **RIES.** *J. Med. Chem,* 1993, vol. 36, 4040
- **ABHA.** *Indian Journal of Chemistry,* 2002, vol. 41, 1978
- **LU.** *Syn. Comm,* 2002, vol. 32, 3703
- *J. Pharm. Sci.,* 1977, vol. 66, 1